# EUROPEAN PATENT SPECIFICATION

(11) **EP 2 788 752 B1**
(45) Date of publication and mention of the grant of the patent: **03.10.2018**
(21) Application number: 12855900.2
(22) Date of filing: 05.12.2012
(51) Int. Cl.: G01N 33/574, G01N 33/542

(54) **METHOD OF THERAPY SELECTION FOR PATIENTS WITH LUNG CANCER**
THERAPIEAUSWAHLVERFAHREN FÜR PATIENTEN MIT LUNGENKREBS
PROCÉDÉ DE SÉLECTION D'UN TRAITEMENT DESTINÉ À DES PATIENTS SOUFFRANT D'UN CANCER DU POUMON

(30) Priority: 05.12.2011 US 201161567085 P; 20.11.2012 US 201261728748 P
(43) Date of publication of application: 15.10.2014
(73) Proprietor: Pierian Holdings, Inc., Franklin, TN 37067 (US)
(72) Inventor: KULLER, Anne, Del Mar, California 92014 (US); LEE, Tani Ann T., San Diego, California 92127 (US); HAZRAR, Saswati, San Diego, California 92122 (US); KIRKLAND, Richard, San Diego, California 92111 (US); LIU, Xinjun, San Diego, California 92130 (US); KIM, Phillip, Irvine, California 92612 (US)
(74) Representative: J A Kemp
(86) International application number: PCT/US2012/068005
(87) International publication number: WO 2013/086031

(56) References cited:
- WO-A1-2009/108637
- WO-A1-2011/008990
- WO-A2-2009/012140
- WO-A2-2011/088149
- US-A1- 2009 035 792
- US-A1- 2009 191 559
- US-A1- 2010 167 945
- US-A1- 2011 281 748
- LIU XINJUN ET AL: "Functional profiling of multiple signal pathway proteins in breast cancer patients", AMERICAN ASSOCIATION FOR CANCER RESEARCH. PROCEEDINGS OF THE ANNUAL MEETING, AMERICAN ASSOCIATION FOR CANCER RESEARCH, US, vol. 51, 1 April 2010 (2010-04-01), page 773, XP001526411, ISSN: 0197-016X
- KIM PHILLIP ET AL: "Functional profiling of signal transduction pathway proteins in gastric cancer patients", AMERICAN ASSOCIATION FOR CANCER RESEARCH. PROCEEDINGS OF THE ANNUAL MEETING, AMERICAN ASSOCIATION FOR CANCER RESEARCH, US, vol. 51, 1 April 2010 (2010-04-01), pages 976-977, XP001526410, ISSN: 0197-016X
- Nicholas B. La Thangue ET AL: "Predictive biomarkers: a paradigm shift towards personalized cancer medicine", NATURE REVIEWS CLINICAL ONCOLOGY, vol. 8, no. 10, 1 January 2011 (2011-01-01), pages 587-596, XP55326955, NY, US ISSN: 1759-4774, DOI: 10.1038/nrclinonc.2011.121

## Description

### BACKGROUND OF THE INVENTION

Signal transduction pathways that mediate cell growth and survival are targets for cancer therapy, as tumorigenesis often involves dysfunctional signal transduction pathways. Signaling abnormalities provide cancer cells increased growth potential, and the ability to avert apoptosis induced by DNA damaging agents.

For example, one well characterized signal transduction pathway is the phosphatidylinositol 3-kinase (PI3K) pathway. It has been implicated in a variety of cellular processes, *e.g.,* malignant transformation, growth factor signaling, inflammation, and immunity. Activation of the pathway is initiated when a growth factor or ligand binds to its cognate receptor tyrosine kinase (RTK). These receptors include members of the human epidermal growth factor receptor family (HER, EGFR or ErbB), platelet-derived growth factor (PDGF) receptor, insulin and insulin-like growth factor 1 (IGF-1) receptors. Subsequent RTK dimerization and phosphorylation enable the PI3K heterodimer to bind directly to activated RTKs and/or adaptor proteins. Activated PI3K catalyzes the phosphorylation of phosphatidylinositol-4,5-biphosphate (PI(4,5)P2 or PIP2) to phosphatidylinositol-3,4,5-triphosphate (P(3,4,5)P3 or PIP3). PIP3 facilitates the phosphorylation of AKT, which is the central effector of the PI3K pathway. AKT transmits signals to a host of downstream substrates, thus controlling a variety of key cellular function, including growth, metabolism, proliferation and survival.

Inappropriate co-opting of the PI3K pathway commonly occurs in human cancer. The PI3K pathway is frequently hyperactivated in breast cancer, as well as other tumor types. It has been shown that 70% of breast cancers have a dysregulated PI3K pathway (Lopez-Knowles et al. Int. J. Cancer, 126,1121-1131 (2010)). It has been well established that mutations in the PIK3CA gene (encodes for the PI3K p110 subunit) are common in tumors, including breast, colon and endometrial cancers, and glioblastomas. Additionally, in many cancers, RTKs are often mutated, amplified, or overexpressed, thereby causing aberrant PI3K activation. Taken together, these findings have made RTKs and PI3K signaling attractive targets for cancer therapeutics.

Currently several PI3K pathway inhibitors are under investigation in preclinical studies and the results appear promising (Markman et al., Annls. Oncol. 21(4), 683-691 (2010)). Inhibition of cancer cell proliferation was seen in some patients receiving PI3K inhibitors (Courtney et al. J. Clin. Oncol. 28(6), 1075-1083 (2010)). While these PI3K therapies have demonstrated success in the treatment of cancers (Markman et al., Ann. Oncol., 23(9):2399-2408, (2012); Bendell et al., J. Clin. Oncol., 30(3):282-290, (2012)), it is important to recognize that not all subjects treated respond, or respond well. There is a need for methods to predict those subjects likely to respond well to targeted inhibitor therapy. Thus, there is a need for predictive biomarker assays that interrogate multiple key signaling pathways that can be used to determine the clinical sensitivity to a particular targeted inhibitor alone and/or inhibitor-combination therapy. The present invention satisfies these and other needs.

WO 2011/008990 describes a method for selecting a suitable anticancer drug for the treatment of a gastric cancer comprising determining the expression level and/or activation level of one or more analytes selected from the group consisting of HER1, HER2, p95HER2, HER3, c-Met, IGF1R, cKIT, PI3K, She, Akt, p70S6K, VEGFR, and PDGFR. WO 2009/012140 relates to a method for selecting a suitable anticancer drug for the treatment of lung tumors comprising detecting an activation state of a plurality of signal transduction molecules.

### BRIEF SUMMARY OF THE INVENTION

The invention provides a method for selecting treatment for a subject diagnosed with lung cancer, the method comprising:
(a) measuring the levels of phospho-PI3K, phospho-AKT, phospho-ERK, phospho-MEK, phospho-RSK, phospho-PRAS40, and phospho-RPS6 in a sample taken from the subject using a proximity assay to determine a pathway profile, wherein the proximity assay is a Collaborative Enzyme Enhanced Reactive immunoassay (CEER);
(b) comparing the subject's pathway profile to a reference pathway profile, wherein the reference pathway profile is determined using a sample from a cancer cell line or from a subject having a specific type of cancer; and
(c) recommending a targeted PI3K inhibitor, a MEK inhibitor or a combination of a PI3K inhibitor and a MEK inhibitor therapy by determining whether the subject is likely to respond to the targeted inhibitor therapy based on the subject's pathway profile.

The present invention also provides a method for optimizing therapy or monitoring therapeutic efficacy of a targeted inhibitor for a subject with lung cancer, the method comprising:
(a) measuring the levels of phospho-PI3K, phospho-AKT, phospho-ERK, phospho-MEK, phospho-RSK, phospho-PRAS40, and phospho-RPS6 in a sample taken from the subject using a proximity assay at a plurality of time points over the course of therapy with a targeted inhibitor selected from the group consisting of a PI3K inhibitor, a MEK inhibitor and a combination thereof to determine a pathway profile, wherein the proximity assay is a CEER;
(b) determining whether the subject is responsive to the targeted inhibitor based on the subject's pathway profile;
(c) comparing the subject's pathway profile to a reference pathway profile, wherein the reference pathway profile is determined using a sample from a cancer cell line or from a subject having a specific type of cancer; and
(d) recommending the administration of the targeted inhibitor be continued if the subject is responsive.

The method may optionally further comprise recommending the administration of the targeted inhibitor be modified or of at least two targeted inhibitors if the subject is nonresponsive. In some embodiments, the first time point in the plurality of time points is prior to the course of therapy with the targeted inhibitor. In some embodiments, the subsequent time point or points in the plurality of time points is during the course of therapy with the targeted inhibitor.

Optionally, in the methods of the invention the sample is a fine needle aspirate (FNA), a tumor tissue biopsy, a tumor cell, or a circulating tumor cell.

In some embodiments of the methods of the present invention, the PI3K inhibitor is selected from the group consisting of BYL719, BAY841236, BAY806946, SF1 126, XL147, XL765, NVP-BEZ235, NVP-BGT226, NVP-BKM120, GDC-0941, PX-866, GSK1059615, CAL-101, and combinations thereof.

In some embodiments of the methods of the present invention, the MEK inhibitor is selected from the group consisting of BAY869766, MEK 162, GDC-0973/RG7420, GDC-0623/RG7421, RG7167, RG7304, XL518, PD-325901, and combinations thereof.

### BRIEF DESCRIPTION OF THE DRAWINGS

**FIG. 1** illustrates an exemplary signal transduction pathway that can be analyzed using CEER. The components of the pathway include detectable signal transduction analytes.
**FIG. 2** illustrates the detection of PI3K activation (*e.g.,* complexation) in two cancer cell lines T47D and BT474 using CEER. "%CV" refers to the percentage coefficient variation. "S/B ratio" refers to the signal-to-background ratio.
**FIG. 3** illustrates the detection of PI3K activation (*e.g.,* complexation) in two cancer cell lines T47D and BT474 after growth factor stimulation. **FIG. 3A** shows that PI3K activation was measured in unstimulated T47D cells and cells stimulated with EGF or HRG. **FIG. 3B** shows that PI3K activation was detected in unstimulated BT474 cells and cells stimulated with heregulin (HRG). "%CV" refers to the percentage coefficient variation. "S/B ratio" refers to the signal-to-background ratio.
**FIG. 4** illustrates pathway profiling of cancer cell lines. **FIG. 4A** shows a bar graph of phospho-PI3K (p-PI3K) levels in various cancer cell lines. **FIG. 4B** shows a bar graph of total (t-PI3K) levels. **FIG. 4C** shows a bar graph of phospho PI3K (p-PI3K) levels in the cancer cell lines prior to and after growth factor stimulation. **FIG. 4D** shows a bar graph of total PI3K (t-PI3K) levels. **FIG. 4E** shows a graph of the ratio of phospho-PI3K to total PI3K levels in the cancer cell lines prior to and after growth factor stimulation.
**FIG. 5** illustrates pathway profiling of cancer cell lines. **FIG. 5A** shows a bar graph of phospho-PI3K levels in the cell lines. **FIG. 5B** shows a bar graph of total PI3K levels. **FIG. 5C** shows a bar graph of phospho AKT levels. **FIG. 5D** shows a bar graph of phospho RPS6 levels. **FIG. 5E** shows a graph of the ratio of phospho-PI3K to total PI3K levels in the cancer cell lines.
**FIG. 6A****, B** show the activation of signal transduction analytes in NCI-H1155 cells after PI3K inhibitor therapy, MEK inhibitor therapy, or combination thereof. The analytes were measured either 4 hours or 24 hours (*) after treatment.
**FIG. 7A****, B** show the activation of signal transduction analytes in NCI-H1299 cells after PI3K inhibitor therapy, MEK inhibitor therapy, or combination thereof. The analytes were measured either 4 hours or 24 hours (*) after treatment.
**FIG. 8A****, B** show the activation of signal transduction analytes in NCI-H2228 cells after PI3K inhibitor therapy, MEK inhibitor therapy, or combination thereof. The analytes were measured either 4 hours or 24 hours (*) after treatment.
**FIG. 9A****, B** show the activation of signal transduction analytes in NCI-H661 cells after PI3K inhibitor therapy, MEK inhibitor therapy, or combination thereof. The analytes were measured either 4 hours or 24 hours (*) after treatment.
**FIG. 10A****, B** show the activation of signal transduction analytes in NCI-H647 cells after PI3K inhibitor therapy, MEK inhibitor therapy, or combination thereof. The analytes were measured either 4 hours or 24 hours after treatment.
**FIG. 11** shows the activation of signal transduction analytes in NCI-H460 cells after PI3K inhibitor (*e.g.,* BAY841236) therapy, MEK inhibitor (*e.g.,* BAY869766) therapy, or combination thereof. The analytes were measured either 4 hours or 24 hours after treatment.
**FIG. 12** shows the activation of signal transduction analytes in NCI-H1975 cells after PI3K inhibitor (*e.g.,* BAY841236) therapy, MEK inhibitor (*e.g.,* BAY869766) therapy, or combination thereof. The analytes were measured either 4 hours or 24 hours after treatment.
**FIG. 13** highlights the level of p-AKT inhibition, p-PRAS40 inhibition, and pRPS6 inhibition at the IC50 of PI3K inhibitor in various cancer cell lines. The table shows the IC50 of the PI3K inhibitor GDC0941 in the cell lines.
**FIG. 14** illustrates that cell lines with PI3K activation have a low IC50 for a PI3K inhibitor. The IC50 value was increased in cell lines (*e.g.,* Calu-6) having two or more alteration in one or more signaling transduction pathways. The graph shows that phospho-PI3K is a marker for sensitivity to PI3K inhibitors.
**FIG. 15** shows the level of expression and activation of signal transduction analytes in samples (SG1, SG2 and SG3) from Patient #1. The samples were obtained from the patient prior to receiving a course of therapy (SG1), and day 8 (SG2) and day 28 (SG3) during therapy. The levels of the analytes were normalized to the expression of the control protein cytokeratin (CK).
**FIG. 16** shows the level of expression and activation of signal transduction analytes in two samples (DPI and DP2) from Patient #2. The samples were obtained from the patient prior to receiving a course of therapy (DP1) and during therapy (DP2). The levels of the analytes were normalized to the expression of the control protein cytokeratin (CK).
**FIG. 17** represents a table of expression and activation of signal transduction analytes for Patient #3. The patient does not have PIK3CA or BRAF mutations. The levels of the activated analyte per unit and the total analyte per unit were calculated based on the determined CU of each analyte measured.
**FIG. 18** shows the levels of expression and activation of signal transduction analytes in two biopsy samples from Patient #4.
**FIG. 19** shows the levels of expression and activation of signal transduction analytes in samples from Patient #5 taken prior to receiving a course of therapy and during therapy.
**FIG. 20A****, B** show the levels of expression and activation of signal transduction analytes in two biopsy samples from Patient #6.
**FIG. 21** shows the levels of expression and activation of signal transduction analytes in samples from colorectal cancer patients before receiving a course of therapy and during therapy.
**FIG. 22** shows the level of expression and activation of signal transduction analytes in samples from non-small cell lung cancer patients before receiving a course of therapy and during therapy.
**FIG. 23** shows the level of activated PI3K (left bar) and activated AKT (right bar) in FNA samples from 60 breast cancer patients.
**FIG. 24** represents the statistical analysis of the data of FIG. 23. In particular, cut-off values for p-PI3K and p-AKT were examined to identify statistically relevant correlations.
**FIG. 25** shows the distribution of p-PI3K levels in the samples and at the different cut-off values.
**FIG. 26** shows the distribution of p-AKT levels in the samples and at the different cut-off values.
**FIG. 27** shows the immunoblot analysis of antibodies raised against the antigen listed and other PI3K antigens. The blots shows that the antibodies TY-12466, YK-12469 and 4G10 can detect the p85 subunit of PI3K in cells stimulated with heregulin.

### DETAILED DESCRIPTION

### I. Introduction

As described above, the activation of signal transduction pathways involved in cell proliferation is a molecular feature that is characteristic of many different types of cancer. In many cases, the activity of particular signal transduction pathways, and components thereof, may serve as molecular signatures for a given type of cancer. Such activated components may further provide useful targets for therapeutic intervention. Accordingly, knowledge of the activity level of a particular signal transduction system within a cancer cell prior to, during, and after treatment provides a physician with highly relevant information that may be used to select an appropriate course of treatment to adopt. Furthermore, the continued monitoring of signal transduction pathways that are active in cancer cells as treatment progresses can provide the physician with additional information on the efficacy of treatment, prompting the physician to either continue a particular course of treatment or to switch to another line of treatment, when, for example, cancer cells have become resistant to treatment through further aberrations that activate either the same or another signal transduction pathway.

Accordingly, the present disclosure provides methods for detecting the status (*e.g.,* expression and/or activation levels) of components of a plurality of signal transduction pathways in tumor cells (*e.g.,* circulating cells, fine needle aspirates, or tissue biopsies). Information on the expression and/or activation states of components of signal transduction pathways can be used for cancer diagnosis, prognosis, and in the design of patient-specific cancer treatments. The disclosure also provides methods for the selection of appropriate therapy (single drugs or combinations of drugs) to down-regulate or shut down a deregulated signaling pathway. Thus, the disclosure can be used to facilitate the design of personalized therapies for cancer patients.

One approach in developing tumor therapy is to block intracellular signaling through activated signal transduction pathways. Preclinical models have demonstrated that the use of PI3K pathway inhibitors can elicit dramatic anticancer responses in breast cancer patients with PI3K activation. Most interestingly, the presence of PIK3CA mutations failed to correlate with those patients who responded to PI3K therapy. The methods described herein may be used for measuring (*e.g.,* detecting and quantitating) PI3K pathway activation as well as other signaling pathways that converge or are compensatory with PI3K signaling. These methods are useful in selecting cancer patients who will be clinically sensitive to PI3K inhibitors and PI3K inhibitor-combination therapy. The continued monitoring of signal transduction pathways that are active in cancer cells as treatment progresses can provide the physician with additional information on the efficacy of treatment, prompting the physician to either continue a particular course of treatment or to switch to another line of treatment, when, for example, cancer cells have become resistant to treatment through further aberrations that activate either the same or another signal transduction pathway.

The methods herein have been used to ascertain that tumor cells can activate one or more compensatory signaling pathways in response to anticancer therapy. Without being bound by any particular theory, it is believed that tumor cells adapt to specific pathway inhibitors by activating associated signaling pathways that are not direct targets of the inhibitor. Thus, combination therapy with pathway-directed inhibitors may be required to achieve optimal response to treatment in some cancers. Moreover, these findings highlight the need for methods to monitor activated signaling pathways in a clinical setting.

### II. Definitions

The term "cancer" is intended to include any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, digestive and gastrointestinal cancers such as gastric cancer (*e.g.,* stomach cancer), colorectal cancer, gastrointestinal stromal tumors (GIST), gastrointestinal carcinoid tumors, colon cancer, rectal cancer, anal cancer, bile duct cancer, small intestine cancer, and esophageal cancer; breast cancer; lung cancer (*e.g.,* non-small cell lung cancer (NSCLC)); gallbladder cancer; liver cancer; pancreatic cancer; appendix cancer; prostate cancer, ovarian cancer; renal cancer (*e.g.,* renal cell carcinoma); cancer of the central nervous system; skin cancer; lymphomas; gliomas; choriocarcinomas; head and neck cancers; osteogenic sarcomas; and blood cancers. Examples of non-small cell lung cancer include, but are not limited to, squamous cell carcinoma, large cell carcinoma, and adenocarcinoma. As used herein, a "tumor" comprises one or more cancerous cells. The invention is limited to lung cancer.

The term "analyte" includes any molecule of interest, typically a macromolecule such as a polypeptide, whose presence, amount (expression level), activation state, and/or identity is determined. In certain instances, the analyte is a signal transduction molecule such as, *e.g.,* a component of a HER1, HER2, HER3, cMet, IGF-1R, MEK, or PI3K/AKT signaling pathway. Other signal transduction molecules are listed below.

The term "signal transduction molecule" or "signal transducer" includes proteins and other molecules that carry out the process by which a cell converts an extracellular signal or stimulus into a response, typically involving ordered sequences of biochemical reactions inside the cell. Examples of signal transduction molecules include, but are not limited to, receptor tyrosine kinases such as EGFR (*e.g.,* EGFR/HER1/ErbB1, HER2/Neu/ErbB2, HER3/ErbB3, HER4/ErbB4), VEGFR1/FLT1, VEGFR2/FLK1/KDR, VEGFR3/FLT4, FLT3/FLK2, PDGFR (*e.g.,* PDGFRA, PDGFRB), c-KIT/SCFR, INSR (insulin receptor), IGF-IR, IGF-IIR, IRR (insulin receptor-related receptor), CSF-1R, FGFR 1-4, HGFR 1-2, CCK4, TRK A-C, c-MET, RON, EPHA 1-8, EPHB 1-6, AXL, MER, TYRO3, TIE 1-2, TEK, RYK, DDR 1-2, RET, c-ROS, V-cadherin, LTK (leukocyte tyrosine kinase), ALK (anaplastic lymphoma kinase), ROR 1-2, MUSK, AATYK 1-3, and RTK 106; truncated forms of receptor tyrosine kinases such as truncated HER2 receptors with missing amino-terminal extracellular domains (*e.g.,* p95ErbB2 (p95m), p110, p95c, p95n, *etc*.), truncated cMET receptors with missing amino-terminal extracellular domains, and truncated HER3 receptors with missing amino-terminal extracellular domains; receptor tyrosine kinase dimers (*e.g.,* p95HER2/HER3; p95HER2/HER2; truncated HER3 receptor with HER1, HER2, HER3, or HER4; HER2/HER2; HER3/HER3; HER2/HER3; HER1/HER2; HER1/HER3; HER2/HER4; HER3/HER4; *etc*.); non-receptor tyrosine kinases such as BCR-ABL, Src, Frk, Btk, Csk, Abl, Zap70, Fes/Fps, Fak, Jak, Ack, and LIMK; tyrosine kinase signaling cascade components such as AKT (*e.g.,* AKT1, AKT2, AKT3), MEK (MAP2K1), ERK2 (MAPK1), ERK1 (MAPK3), PI3K (*e.g.,* PIK3CA (p110), PIK3R1 (p85)), PDK1, PDK2, phosphatase and tensin homolog (PTEN), SGK3, 4E-BP1, P70S6K (*e.g.,* p70 S6 kinase splice variant alpha I), protein tyrosine phosphatases (*e.g.,* PTP1B, PTPN13, BDP1, *etc*.), RAF, PLA2, MEKK, JNKK, JNK, p38, Shc (p66), Ras (*e.g.,* K-Ras, N-Ras, H-Ras), Rho, Rac1, Cdc42, PLC, PKC, p53, cyclin D1, STAT1, STAT3, phosphatidylinositol 4,5-bisphosphate (PIP2), phosphatidylinositol 3,4,5-trisphosphate (PIP3), mTOR, BAD, p21, p27, ROCK, IP3, TSP-1, NOS, GSK-3β, RSK 1-3, JNK, c-Jun, Rb, CREB, Ki67, and paxillin; nuclear hormone receptors such as estrogen receptor (ER), progesterone receptor (PR), androgen receptor, glucocorticoid receptor, mineralocorticoid receptor, vitamin A receptor, vitamin D receptor, retinoid receptor, thyroid hormone receptor, and orphan receptors; nuclear receptor coactivators and repressors such as amplified in breast cancer-1 (AIB1) and nuclear receptor corepressor 1 (NCOR), respectively; and combinations thereof.

The term "activation state" refers to whether a particular signal transduction molecule or analyte such as a PI3K signaling pathway component is activated. Similarly, the term "activation level" refers to what extent a particular signal transduction molecule such as a PI3K signaling pathway component is activated. The activation state typically corresponds to the phosphorylation, ubiquitination, and/or complexation status of one or more signal transduction molecules. Non-limiting examples of activation states (listed in parentheses) include: HER1/EGFR (EGFRvIII, phosphorylated (p-) EGFR, EGFR:Shc, ubiquitinated (u-) EGFR, p-EGFRvIII); ErbB2 (p-ErbB2, p95HER2 (truncated ErbB2), p-p95HER2, ErbB2:Shc, ErbB2:PI3K, ErbB2:EGFR, ErbB2:ErbB3, ErbB2:ErbB4); ErbB3 (p-ErbB3, truncated ErbB3, ErbB3:PI3K, p-ErbB3:PI3K, ErbB3:Shc); ErbB4 (p-ErbB4, ErbB4:Shc); c-MET (p-c-MET, truncated c-MET, c-Met:HGF complex); AKT1 (p-AKT1); AKT2 (p-AKT2); AKT3 (p-AKT3); PTEN (p-PTEN); P70S6K (p-P70S6K); MEK (p-MEK); ERK1 (p-ERK1); ERK2 (p-ERK2); PDK1 (p-PDK1); PDK2 (p-PDK2); SGK3 (p-SGK3); 4E-BP1 (p-4E-BP1); PIK3R1 (p-PIK3R1); c-KIT (p-c-KIT); ER (p-ER); IGF-1R (p-IGF-1R, IGF-1R:IRS, IRS:PI3K, p-IRS, IGF-1R:PI3K); INSR (p-INSR); FLT3 (p-FLT3); HGFR1 (p-HGFR1); HGFR2 (p-HGFR2); RET (p-RET); PDGFRA (p-PDGFRA); PDGFRB (p-PDGFRB); VEGFR1 (p-VEGFR1, VEGFR1:PLCγ, VEGFR1:Src); VEGFR2 (p-VEGFR2, VEGFR2:PLCγ, VEGFR2:Src, VEGFR2:heparin sulphate, VEGFR2:VE-cadherin); VEGFR3 (p-VEGFR3); FGFR1 (p-FGFR1); FGFR2 (p-FGFR2); FGFR3 (p-FGFR3); FGFR4 (p-FGFR4); TIE1 (p-TIEl); TIE2 (p-TIE2); EPHA (p-EPHA); EPHB (p-EPHB); GSK-3β (p-GSK-3β); NFKB (p-NFKB), IKB (p-IKB, p-P65:IKB); BAD (p-BAD, BAD:14-3-3); mTOR (p-mTOR); Rsk-1 (p-Rsk-1); Jnk (p-Jnk); P38 (p-P38); STAT1 (p-STAT1); STAT3 (p-STAT3); FAK (p-FAK); RB (p-RB); Ki67; p53 (p-p53); CREB (p-CREB); c-Jun (p-c-Jun); c-Src (p-c-Src); paxillin (p-paxillin); GRB2 (p-GRB2), Shc (p-Shc), Ras (p-Ras), GAB1 (p-GAB1), SHP2 (p-SHP2), GRB2 (p-GRB2), CRKL (p-CRKL), PLCγ (p-PLCγ), PKC (*e.g.,* p-PKCα, p-PKCβ, p-PKCδ), adducin (p-adducin), RB1 (p-RB1), and PYK2 (p-PYK2).

The term "signaling" or "pathway" includes any component of a signal transduction pathway to which the term is referring. For example, PI3K signaling pathway members refers to any member of the PI3K signaling pathway, including PI3K, AKT, PTEN, PIP3, PDK1, PKB, 4E-8P1, mTOR, P70S6K, and RPS6. MEK signaling pathway members include RAS, RAF, MEK, ERK (MAPK), ELK1, FOS, MNK1, RSK, and elF43.

The term "targeted therapy" or "pathway-directed therapy" includes the use of therapeutic agents which can alter the expression and/or activation state of proteins or molecules deregulated in a disease state.

The term "tumor adaptation" includes the ability of a tumor to progress after therapeutic interventions. For instance, tumor adaptation occurs as a tumor becomes resistant to anticancer therapy. Tumor adaptation can occur when a signaling pathway in a tumor cell is blocked by inhibitor treatment and another signaling pathway is activated, thus making the tumor cell resistance to the inhibitor treatment.

The term "disease progression" includes a classification of a cancer that continues to grow or spread, which can lead to additional signs or symptoms of cancer. For example, the recurrence of tumors in lung tissue in patients with NSCLC is described herein as disease progression.

The term "feedback inhibition" or "negative feedback loop" includes a signal transduction mechanism in which a particular signal transduction pathway is inhibited (e.g., blocked, inactivated) when a particular component of the signal transduction pathway has accumulated to a certain level, thereby controlling the activity of the pathway.

The term "disease progression" includes a classification of a cancer that continues to grow or spread, which can lead to additional signs or symptoms of cancer. For example, the recurrence of tumors in lung tissue in patients with NSCLC is described herein as disease progression.

The term "responsive" as used herein includes the ability of a tumor cell or a subject to respond (e.g., react, change, modify) to a therapy that abrogates an oncogenic signaling pathway or alleviates the disease state. For instance, a subject who is responsive to a drug treatment may experience a reduction in disease progression or tumor adaptation, or an amelioration of disease. The term "nonresponsive" includes the inability of a tumor cell or a subject to react to a therapy.

The term "pathway profile" or "pathway signature" includes a determination (e.g., measurement, categorization, analysis, or classification) of the activation states of one or more signal transduction molecules in a tumor cell from a cell line or a patient's cancer. In some instances, the profile or signature is generated using a tumor cell in the absence or presence of any anticancer drug.

The term "serial changes" includes the ability of an assay to detect changes in the expression level and/or activation level of a protein in a sample taken from a subject at different points in time. For example, the expression level and/or activation level of PI3K protein can be monitored in a patient during the course of therapy, including a time prior to starting therapy.

As used herein, the term "dilution series" is intended to include a series of descending concentrations of a particular sample (*e.g.,* cell lysate) or reagent (*e.g.,* antibody). A dilution series is typically produced by a process of mixing a measured amount of a starting concentration of a sample or reagent with a diluent (*e.g.,* dilution buffer) to create a lower concentration of the sample or reagent, and repeating the process enough times to obtain the desired number of serial dilutions. The sample or reagent can be serially diluted at least 2, 3, 4, 5, 6, 7, 8, 9, 10, 15, 20, 25, 30, 35, 40, 45, 50, 100, 500, or 1000-fold to produce a dilution series comprising at least 2, 3, 4, 5, 6, 7, 8, 9, 10, 11, 12, 13, 14, 15, 16, 17, 18, 19, 20, 25, 30, 35, 40, 45, or 50 descending concentrations of the sample or reagent. For example, a dilution series comprising a 2-fold serial dilution of a capture antibody reagent at a 1 mg/ml starting concentration can be produced by mixing an amount of the starting concentration of capture antibody with an equal amount of a dilution buffer to create a 0.5 mg/ml concentration of the capture antibody, and repeating the process to obtain capture antibody concentrations of 0.25 mg/ml, 0.125 mg/ml, 0.0625 mg/ml, 0.0325 mg/ml, *etc.*

The term "superior dynamic range" as used herein refers to the ability of an assay to detect a specific analyte in as few as one cell or in as many as thousands of cells. For example, the immunoassays described herein possess superior dynamic range because they advantageously detect a particular signal transduction molecule of interest in about 1-10,000 cells (*e.g.,* about 1, 5, 10, 25, 50, 75, 100, 250, 500, 750, 1000, 2500, 5000, 7500, or 10,000 cells) using a dilution series of capture antibody concentrations.

As used herein, the term "circulating cells" comprises extratumoral cells that have either metastasized or micrometastasized from a solid tumor. Examples of circulating cells include, but are not limited to, circulating tumor cells, cancer stem cells, and/or cells that are migrating to the tumor (*e.g.,* circulating endothelial progenitor cells, circulating endothelial cells, circulating pro-angiogenic myeloid cells, circulating dendritic cells, *etc*.). Patient samples containing circulating cells can be obtained from any accessible biological fluid (*e.g.,* whole blood, serum, plasma, sputum, bronchial lavage fluid, urine, nipple aspirate, lymph, saliva, fine needle aspirate, *etc*.). In certain instances, the whole blood sample is separated into a plasma or serum fraction and a cellular fraction (*i.e.,* cell pellet). The cellular fraction typically contains red blood cells, white blood cells, and/or circulating cells of a solid tumor such as circulating tumor cells (CTCs), circulating endothelial cells (CECs), circulating endothelial progenitor cells (CEPCs), cancer stem cells (CSCs), disseminated tumor cells of the lymph node, and combinations thereof. The plasma or serum fraction usually contains, *inter alia,* nucleic acids (*e.g.,* DNA, RNA) and proteins that are released by circulating cells of a solid tumor.

Circulating cells are typically isolated from a patient sample using one or more separation methods including, for example, immunomagnetic separation (*see, e.g.,* Racila et al., Proc. Natl. Acad. Sci. USA, 95:4589-4594 (1998); Bilkenroth et al., Int. J. Cancer, 92:577-582 (2001)), the CellTracks® System by Immunicon (Huntingdon Valley, PA), microfluidic separation (*see, e.g.,* Mohamed et al., IEEE Trans. Nanobiosci., 3:251-256 (2004); Lin et al., Abstract No. 5147, 97th AACR Annual Meeting, Washington, D.C. (2006)), FACS (*see, e.g.,* Mancuso et al., Blood, 97:3658-3661 (2001)), density gradient centrifugation (*see, e.g.,* Baker et al., Clin. Cancer Res., 13:4865-4871 (2003)), and depletion methods (*see, e.g.,* Meye et al., Int. J. Oncol., 21:521-530 (2002)).

The term "sample" as used herein includes any biological specimen obtained from a patient. Samples include, without limitation, whole blood, plasma, serum, red blood cells, white blood cells (*e.g.,* peripheral blood mononuclear cells), ductal lavage fluid, nipple aspirate, lymph (*e.g.,* disseminated tumor cells of the lymph node), bone marrow aspirate, saliva, urine, stool (*i.e.,* feces), sputum, bronchial lavage fluid, tears, fine needle aspirate (*e.g.,* harvested by random periareolar fine needle aspiration), any other bodily fluid, a tissue sample (*e.g.,* tumor tissue) such as a biopsy of a tumor (*e.g.,* needle biopsy) or a lymph node (*e.g.,* sentinel lymph node biopsy), a tissue sample (*e.g.,* tumor tissue) such as a surgical resection of a tumor, and cellular extracts thereof. The sample may be whole blood or a fractional component thereof such as plasma, serum, or a cell pellet. A sample may be obtained by isolating circulating cells of a solid tumor from whole blood or a cellular fraction thereof using any technique known in the art. The sample may be a formalin fixed paraffin embedded (FFPE) tumor tissue sample, *e.g.,* from a solid tumor of the stomach or other portion of the gastrointestinal tract.

A "biopsy" refers to the process of removing a tissue sample for diagnostic or prognostic evaluation, and to the tissue specimen itself. Any biopsy technique known in the art can be applied to the methods and compositions described herein. The biopsy technique applied will generally depend on the tissue type to be evaluated and the size and type of the tumor (*i.e.,* solid or suspended (*i.e.,* blood or ascites)), among other factors. Representative biopsy techniques include excisional biopsy, incisional biopsy, needle biopsy (*e.g.,* core needle biopsy, fine-needle aspiration biopsy, *etc*.), surgical biopsy, and bone marrow biopsy. Biopsy techniques are discussed, for example, in Harrison's Principles of Internal Medicine, Kasper, et al., eds., 16th ed., 2005, Chapter 70, and throughout Part V. One skilled in the art will appreciate that biopsy techniques can be performed to identify cancerous and/or precancerous cells in a given tissue sample.

As used herein, the term "circulating cells" comprises extratumoral cells that have either metastasized or micrometastasized from a solid tumor. Examples of circulating cells include, but are not limited to, circulating tumor cells, cancer stem cells, and/or cells that are migrating to the tumor (*e.g.,* circulating endothelial progenitor cells, circulating endothelial cells, circulating pro-angiogenic myeloid cells, circulating dendritic cells, etc.).

The term "subject" or "patient" or "individual" typically includes humans, but can also include other animals such as, *e.g.,* other primates, rodents, canines, felines, equines, ovines, porcines, and the like.

The term "Gleason grade" for prostate cancer is defined as a grade from 1-10. Two Gleason grade numbers are actually determined and then added up to get the final Gleason score, one score for the primary grade and one score for the secondary grade. The Gleason score is the sum of the primary and secondary grades of the biopsied tissue samples by the pathologist. The lowest number on the Gleason grade scale is 1, and the highest is 5. As a result, the total score can be from a 2 (1 + 1) to a 10 (5 + 5). Scores from 2 to 4 are very low on the cancer aggression scale. Scores from 5 to 6 are mildly aggressive. A score of 7 indicates that the cancer is moderately aggressive. Scores from 8 to 10 indicate that the cancer is highly aggressive.

### III. Description

The present disclosure provides methods for the measurement (*e.g.,* detection and quantitation) of the levels of expression and/or the degree of activation (*e.g.,* phosphorylation) of signal transduction analytes in a tumor sample from a patient. In another aspect, the methods provide a pathway profile specific to the patient, wherein the profile represents the expression and/or activation status of the signal transduction analytes relative to a reference pathway profile. In some aspects, the reference profile represents a pathway profile of a healthy subject or a subject not suspected of having cancer. In other aspects, the reference profile represents a pathway profile of a cancer cell line.

As such, the present disclosure advantageously provides benefits to patients with solid tumors, such as breast cancer, lung cancer (*e.g.,* non-small cell lung cancer), gastric cancer, pancreatic cancer, prostate cancer, and colorectal cancer. In some aspects, methods are useful in selecting cancer patients who will be clinically sensitive to anticancer drugs (*e.g.,* HER family inhibitors, PI3K inhibitors, AKT inhibitors, MEK inhibitors, mTOR inhibitors, aromatase inhibitors, PTEN inhibitors, cMET inhibitors, and the like) and combinations thereof. In some aspects, the methods are used to select the first line therapy for a patient. In other aspects, the methods are performed to choose the therapy for a patient who has progressing, recurring, or relapsing cancer. The methods herein are also useful for selecting combination therapy or selecting the proper dose and treatment regimen.

In some aspects, a reference pathway profile is generated using a sample obtained from a patient having a specific type of cancer (*e.g.,* breast tumor, lung tumor, colorectal tumor, pancreatic tumor, prostate tumor, etc.) prior to anticancer drug treatment.

In another aspect, the present disclosure provides methods for determining a reference pathway profile by measuring (*e.g.,* detecting and quantitating) the level of expression and/or the degree of activation (*e.g.,* phosphorylation or complexation) of signal transduction analytes in a sample derived from a cancer cell line. In some instances, the cancer cell line is stimulated with a growth factor (e.g., heregulin, EGF, FGF, TGF-α, etc.) and/or a targeted inhibitor. In another aspect, the methods provide a pathway profile specific to the cell line and/or a targeted inhibitor, wherein the profile represents the expression and/or activation status of the signal transduction analytes of the cell line before, during, and/or after treatment with a targeted inhibitor or combination thereof.

In some aspects, the cell line is derived from a patient with cancer such as, but not limited to, breast cancer, lung cancer (*e.g.,* non-small cell lung cancer), gastric cancer, skin cancer, pancreatic cancer, prostate cancer, and colorectal cancer. In other aspects, the cell line is derived from human tissue such as, bladder, breast, lung, pancreas, stomach, skin, and the like. In yet other aspects, the cell line is a cancer cell line. In some aspects, the methods are useful in measuring the change in one or more signal transduction analytes in a cancer cell line treated with an anticancer drug.

In another aspect, the present disclosure provides methods for selecting an anticancer drug treatment for a patient with solid tumor cancer by comparing the detection and/or quantitation of the activation status of signal transduction proteins in tumor samples either in the presence or absence of an anticancer drug. For instance, samples from a patient are exposed to a variety of anticancer drugs, and the anticancer drug eliciting the most profound anticancer effect on the cells, as determined by the activation status of a particular set of signal transduction analytes is selected for the patient as the optimal therapy. The methods of the present disclosure can be used to design patient specific personalized therapies for cancer. In other aspects, the methods can be used to identify druggable targets for developing new therapies.

The present disclosure also provides methods for selecting appropriate therapies to down-regulate (*e.g.,* inactivate or shut-down) one or more deregulated signal transduction pathways involved in cancer. Thus, the present disclosure can be used to facilitate the design of personalized therapies based on the particular molecular signature provided by the collection of total and/or activated signal transduction proteins in a given patient's tumor or sample.

In another aspect, the disclosure provides methods for monitoring disease progression by measuring (*e.g.,* detection and quantitation) of the level of expression and/or the degree of activation (*e.g.,* phosphorylation) of signal transduction analytes in tumor samples from a patient taken during the course of therapy. Continued monitoring of signal transduction pathways that are active in cancer cells as treatment progresses provides the clinician with additional information on the efficacy of treatment, prompting the clinician to either continue a particular course of treatment or to switch to another line of treatment, when, for example, the cancer cells have become resistant to treatment through activation of either the same or another signal transduction pathway. The methods described herein are used to ascertain that tumor cells in a patient have activated one or more compensatory signal pathways in response to anticancer therapy.

In some aspects, methods for monitoring disease are performed periodically to track changes in the tumor cells over time. Longitudinal analysis of changes of pathway analytes can be a prognostic marker of therapeutic response or disease progression. In yet another aspect, the compositions and methods described herein advantageously identify patients who are resistant to anticancer therapy due to mutations in the target protein kinase, acquired resistance to therapeutic agent, adaptation by signal transduction molecules to therapy, non-compliance with the therapeutic regimen, and/or administration of a suboptimal drug dose. In some aspects, the methods include predicting a patient's response to a particular anticancer therapy. For example, the method of the disclosure can be used to predict that a patient with a particular pathway profile (*e.g.,* activated PI3K, EGFR, HER2 and/or HER3) will likely benefit from a combination therapy (e.g., HER2-modulating drug and TKI), even though the patient was previously diagnosed with triple negative breast cancer (e.g., ER-, PR-, HER2-).

In some aspects, methods of the present disclosure are performed on samples from patients who are receiving one or more targeted therapies by screening and monitoring them throughout the course of therapy and evaluating whether the patients should be switched to an alternative targeted therapy or combination therapy. In certain aspects, the present disclosure provides methods to determine whether a cancer or tumor has adapted to existing anticancer therapy. In certain instances, tumor adaptation to therapy results in activation of compensatory signaling pathways which can be detected using methods of the present disclosure. In other instances, determination of tumor adaption in a patient indicates that the patient's treatment should be switched to an alternative targeted therapy or a combination therapy.

In certain aspects, the methods described herein are to monitor and follow cancer or tumor adaptation to existing anticancer therapy. In certain instances, by following and monitoring the activation of pathway analytes using CEER techniques, it can be ascertained whether there is pathway compensation for existing therapy by for example, shunting activation and/or expression to an associated pathway. These techniques allow for evaluation of therapy efficacy. If the original pathway activation is shut down or diminished, it is important to interrogate the associated pathways to ascertain whether there is pathway compensation in another pathway. In these instances, a combination therapy regimen may be recommended, or switching therapies altogether may be recommended. For example, as illustrated in Examples 3, 4 and 6 described herein, methods of the present disclosure are used to monitor levels of activated HER1, HER2, HER3, cMET, IGF-1R, PI3K, AKT, ERK, MEK, p70S6K, PDK1, PRAS40, PTEN, RPS6, SHC in tumor cells from patients who relapsed on anticancer therapy. In these patients' tumors, compensatory signaling pathways not directly targeted by the therapy were activated. Advantageously, using the methods herein, the pathway profiling analysis indicates that the patients can clinically benefit from PI3K inhibitor therapy, MEK inhibitor therapy, or combination therapy.

Analysis of expression of target protein or genetic mutation alone has limitations for selecting the appropriate therapy regimen with maximal clinical efficacy for a subject with cancer or suspected of having cancer. Comprehensive pathway profiling of signal transduction analytes provides insightful information on the efficacy of specific anticancer agents on their intended target proteins. The methods of the present disclosure also provide valuable information regarding potential drug resistance mechanisms such as activation of compensatory pathways and provides a guide to effective therapeutic treatments and regimens for patients with cancer.

In the methods of the invention, the method is for selecting treatment for a subject diagnosed with lung cancer, or for optimizing or monitoring therapeutic efficacy of a targeted inhibitor for a subject with lung cancer.

### A. Antibody Arrays

In certain aspects, the expression level and/or activation state of one or more (*e.g.,* a plurality) of signal transduction molecules (*e.g.,* a receptor tyrosine kinase such as HER2 or other members of the ErbB family, or a signaling pathway component such as PI3K) in a cellular extract of cancer cells such as breast cancer, lung cancer, pancreatic cancer, colorectal cancer, gastric cancer, skin cancer or other cancer cells is detected using an antibody-based array comprising a dilution series of capture antibodies restrained on a support. The arrays typically comprise a plurality of different capture antibodies at a range of capture antibody concentrations that are coupled to the surface of the solid support in different addressable locations.

In some particular aspects, the signal transduction pathway profiling of the disclosure comprises determining the expression level (*e.g.,* total amount) of at least one or more of HER1, HER2, cMET, cKIT, IGF-IR, PI3K, AKT, ERK, and/or CK (i.e., cytokeratin) and/or determining the activation level (*e.g.,* level of phosphorylation ("p") or complex formation) of at least one or more of HER1 (*e.g.,* pHER1), HER2 (*e.g.,* pHER2), HER3 *(e.g.,* pHER3), cMET (*e.g.,* pcMET), cKIT (*e.g.,* pcKIT), IGF-IR (*e.g.,* pIGF-1R), PI3K (*e.g.,* PI3K complex), AKT (*e.g.,* pAKT), ERK (*e.g.,* pERK), SHC (*e.g.,* pSHC), PRAS40 (*e.g.,* pPRAS40), p70S6K (*e.g.,* p-p70S6K), RPS6 (*e.g.,* pRPS6), MEK (*e.g.,* pMEK), PDK1 (*e.g.,* pPDK1), and/or PTEN (*e.g.,* pPTEN). In the methods of the present invention, the levels of phospho-PI3K, phospho-AKT, phospho-ERK, phospho-MEK, phospho-RSK, phospho-PRAS40 and phospho-RPS6 are measured in a sample taken from a subject with lung cancer.

In one particular aspect, the present disclosure provides an addressable array having superior dynamic range comprising a plurality of dilution series of capture antibodies restrained on a solid support, in which the capture antibodies in each dilution series are specific for one or more analytes corresponding to a component of a signal transduction pathway and other target proteins. In various instances, this aspect includes arrays that comprise components of signal transduction pathways characteristic of particular tumors, *e.g.,* signal transduction pathways active in tumor cells (*e.g.,* HER pathway, cMET pathway, IGF1R pathway, MEK pathyway, PI3K pathway). Thus, the disclosure may be advantageously practiced wherein each signal transduction molecule or other protein of interest with a potential expression or activation defect causing cancer is represented on a single array or chip. In some aspects, the components of a given signal transduction pathway active in a particular cancer cell are arrayed in a linear sequence that corresponds to the sequence in which information is relayed through a signal transduction pathway within a cell. The capture antibodies specific for one or more components of a given signal transduction pathway active in a particular cancer cell can also be printed in a randomized fashion to minimize any surface-related artifacts. Examples of such a multiplexed immunoarray, include a Collaborative Enzyme Enhanced Reactive ImmunoAssay (CEER), also known as the Collaborative Proximity Immunoassay (COPIA). CEER is described in the following patent documents U.S. Patent No. 8,163,499; PCT Publication Nos. WO 2008/036802, WO 2009/012140, WO 2009/108637, WO 2010/132723, WO 2011/008990, WO 2011/050069; and PCT Application Nos. PCT/US2011/66624, filed December 21, 2011, PCT/US12/27574, filed March 2, 2012, and PCT/US2012/53505, filed August 31, 2012. The methods of the present invention use a proximity assay which is a CEER.

In certain aspects of the disclosure, the expression level and/or activation state of one or more (*e.g.,* a plurality) of signal transduction analyte(s) in a cellular extract of cancer cells is detected using an immunoassay such as an ELISA. Suitable ELISA kits for determining the presence or level of a signal transduction analyte are, *e.g.,* Antigenix America Inc. (Huntington Station, NY), Promega (Madison, WI), R&D Systems, Inc. (Minneapolis, MN), Invitrogen (Camarillo, CA), CHEMICON International, Inc. (Temecula, CA), Neogen Corp. (Lexington, KY), PeproTech (Rocky Hill, NJ), Alpco Diagnostics (Salem, NH), Pierce Biotechnology, Inc. (Rockford, IL), and/or Abazyme (Needham, MA).

### 1. Single Detection Assays

In some aspects, the assay for detecting the expression and/or activation level of a particular analyte (*e.g.,* a signal transduction molecule such as a component of the PI3K signaling pathway, MEK signaling pathway, HER2 signaling pathway, HER3 signaling pathway, and/or other receptor tyrosine kinase signaling pathway) of interest in a cellular extract of cells such as tumor cells is a multiplex, high-throughput two-antibody assay having superior dynamic range. As a non-limiting example, the two antibodies used in the assay can comprise: (1) a capture antibody specific for the analyte; and (2) a detection antibody specific for an activated form of the analyte (*i.e.,* activation state-dependent antibody). The activation state-dependent antibody is capable of detecting, for example, the phosphorylation, ubiquitination, and/or complexation state of the analyte. Alternatively, the detection antibody comprises an activation state-independent antibody, which detects the total amount of the analyte in the cellular extract.

In one particular aspect, the two-antibody assay for detecting the expression or activation level of an analyte of interest comprises:
(i) incubating the cellular extract with one or a plurality of dilution series of capture antibodies to form a plurality of captured analytes;
(ii) incubating the plurality of captured analytes with detection antibodies specific for the corresponding analytes to form a plurality of detectable captured analytes, wherein the detection antibodies comprise activation state-dependent antibodies for detecting the activation (*e.g.,* phosphorylation) level of the analyte or activation state-independent antibodies for detecting the expression level (*e.g.,* total amount) of the analyte;
(iii) incubating the plurality of detectable captured analytes with first and second members of a signal amplification pair to generate an amplified signal; and
(iv) detecting the amplified signal generated from the first and second members of the signal amplification pair.

The capture antibodies and detection antibodies are preferably selected to minimize competition between them with respect to analyte binding (*i.e.,* both capture and detection antibodies can simultaneously bind their corresponding signal transduction molecules).

In one aspect, the detection antibodies are conjugated to a sulfhydryl-activated dextran molecule. In some aspects, the sulfhydryl-activated dextran molecule has a molecular weight of 500 kDa.

In one aspect, the detection antibodies comprise a first member of a binding pair (*e.g.,* biotin) and the first member of the signal amplification pair comprises a second member of the binding pair (*e.g.,* streptavidin). The binding pair members can be coupled directly or indirectly to the detection antibodies or to the first member of the signal amplification pair using methods well-known in the art. In certain instances, the first member of the signal amplification pair is a peroxidase (*e.g.,* horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, *etc*.), and the second member of the signal amplification pair is a tyramide reagent (*e.g.,* biotin-tyramide). In these instances, the amplified signal is generated by peroxidase oxidization of the tyramide reagent to produce an activated tyramide in the presence of hydrogen peroxide (H₂O₂).

The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores include, but are not limited to, an Alexa Fluor® dye (*e.g.,* Alexa Fluor® 555), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The streptavidin label can be coupled directly or indirectly to the fluorophore or peroxidase using methods well-known in the art. Non-limiting examples of chromogenic reagents include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

An exemplary protocol for performing the two-antibody assays described herein is provided in PCT Publication No. WO2009/108637.

In another aspect of a two-antibody approach, the present disclosure provides a method for detecting the expression or activation level of a truncated receptor, the method comprising:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with a dilution series of one or a plurality of capture antibodies specific for an intracellular domain (ICD) binding region of the full-length receptor to form a plurality of captured truncated receptors;
(iv) incubating the plurality of captured truncated receptors with detection antibodies specific for an ICD binding region of the full-length receptor to form a plurality of detectable captured truncated receptors, wherein the detection antibodies comprise activation state-dependent antibodies for detecting the activation (*e.g.,* phosphorylation) level of the truncated receptor or activation state-independent antibodies for detecting the expression level (*e.g.,* total amount) of the truncated receptor;
(v) incubating the plurality of detectable captured truncated receptors with first and second members of a signal amplification pair to generate an amplified signal; and
(vi) detecting an amplified signal generated from the first and second members of the signal amplification pair.

In certain aspects, the truncated receptor is p95HER2 and the full-length receptor is HER2. In certain other aspects, the plurality of beads specific for an extracellular domain (ECD) binding region comprises a streptavidin-biotin pair, wherein the biotin is attached to the bead and the biotin is attached to an antibody (*e.g.,* wherein the antibody is specific for the ECD binding region of the full-length receptor).

PCT Publication No. WO2009/108637 shows that beads coated with an antibody directed to the extracellular domain (ECD) of a receptor of interest binds the full-length receptor (*e.g.,* HER2), but not the truncated receptor (*e.g.,* p95HER2) to remove any full-length receptor from the assay. PCT Publication No. WO2009/108637 shows that the truncated receptor (*e.g.,* p95HER2), once bound to a capture antibody, may then be detected by a detection antibody that is specific for the intracellular domain (ICD) of the full-length receptor (*e.g.,* HER2). The detection antibody may be directly conjugated to horseradish peroxidase (HRP). Tyramide signal amplification (TSA) may then be performed to generate a signal to be detected. The expression level or activation state of the truncated receptor (*e.g.,* p95HER2) can be interrogated to determine, *e.g.,* its total concentration or its phosphorylation state, ubiquitination state, and/or complexation state.

### 2. Dual Detection Assays

In one aspect, the present disclosure provides a method for detecting and/or quantitating homo- or heterodimerization of receptor tyrosine kinases (RTK) including, but not limited to, HER1/HER2 dimers, HER1/HER3 dimers, HER2/HER3 dimers, HER2/HER2 dimers, HER2/HER4 dimers, p95HER2/HER3 dimers, p95HER2/HER2 dimers, and the like. A homodimer is formed by two identical molecules such as HER2/HER2 in a process called homodimerization, whereas a heterodimer is formed by two different macromolecules such as HER1/HER3 in a process called heterodimerization. In this aspect, the proximity assay comprises 3 antibodies: (1) a capture antibody specific for one member of the dimer pair; (2) a first detection antibody specific for a first member of the dimer pair, wherein the first detection antibody is specific for a different domain than the capture antibody; and a (3) a second detection antibody specific for a second member of the dimer pair. CEER technology relating to detecting RTK dimerization in cancer cells are described in U.S. Patent Publication Nos. 2008/0261829, 2009/0035792, 2010/0167945, 2011/0071042 and 2011/0281748.

In another aspect, the present disclosure provides a method for detecting and/or quantitating the amount of PI3K complexation and the amount of activation and/or phosphorylation of a PI3K complex. The PI3K complex comprises i) a dimerized receptor tyrosine kinase pair; ii) a PI3K p85 subunit and a PI3K p110 (*e.g.,* α or β) subunit. The assay comprises 3 antibodies: (1) a capture antibody specific for either the PI3K p85 or the PI3K p110 subunit; (2) a first detection antibody specific for a first member of the dimer pair, or PI3K subunit, wherein the first detection antibody is specific for a different domain than the capture antibody and wherein the PI3K subunit may be activated; and (3) a second detection antibody specific for a second member of the dimer pair or a PI3K subunit

In one particular aspect, the proximity assay for measuring (*e.g.,* detecting and quantitating) the level of a PI3K complex, wherein the PI3K complex comprises a) a dimerized receptor tyrosine kinase pair; b) a PI3K p85 subunit and a PI3K p110 subunit, comprises:
(i) incubating a cellular extract with one or a plurality of dilution series of capture antibodies to form a plurality of captured analytes;
(ii) incubating the plurality of captured analytes with first detection antibodies comprising either a) a first or a plurality of first activation state-independent antibodies specific for one member of a dimerized receptor tyrosine kinase pair or b) a PI3K p110 subunit; and second detection antibodies comprising either a) second or a plurality of second activation state-independent antibodies specific for a) one member of a dimerized receptor tyrosine kinase pair, a PI3K p85 or a PI3K p110 subunit or b) activation state-dependent antibodies specific for a PI3K p85 subunit and/or a PI3K p110 subunit to form a plurality of detectable captured dimerized and complexed analytes,
   wherein the first detection antibodies are labeled with a facilitating moiety, the second detection antibodies are labeled with a first member of a signal amplification pair, and the facilitating moiety generates an oxidizing agent which channels to and reacts with the first member of the signal amplification pair;
(iii) incubating the plurality of detectable captured dimerized analytes with a second member of the signal amplification pair to generate an amplified signal; and
(iv) detecting the amplified signal generated from the first and second members of the signal amplification pair, wherein the amount of the amplified signal is correlative to the amount of PI3K complex in the cellular extract.

In some aspects, the level of PI3K complex is calibrated against a standard curve generated for said PI3K complex comprising i) dimerization of at least two receptor tyrosine kinases (RTKs); ii) a PI3K p85 subunit and a PI3K p110 subunit.

In some aspects, the level of PI3K complex activation is determined by a) comparing the amount of phospho-PI3K to the total level of PI3K present in the sample, and b) establishing a ratio of activated PI3K complex to total PI3K. The level of the PI3K complex activation is determined based on the ratio. In some instances, the level of the PI3K complex activation is below a cut-off threshold, which indicates that the subject would not benefit from PI3K inhibitor treatment. In some instances, the level of the PI3K complex activation is above the cut-off threshold, which indicates that the subject would benefit from PI3K inhibitor treatment.

In some aspects, the level of PI3K complex activation (*e.g.,* phosphorylated PI3K complex) indicates that a PI3K inhibitor alone should be selected for the subject. In other aspects, the level of PI3K complex activation indicates that a combination therapy comprising a PI3K inhibitor and another anticancer drug should be selected for the subject.

CEER technology relating to detecting PI3K complexation in tumor cells are described in International Patent Application No. PCT/US2012/053505, filed Aug. 31,2012.

In some aspects, determining the activation status of a specific signal transduction analyte includes generating a ratio of the amount of the activated analyte to the amount of the total analyte. For instance, a ratio of activated PI3K to total PI3K represents the activation status of PI3K in a sample. A ratio of the activated analyte of interest to the total analyte allows for comparison between samples with differential expression of the analyte of interest.

In some aspects, the activation status of a set of signal transduction analytes from a patient sample is transformed into a pathway profile by one or more statistical algorithm.

In some aspects, the assay for detecting the expression and/or activation level of a particular analyte (*e.g.,* a signal transduction molecule such as a component of the HER2 signaling pathway, HER3 signaling pathway, and/or other receptor tyrosine kinase signaling pathway) of interest in a cellular extract of cells such as tumor cells comprises a multiplex, high-throughput proximity (*i.e.,* three-antibody) assay having superior dynamic range. As a non-limiting example, the three antibodies used in the proximity assay can comprise: (1) a capture antibody specific for the analyte; (2) a detection antibody specific for an activated form of the analyte (*i.e.,* activation state-dependent antibody); and (3) a detection antibody which detects the total amount of the analyte (*i.e.,* activation state-independent antibody). The activation state-dependent antibody is capable of detecting, *e.g.,* the phosphorylation, ubiquitination, and/or complexation state of the analyte. The activation state-independent antibody is capable of detecting the total amount of the analyte. In particular aspects, the proximity assays described herein are termed Collaborative Enzyme Enhanced Reactive-immunoassay (CEER).

In one particular aspect, the proximity assay (*e.g.,* CEER) for detecting the activation level or status of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of activation state-independent antibodies and one or a plurality of activation state-dependent antibodies specific for the corresponding analytes to form a plurality of detectable captured analytes,
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular aspect, the proximity assay (*e.g.,* CEER) for detecting the activation level or status of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of activation state-independent antibodies and one or a plurality of activation state-dependent antibodies specific for an ICD binding region of the full-length receptor to form a plurality of detectable captured truncated receptors,
   wherein the activation state-independent antibodies are labeled with a facilitating moiety, the activation state-dependent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain aspects, the truncated receptor is p95HER2 and the full-length receptor is HER2. In certain other aspects, the plurality of beads specific for an extracellular domain (ECD) binding region comprises a streptavidin-biotin pair, wherein the biotin is attached to the bead and the biotin is attached to an antibody (*e.g.,* wherein the antibody is specific for the ECD binding region of the full-length receptor).

In one aspect, the detection antibodies are conjugated to a sulfhydryl-activated dextran molecule. The sulfhydryl-activated dextran molecule typically has a molecular weight of about 500 kDa (e.g., about 250, 300, 350, 400, 450, 500, 550, 600, 650, 700, or 750 kDa).

In alternative aspects, the activation state-dependent antibodies can be labeled with a facilitating moiety and the activation state-independent antibodies can be labeled with a first member of a signal amplification pair.

As another non-limiting example, the three antibodies used in the proximity assay described herein (*e.g.,* CEER) can comprise: (1) a capture antibody specific for the analyte; (2) a first detection antibody specific which detects the total amount of the analyte (*i.e.,* a first activation state-independent antibody); and (3) a second detection antibody which detects the total amount of the analyte (*i.e.,* a second activation state-independent antibody). In preferred aspects, the first and second activation state-independent antibodies recognize different (*e.g.,* distinct) epitopes on the analyte.

In one particular aspect, the proximity assay (*e.g.,* CEER) for detecting the expression level of an analyte of interest comprises:
(i) incubating the cellular extract with one or a plurality of dilution series of capture antibodies to form a plurality of captured analytes;
(ii) incubating the plurality of captured analytes with detection antibodies comprising one or a plurality of first and second activation state-independent antibodies specific for the corresponding analytes to form a plurality of detectable captured analytes,
   wherein the first activation state-independent antibodies are labeled with a facilitating moiety, the second activation state-independent antibodies are labeled with a first member of a signal amplification pair, and the facilitating moiety generates an oxidizing agent which channels to and reacts with the first member of the signal amplification pair;
(iii) incubating the plurality of detectable captured analytes with a second member of the signal amplification pair to generate an amplified signal; and
(iv) detecting the amplified signal generated from the first and second members of the signal amplification pair.

In another particular aspect, the proximity assay (*e.g.,* CEER) for detecting the expression level of an analyte of interest that is a truncated receptor comprises:
(i) incubating the cellular extract with a plurality of beads specific for an extracellular domain (ECD) binding region of a full-length receptor;
(ii) removing the plurality of beads from the cellular extract, thereby removing the full-length receptor to form a cellular extract devoid of the full-length receptor;
(iii) incubating the cellular extract devoid of the full-length receptor with one or a plurality of capture antibodies specific for an intracellular domain (ICD) binding region of the full-length receptor to form a plurality of captured truncated receptors;
(iv) incubating the plurality of captured truncated receptors with detection antibodies comprising one or a plurality of first and second activation state-independent antibodies specific for an ICD binding region of the full-length receptor to form a plurality of detectable captured truncated receptors,
   wherein the first activation state-independent antibodies are labeled with a
   facilitating moiety, the second activation state-independent antibodies are labeled with a first member of a signal amplification pair, and the facilitating moiety generates an oxidizing agent which channels to and reacts with the first member of the signal amplification pair;
(v) incubating the plurality of detectable captured truncated receptors with a second member of the signal amplification pair to generate an amplified signal; and
(vi) detecting the amplified signal generated from the first and second members of the signal amplification pair.

In certain aspects, the truncated receptor is p95HER2 and the full-length receptor is HER2. In certain other aspects, the plurality of beads specific for an extracellular domain (ECD) binding region comprises a streptavidin-biotin pair, wherein the biotin is attached to the bead and the biotin is attached to an antibody (*e.g.,* wherein the antibody is specific for the ECD binding region of the full-length receptor).

In alternative aspects, the first activation state-independent antibodies can be labeled with a first member of a signal amplification pair and the second activation state-independent antibodies can be labeled with a facilitating moiety.

The proximity assays described herein are typically antibody-based arrays which comprise one or a plurality of different capture antibodies at a range of capture antibody concentrations that are coupled to the surface of a solid support in different addressable locations. Examples of suitable solid supports are described above.

The capture antibodies, activation state-independent antibodies, and activation state-dependent antibodies are preferably selected to minimize competition between them with respect to analyte binding (*i.e.,* all antibodies can simultaneously bind their corresponding signal transduction molecules).

In some aspects, activation state-independent antibodies for detecting activation levels of one or more of the analytes or, alternatively, first activation state-independent antibodies for detecting expression levels of one or more of the analytes further comprise a detectable moiety. In such instances, the amount of the detectable moiety is correlative to the amount of one or more of the analytes in the cellular extract. Examples of detectable moieties include, but are not limited to, fluorescent labels, chemically reactive labels, enzyme labels, radioactive labels, and the like. Preferably, the detectable moiety is a fluorophore such as an Alexa Fluor® dye (*e.g.,* Alexa Fluor® 647), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The detectable moiety can be coupled directly or indirectly to the activation state-independent antibodies using methods well-known in the art.

In certain instances, activation state-independent antibodies for detecting activation levels of one or more of the analytes or, alternatively, first activation state-independent antibodies for detecting expression levels of one or more of the analytes are directly labeled with the facilitating moiety. The facilitating moiety can be coupled to activation state-independent antibodies using methods well-known in the art. A suitable facilitating moiety includes any molecule capable of generating an oxidizing agent which channels to (*i.e.,* is directed to) and reacts with (*i.e.,* binds, is bound by, or forms a complex with) another molecule in proximity (*i.e.,* spatially near or close) to the facilitating moiety. Examples of facilitating moieties include, without limitation, enzymes such as glucose oxidase or any other enzyme that catalyzes an oxidation/reduction reaction involving molecular oxygen (O₂) as the electron acceptor, and photosensitizers such as methylene blue, rose bengal, porphyrins, squarate dyes, phthalocyanines, and the like. Non-limiting examples of oxidizing agents include hydrogen peroxide (H₂O₂), a singlet oxygen, and any other compound that transfers oxygen atoms or gains electrons in an oxidation/reduction reaction. Preferably, in the presence of a suitable substrate (*e.g.,* glucose, light, *etc*.), the facilitating moiety (*e.g.,* glucose oxidase, photosensitizer, *etc*.) generates an oxidizing agent (*e.g.,* hydrogen peroxide (H₂O₂), single oxygen, *etc*.) which channels to and reacts with the first member of the signal amplification pair (*e.g.,* horseradish peroxidase (HRP), hapten protected by a protecting group, an enzyme inactivated by thioether linkage to an enzyme inhibitor, *etc*.) when the two moieties are in proximity to each other.

In certain instances, the facilitating moiety and the activation state-independent antibodies can be conjugated to a sulfhydryl-activated dextran molecule. The sulfhydryl-activated dextran molecule typically has a molecular weight of about 500 kDa (e.g., about 250, 300, 350, 400, 450, 500, 550, 600, 650, 700, or 750 kDa).

In certain other instances, activation state-independent antibodies for detecting activation levels of one or more of the analytes or, alternatively, first activation state-independent antibodies for detecting expression levels of one or more of the analytes are indirectly labeled with the facilitating moiety via hybridization between an oligonucleotide linker conjugated to the activation state-independent antibodies and a complementary oligonucleotide linker conjugated to the facilitating moiety. The oligonucleotide linkers can be coupled to the facilitating moiety or to the activation state-independent antibodies using methods well-known in the art. In some aspects, the oligonucleotide linker conjugated to the facilitating moiety has 100% complementarity to the oligonucleotide linker conjugated to the activation state-independent antibodies. In other aspects, the oligonucleotide linker pair comprises at least one, two, three, four, five, six, or more mismatch regions, *e.g.,* upon hybridization under stringent hybridization conditions. One skilled in the art will appreciate that activation state-independent antibodies specific for different analytes can either be conjugated to the same oligonucleotide linker or to different oligonucleotide linkers.

The length of the oligonucleotide linkers that are conjugated to the facilitating moiety or to the activation state-independent antibodies can vary. In general, the linker sequence can be at least about 5, 10, 15, 20, 25, 30, 35, 40, 45, 50, 75, or 100 nucleotides in length. Typically, random nucleic acid sequences are generated for coupling. As a non-limiting example, a library of oligonucleotide linkers can be designed to have three distinct contiguous domains: a spacer domain; signature domain; and conjugation domain. Preferably, the oligonucleotide linkers are designed for efficient coupling without destroying the function of the facilitating moiety or activation state-independent antibodies to which they are conjugated.

The oligonucleotide linker sequences can be designed to prevent or minimize any secondary structure formation under a variety of assay conditions. Melting temperatures are typically carefully monitored for each segment within the linker to allow their participation in the overall assay procedures. Generally, the range of melting temperatures of the segment of the linker sequence is between 1-10°C. Computer algorithms (*e.g.,* OLIGO 6.0) for determining the melting temperature, secondary structure, and hairpin structure under defined ionic concentrations can be used to analyze each of the three different domains within each linker. The overall combined sequences can also be analyzed for their structural characterization and their comparability to other conjugated oligonucleotide linker sequences, *e.g.,* whether they will hybridize under stringent hybridization conditions to a complementary oligonucleotide linker.

The spacer region of the oligonucleotide linker provides adequate separation of the conjugation domain from the oligonucleotide crosslinking site. The conjugation domain functions to link molecules labeled with a complementary oligonucleotide linker sequence to the conjugation domain via nucleic acid hybridization. The nucleic acid-mediated hybridization can be performed either before or after antibody-analyte (*i.e.,* antigen) complex formation, providing a more flexible assay format. Unlike many direct antibody conjugation methods, linking relatively small oligonucleotides to antibodies or other molecules has minimal impact on the specific affinity of antibodies towards their target analyte or on the function of the conjugated molecules.

In some aspects, the signature sequence domain of the oligonucleotide linker can be used in complex multiplexed protein assays. Multiple antibodies can be conjugated with oligonucleotide linkers with different signature sequences. In multiplex immunoassays, reporter oligonucleotide sequences labeled with appropriate probes can be used to detect cross-reactivity between antibodies and their antigens in the multiplex assay format.

Oligonucleotide linkers can be conjugated to antibodies or other molecules using several different methods. For example, oligonucleotide linkers can be synthesized with a thiol group on either the 5' or 3' end. The thiol group can be deprotected using reducing agents (*e.g.,* TCEP-HCl) and the resulting linkers can be purified by using a desalting spin column. The resulting deprotected oligonucleotide linkers can be conjugated to the primary amines of antibodies or other types of proteins using heterobifunctional cross linkers such as SMCC. Alternatively, 5'-phosphate groups on oligonucleotides can be treated with water-soluble carbodiimide EDC to form phosphate esters and subsequently coupled to amine-containing molecules. In certain instances, the diol on the 3'-ribose residue can be oxidized to aldehyde groups and then conjugated to the amine groups of antibodies or other types of proteins using reductive amination. In certain other instances, the oligonucleotide linker can be synthesized with a biotin modification on either the 3' or 5' end and conjugated to streptavidin-labeled molecules.

Oligonucleotide linkers can be synthesized using any of a variety of techniques known in the art, such as those described in Usman et al., J. Am. Chem. Soc., 109:7845 (1987); Scaringe et al., Nucl. Acids Res., 18:5433 (1990); Wincott et al., Nucl. Acids Res., 23:2677-2684 (1995); and Wincott et al., Methods Mol. Bio., 74:59 (1997). In general, the synthesis of oligonucleotides makes use of common nucleic acid protecting and coupling groups, such as dimethoxytrityl at the 5'-end and phosphoramidites at the 3'-end. Suitable reagents for oligonucleotide synthesis, methods for nucleic acid deprotection, and methods for nucleic acid purification are known to those of skill in the art.

In certain instances, activation state-dependent antibodies for detecting activation levels of one or more of the analytes or, alternatively, second activation state-independent antibodies for detecting expression levels of one or more of the analytes are directly labeled with the first member of the signal amplification pair. The signal amplification pair member can be coupled to activation state-dependent antibodies to detect activation levels or second activation state-independent antibodies to detect expression levels using methods well-known in the art. In certain other instances, activation state-dependent antibodies or second activation state-independent antibodies are indirectly labeled with the first member of the signal amplification pair via binding between a first member of a binding pair conjugated to the activation state-dependent antibodies or second activation state-independent antibodies and a second member of the binding pair conjugated to the first member of the signal amplification pair. The binding pair members (*e.g.,* biotin/streptavidin) can be coupled to the signal amplification pair member or to the activation state-dependent antibodies or second activation state-independent antibodies using methods well-known in the art. Examples of signal amplification pair members include, but are not limited to, peroxidases such horseradish peroxidase (HRP), catalase, chloroperoxidase, cytochrome c peroxidase, eosinophil peroxidase, glutathione peroxidase, lactoperoxidase, myeloperoxidase, thyroid peroxidase, deiodinase, and the like. Other examples of signal amplification pair members include haptens protected by a protecting group and enzymes inactivated by thioether linkage to an enzyme inhibitor.

In one example of proximity channeling, the facilitating moiety is glucose oxidase (GO) and the first member of the signal amplification pair is horseradish peroxidase (HRP). When the GO is contacted with a substrate such as glucose, it generates an oxidizing agent (*i.e.,* hydrogen peroxide (H₂O₂)). If the HRP is within channeling proximity to the GO, the H₂O₂ generated by the GO is channeled to and complexes with the HRP to form an HRP-H₂O₂ complex, which, in the presence of the second member of the signal amplification pair (*e.g.,* a chemiluminescent substrate such as luminol or isoluminol or a fluorogenic substrate such as tyramide (*e.g.,* biotin-tyramide), homovanillic acid, or 4-hydroxyphenyl acetic acid), generates an amplified signal. Methods of using GO and HRP in a proximity assay are described in, *e.g.,* Langry et al., U.S. Dept. of Energy Report No. UCRL-ID-136797 (1999). When biotin-tyramide is used as the second member of the signal amplification pair, the HRP-H₂O₂ complex oxidizes the tyramide to generate a reactive tyramide radical that covalently binds nearby nucleophilic residues. The activated tyramide is either directly detected or detected upon the addition of a signal-detecting reagent such as, for example, a streptavidin-labeled fluorophore or a combination of a streptavidin-labeled peroxidase and a chromogenic reagent. Examples of fluorophores include, but are not limited to, an Alexa Fluor® dye (*e.g.,* Alexa Fluor® 555), fluorescein, fluorescein isothiocyanate (FITC), Oregon Green™; rhodamine, Texas red, tetrarhodamine isothiocynate (TRITC), a CyDye™ fluor (*e.g.,* Cy2, Cy3, Cy5), and the like. The streptavidin label can be coupled directly or indirectly to the fluorophore or peroxidase using methods well-known in the art. Non-limiting examples of chromogenic reagents include 3,3',5,5'-tetramethylbenzidine (TMB), 3,3'-diaminobenzidine (DAB), 2,2'-azino-bis(3-ethylbenzothiazoline-6-sulfonic acid) (ABTS), 4-chloro-1-napthol (4CN), and/or porphyrinogen.

In another example of proximity channeling, the facilitating moiety is a photosensitizer and the first member of the signal amplification pair is a large molecule labeled with multiple haptens that are protected with protecting groups that prevent binding of the haptens to a specific binding partner (*e.g.,* ligand, antibody, *etc*.). For example, the signal amplification pair member can be a dextran molecule labeled with protected biotin, coumarin, and/or fluorescein molecules. Suitable protecting groups include, but are not limited to, phenoxy-, analino-, olefin-, thioether-, and selenoether-protecting groups. Additional photosensitizers and protected hapten molecules are described in U.S. Patent No. 5,807,675. When the photosensitizer is excited with light, it generates an oxidizing agent (*i.e.,* singlet oxygen). If the hapten molecules are within channeling proximity to the photosensitizer, the singlet oxygen generated by the photosensitizer is channeled to and reacts with thioethers on the protecting groups of the haptens to yield carbonyl groups (ketones or aldehydes) and sulphinic acid, releasing the protecting groups from the haptens. The unprotected haptens are then available to specifically bind to the second member of the signal amplification pair (*e.g.,* a specific binding partner that can generate a detectable signal). For example, when the hapten is biotin, the specific binding partner can be an enzyme-labeled streptavidin. Exemplary enzymes include alkaline phosphatase, β-galactosidase, HRP, *etc.* After washing to remove unbound reagents, the detectable signal can be generated by adding a detectable (*e.g.,* fluorescent, chemiluminescent, chromogenic, *etc*.) substrate of the enzyme and detected using suitable methods and instrumentation known in the art. Alternatively, the detectable signal can be amplified using tyramide signal amplification and the activated tyramide either directly detected or detected upon the addition of a signal-detecting reagent as described above.

In yet another example of proximity channeling, the facilitating moiety is a photosensitizer and the first member of the signal amplification pair is an enzyme-inhibitor complex. The enzyme and inhibitor (*e.g.,* phosphonic acid-labeled dextran) are linked together by a cleavable linker (*e.g.,* thioether). When the photosensitizer is excited with light, it generates an oxidizing agent (*i.e.,* singlet oxygen). If the enzyme-inhibitor complex is within channeling proximity to the photosensitizer, the singlet oxygen generated by the photosensitizer is channeled to and reacts with the cleavable linker, releasing the inhibitor from the enzyme, thereby activating the enzyme. An enzyme substrate is added to generate a detectable signal, or alternatively, an amplification reagent is added to generate an amplified signal.

In a further example of proximity channeling, the facilitating moiety is HRP, the first member of the signal amplification pair is a protected hapten or an enzyme-inhibitor complex as described above, and the protecting groups comprise p-alkoxy phenol. The addition of phenylenediamine and H₂O₂ generates a reactive phenylene diimine which channels to the protected hapten or the enzyme-inhibitor complex and reacts with p-alkoxy phenol protecting groups to yield exposed haptens or a reactive enzyme. The amplified signal is generated and detected as described above (*see, e.g.,* U.S. Patent Nos. 5,532,138 and 5,445,944).

An exemplary protocol for performing the proximity assays described herein is provided in PCT Publication No. WO2009/108637.

In another aspect, the present disclosure provides kits for performing the proximity assays described above comprising: (a) a dilution series of one or a plurality of capture antibodies restrained on a solid support; and (b) one or a plurality of detection antibodies (*e.g.,* a combination of activation state-independent antibodies and activation state-dependent antibodies for detecting activation levels and/or a combination of first and second activation state-independent antibodies for detecting expression levels). In some instances, the kits can further contain instructions for methods of using the kit to detect the expression and/or activation status of one or a plurality of signal transduction molecules of cells such as tumor cells. The kits may also contain any of the additional reagents described above with respect to performing the specific methods of the present disclosure such as, for example, first and second members of the signal amplification pair, tyramide signal amplification reagents, substrates for the facilitating moiety, wash buffers, *etc.*

### 3. Antibodies

The methods described herein utilize antibodies for detecting, measuring, and quantitating the level of expression and/or the degree of activation (e.g., phosphorylation or complexation) of signal transduction analytes (e.g., HER1, HER2, HER3, cMET, IGF-1R, PI3K, AKT, ERK, MEK, p70S6K, PDK1, PRAS40, PTEN, RPS6, and SHC). Antibodies are commercially available from, for example, Millipore, Promega (Madison, WI), R&D Systems, Inc. (Minneapolis, MN), Life Technologies (Carlsbad, CA), CHEMICON International, Inc. (Temecula, CA), Santa Cruz Biotechnology (Santa Cruz, CA), Thermo Fisher Scientific (Waltham, MA), Abcam (Cambridge, MA), and Lab Vision (Kalamazoo, MI).

In some aspects, antibodies useful for the detecting of the activation status of signal transduction analytes include antibodies that recognize (e.g., bind to or can form a complex with) activated signal transduction analytes (*e.g.,* phosphorylated amino acid residues of the analyte) including, but not limited to, p-HER2 (e.g., Y1248), p-ERK (e.g., T202/Y204), p-AKT (e.g., T308, S473), p-MEK (e.g., S217/221), p-SHC, p-PDK1, p-PTEN, p-P70S6K (e.g., T389 (T229)), p-PI3K (e.g., p85 subunit Y688), p-PRAS40 (e.g., T246), and p-RPS6 (e.g., S235/236).

Antibodies useful for the detection of PI3K or activated PI3K include antibodies that recognize the p85 alpha subunit, the N-SH2 epitope of p85, the N-SH3 epitope or p85, the p110 alpha catalytic subunit, and/or a peptide that corresponds to amino acids 1052-1068 of the human p110 subunit of PI3K. The antibody for activated PI3K may bind to the peptide of CGFAEPYNL-pY-SSLKELV (SEQ ID NO: 1).

Antibodies for the detection of HER family of receptor tyrosine kinases include antibodies that recognize the extracellular domain of the receptors, the c-terminus of the receptors, the cytoplasmic domain of the receptors, and peptides that corresponds to amino acids 1295-1323 or 1242-1255 of HER3. Antibodies for detecting a member of the HER family include those that bind to cell lines such as T47D, SKBR3, and MAD109.

Antibodies that recognize c-MET or activated c-MET includes those that bind to the extracellular domain (e.g., N-terminus) of c-MET, the cytoplasmic domain (e.g., C-terminus) of the protein, a non-phosphopeptide corresponding to the amino acid residues around Y1234 of c-MET, and a phosphopeptide corresponding to the amino acid residues around phospho Y1003 of c-MET.

Antibodies useful for detecting IGF-1R and activated IGF-1R include antibodies that recognize the α subunit of IGF-1R, the β subunit of IGF-1R, an epitope between amino acid 31-932 of IGF-1R, the extracellular domain of the protein, and phosphopeptides corresponding to the amino acid residues around phospho Y1158, phospho Y1158/Y1162/Y1163, phospho Y1162/Y1163, or phospho Y1161/Y1165/Y1166.

Antibodies useful for detecting AKT or activated AKT include those that recognize S473, T308, the N-terminus region of the protein (e.g., amino acids 1-149 and 2-480).

Antibodies for the detection of ERK or p-ERK include those that bind to phospho T202/Y204, phospho T185/Y187, and an epitope between amino acids 1-360 of ERK2.

Antibodies useful for detecting p70S6K or activated p70S6K include those that recognize phospho T229 or phospho T389 of p70S6K, and an epitope between amino acid 26-43 of p70S6K.

Antibodies useful for detecting PRAS40 or p-PRAS40 include antibodies that recognize phospho T246 of PRAS40, the full-length protein, and an epitope between amino acids 119-256 of PRAS40.

Antibodies useful for detecting PDK1 or p-PDK1 include those that recognize phospho S241 of PDK1 and epitopes of PDK1 between amino acids 1-556 or 411-556.

Antibodies useful for detecting RPS6 or activated RPS6 include those that recognize an epitope between 1-249 of RPS6, and phospho S235/S236 of RPS6.

Antibodies useful for detecting SHC or p-SHC include antibodies that recognize phospho S36, phospho Y239 or phospho Y317 of SHC, the SH2 domain of the protein, and epitopes between amino acids 379-470, 488-579 of SHC.

Antibodies that can be used for detecting PTEN or p-PTEN include antibodies that recognize phospho S370, phospho S380, or phospho S385 of PTEN, the full-length protein, the C-terminal domain of PTEN, and epitopes between amino acids 2-403, 239-403, 385-403 of PTEN.

Antibodies useful for the detection of MEK or p-MEK include antibodies that recognize an epitope between amino acids 2-393 of MEK1, phospho S218/222 of MEK, and the N-terminus of the protein.

### B. Pathway Profiling

In some aspects, the methods include generating one or more pathway profiles: a reference pathway profile and a test pathway profile, which are then compared to determine the effect of a particular treatment regimen. A detailed description of generating pathway profiles for selecting an anticancer drug for the treatment of breast tumor is found in U.S. Patent No. 8,163,499.

In some aspects, a reference pathway profile is generated using a sample obtained from a patient having a specific type of cancer (*e.g.,* breast tumor, lung tumor, colorectal tumor, etc.) prior to anticancer drug treatment. Rare circulating cells derived from the cancerous tumor are isolated from the sample using, *e.g.,* immunomagnetic separation techniques which are known to those skilled in the art or filtration separation techniques, *e.g.,* as described in International Application No. PCT/US2012/02549, filed Feb. 16, 2012. The isolated circulating cells are stimulated in vitro with one or more growth factors. The stimulated cells are then lysed to produce a cellular extract. The cellular extract is applied to an assay (*e.g.,* CEER assay) that detects the activation states each signal transduction analyte of interest that may be altered in the patient's type of cancer. A reference pathway profile is thus generated providing the activation states of signal transduction molecules in the patient's in the absence of any anticancer drugs.

In some aspects, a test pathway profile is generated using a second sample obtained from the patient having the specific type of cancer (*e.g.,* breast tumor, lung tumor, colorectal tumor, etc.) either prior to anticancer drug treatment or after administration of an anticancer drug (*e.g.,* at any time throughout the course of cancer treatment). Rare circulating cells derived from the cancerous tumor are isolated from the sample. If isolated cells are obtained from a patient who has not received treatment with an anticancer drug, the isolated cells are incubated with anticancer drugs which target one or more of the activated signal transduction molecules determined from the reference pathway profile described above. For example, if it is determined from the reference pathway profile that PI3K is activated, then the cells can be incubated with a PI3K inhibitor alone or in combination with another anticancer drug. The isolated cells can then be stimulated in vitro with one or more growth factors. The isolated cells are then lysed to produce a cellular extract. The cellular extract is applied to an assay (*e.g.,* CEER assay) that detects the activation states each signal transduction analyte of interest. A test pathway profile for the patient is thus generated providing the activation states of signal transduction molecules in the patient's cancer in the presence of specific anticancer drugs.

The anticancer drugs are determined to be suitable or unsuitable for treatment of the patient's cancer by comparing the test pathway profile to the reference pathway profile. For example, if drug treatment causes most or all of the signal transduction molecules to be substantially less activated than in the absence of the drugs, *e.g.,* a change from strong activation without the drugs to weak, very weak, or less activation with the drugs, then the treatment is determined to be suitable for the patient's cancer. In such instances, treatment is either initiated with the suitable anticancer drug in a patient who has not received drug therapy or subsequent treatment is continued with the suitable anticancer drug in a patient already receiving the drug. However, if the drug treatment is deemed unsuitable for treatment of the patient's cancer, different drugs are selected and used to generate a new test pathway profile, which is then compared to the reference activation profile. In such instances, treatment is either initiated with a suitable anticancer drug in a patient who has not received drug therapy or subsequent treatment is changed to a suitable anticancer drug in a patient currently receiving the unsuitable drug.

In one aspect, a reference pathway profile and test pathway profile are generated using cells of a cancer cell line. For a reference pathway profile, the cells are stimulated in vitro with one or more growth factors. The stimulated cells are then lysed to produce a cellular extract. The cellular extract is applied to an assay (*e.g.,* CEER assay) that detects the activation states each signal transduction analyte of interest that may be altered in the cell line in the absence of any anticancer drug. A test pathway profile is generated using a second sample from the same cell line. The cells are incubated with one or more anticancer drugs. In some aspects, the drug targets one or more of the activated signal transduction molecules determined from the reference pathway profile described above. For example, if it is determined from the reference pathway profile that PI3K is activated, then the cells can be incubated with a PI3K inhibitor alone or in combination with another anticancer drug. The isolated cells are then stimulated in vitro with one or more growth factors factors, and then lysed to produce a cellular extract. The cellular extract is applied an assay (*e.g.,* CEER assay) that detects the activation states each signal transduction analyte of interest. A test pathway profile for the cell line is thus generated providing the activation states of signal transduction molecules in the presence of specific anticancer drugs. By comparing the change in the activation status between the reference and test pathway profiles, it can be determined whether the anticancer drug inhibits the activation of specific analytes. It can also be determined if the drug induces a compensatory signal transduction pathway.

In some aspects, a reference pathway profile is generated using cells of a cancer cell line and a test pathway profile is generated using a sample obtained from the patient having the specific type of cancer (*e.g.,* breast tumor, lung tumor, colorectal tumor, prostate tumor, gastric tumor, pancreatic tumor, etc.) either prior to anticancer drug treatment or after administration of an anticancer drug (*e.g.,* at any time throughout the course of cancer treatment). By comparing the change in the activation status between the reference and test pathway profiles, it can be determined whether the anticancer drug inhibits the activation of specific analytes.

In some aspects, the present disclosure provides methods for selecting anticancer drug therapy, optimizing anticancer drug therapy, and/or monitoring the efficacy of anticancer drug treatment by applying a statistical algorithm to one or more (*e.g.,* a combination of 2, 3, 4, 5, 6, 7, 8, 9, 10, 11, 12, 13, 14, 15, 16, 17, 18, 19, 20, 25, 30, 35, 40, 45, 50, 55, 60, or more) pathway analytes to generate a pathway profile. In particular aspects, quantile analysis is applied to the expression or activation status of one or more markers to guide treatment decisions for patients receiving anticancer drug therapy. In other aspects, one or a combination of two of more learning statistical classifier systems are applied to the expression or activation status of one or more markers to guide treatment decisions for patients receiving anticancer drug therapy. The statistical analyses advantageously provide improved sensitivity, specificity, negative predictive value, positive predictive value, and/or overall accuracy for selecting an initial anticancer drug therapy and for determining when or how to adjust or modify (*e.g.,* increase or decrease) the subsequent dose of an anticancer drug, to combine one or more an anticancer drugs (*e.g.,* at an increased, decreased, or same dose), and/or to change the current course of therapy (*e.g.,* switch to a different anticancer drug).

The term "statistical analysis" or "statistical algorithm" or "statistical process" includes any of a variety of statistical methods and models used to determine relationships between variables. In the present disclosure, the variables are the presence, level, or genotype of at least one marker of interest. Any number of markers can be analyzed using a statistical analysis described herein. For example, the presence or level of 1, 2, 3, 4, 5, 6, 7, 8, 9, 10, 11, 12, 13, 14, 15, 16, 17, 18, 19, 20, 25, 30, 35, 40, 45, 50, 55, 60, or more markers can be included in a statistical analysis. In one aspect, logistic regression is used. In another aspect, linear regression is used. In yet another aspect, ordinary least squares regression or unconditional logistic regression is used. In certain preferred aspects, the statistical analyses of the present disclosure comprise a quantile measurement of one or more markers, *e.g.,* within a given population, as a variable. Quantiles are a set of "cut points" that divide a sample of data into groups containing (as far as possible) equal numbers of observations. For example, quartiles are values that divide a sample of data into four groups containing (as far as possible) equal numbers of observations. The lower quartile is the data value a quarter way up through the ordered data set; the upper quartile is the data value a quarter way down through the ordered data set. Quintiles are values that divide a sample of data into five groups containing (as far as possible) equal numbers of observations. The present disclosure can also include the use of percentile ranges of marker levels (*e.g.,* tertiles, quartile, quintiles, *etc*.), or their cumulative indices (*e.g.,* quartile sums of marker levels to obtain quartile sum scores (QSS), *etc*.) as variables in the statistical analyses (just as with continuous variables).

In certain aspects, the present disclosure involves detecting or determining the presence, level (*e.g.,* magnitude), and/or genotype of one or more markers of interest using quartile analysis. In this type of statistical analysis, the level of a marker of interest is defined as being in the first quartile (<25%), second quartile (25-50%), third quartile (51%-<75%), or fourth quartile (75-100%) in relation to a reference database of samples. These quartiles may be assigned a quartile score of 1, 2, 3, and 4, respectively. In certain instances, a marker that is not detected in a sample is assigned a quartile score of 0 or 1, while a marker that is detected (*e.g.,* present) in a sample (*e.g.,* sample is positive for the marker) is assigned a quartile score of 4. In some aspects, quartile 1 represents samples with the lowest marker levels, while quartile 4 represent samples with the highest marker levels. In other aspects, quartile 1 represents samples with a particular marker genotype (*e.g.,* wild-type allele), while quartile 4 represent samples with another particular marker genotype (*e.g.,* allelic variant). The reference database of samples can include a large spectrum of patients with a solid tumor cancer. From such a database, quartile cut-offs can be established.

In some aspects, the statistical analyses of the present disclosure comprise one or more learning statistical classifier systems. As used herein, the term "learning statistical classifier system" includes a machine learning algorithmic technique capable of adapting to complex data sets (*e.g.,* panel of markers of interest) and making decisions based upon such data sets. In some aspects, a single learning statistical classifier system such as a decision/classification tree (*e.g.,* random forest (RF) or classification and regression tree (C&RT)) is used. In other aspects, a combination of 2, 3, 4, 5, 6, 7, 8, 9, 10, or more learning statistical classifier systems are used, preferably in tandem. Examples of learning statistical classifier systems include, but are not limited to, those using inductive learning (*e.g.,* decision/classification trees such as random forests, classification and regression trees (C&RT), boosted trees, *etc*.), Probably Approximately Correct (PAC) learning, connectionist learning (*e.g.,* neural networks (NN), artificial neural networks (ANN), neuro fuzzy networks (NFN), network structures, the Cox Proportional-Hazards Model (CPHM), perceptrons such as multi-layer perceptrons, multi-layer feed-forward networks, applications of neural networks, Bayesian learning in belief networks, *etc*.), reinforcement learning (*e.g.,* passive learning in a known environment such as naive learning, adaptive dynamic learning, and temporal difference learning, passive learning in an unknown environment, active learning in an unknown environment, learning action-value functions, applications of reinforcement learning, *etc*.), and genetic algorithms and evolutionary programming. Other learning statistical classifier systems include support vector machines (*e.g.,* Kernel methods), multivariate adaptive regression splines (MARS), Levenberg-Marquardt algorithms, Gauss-Newton algorithms, mixtures of Gaussians, gradient descent algorithms, and learning vector quantization (LVQ).

Random forests are learning statistical classifier systems that are constructed using an algorithm developed by Leo Breiman and Adele Cutler. Random forests use a large number of individual decision trees and decide the class by choosing the mode (*i.e.,* most frequently occurring) of the classes as determined by the individual trees. Random forest analysis can be performed, *e.g.,* using the RandomForests software available from Salford Systems (San Diego, CA). *See, e.g.,* Breiman, Machine Learning, 45:5-32 (2001); and http://stat-www.berkeley.edu/users/breiman/RandomForests/cc_home.htm, for a description of random forests.

Classification and regression trees represent a computer intensive alternative to fitting classical regression models and are typically used to determine the best possible model for a categorical or continuous response of interest based upon one or more predictors. Classification and regression tree analysis can be performed, *e.g.,* using the C&RT software available from Salford Systems or the Statistica data analysis software available from StatSoft, Inc. (Tulsa, OK). A description of classification and regression trees is found, *e.g.,* in Breiman et al. "Classification and Regression Trees," Chapman and Hall, New York (1984); and Steinberg et al., "CART: Tree-Structured Non-Parametric Data Analysis," Salford Systems, San Diego, (1995).

Neural networks are interconnected groups of artificial neurons that use a mathematical or computational model for information processing based on a connectionist approach to computation. Typically, neural networks are adaptive systems that change their structure based on external or internal information that flows through the network. Specific examples of neural networks include feed-forward neural networks such as perceptrons, single-layer perceptrons, multi-layer perceptrons, backpropagation networks, ADALINE networks, MADALINE networks, Learnmatrix networks, radial basis function (RBF) networks, and self-organizing maps or Kohonen self-organizing networks; recurrent neural networks such as simple recurrent networks and Hopfield networks; stochastic neural networks such as Boltzmann machines; modular neural networks such as committee of machines and associative neural networks; and other types of networks such as instantaneously trained neural networks, spiking neural networks, dynamic neural networks, and cascading neural networks. Neural network analysis can be performed, *e.g.,* using the Statistica data analysis software available from StatSoft, Inc. S*ee, e.g.,* Freeman et al., In "Neural Networks: Algorithms, Applications and Programming Techniques," Addison-Wesley Publishing Company (1991); Zadeh, Information and Control, 8:338-353 (1965); Zadeh, "IEEE Trans. on Systems, Man and Cybernetics," 3:28-44 (1973); Gersho et al., In "Vector Quantization and Signal Compression," Kluywer Academic Publishers, Boston, Dordrecht, London (1992); and Hassoun, "Fundamentals of Artificial Neural Networks," MIT Press, Cambridge, Massachusetts, London (1995), for a description of neural networks.

Support vector machines are a set of related supervised learning techniques used for classification and regression and are described, *e.g.,* in Cristianini et al., "An Introduction to Support Vector Machines and Other Kernel-Based Learning Methods," Cambridge University Press (2000). Support vector machine analysis can be performed, *e.g.,* using the SVM*^{light}* software developed by Thorsten Joachims (Cornell University) or using the LIBSVM software developed by Chih-Chung Chang and Chih-Jen Lin (National Taiwan University).

The various statistical methods and models described herein can be trained and tested using a cohort of samples (*e.g.,* circulating tumor cells, FNA, and biopsy) from healthy individuals and patients with cancer. For example, samples from patients diagnosed by a physician, preferably by an oncologist, as having cancer, or a clinical subtype thereof using a biopsy and the like, are suitable for use in training and testing the statistical methods and models of the present disclosure. Samples from healthy individuals can include those that were not identified as cancer samples. One skilled in the art will know of additional techniques and diagnostic criteria for obtaining a cohort of patient samples that can be used in training and testing the statistical methods and models of the present disclosure.

### C. Detecting Therapeutic Resistance

Single-agent targeted therapy can be ineffective due to feedback inhibition or activation of one or more parallel/compensatory signaling pathways. In some instances, inhibition of one signaling pathway leads to activation of another related or unrelated signaling pathway. The methods of the present disclosure are used for detecting the presence of feedback inhibition and/or activation of a compensatory signaling pathway due to single-agent drug therapy, and recommending the selection of a combination drug therapy for the treatment of cancer. For example, if drug treatment causes most or all of the signal transduction molecules in a first signaling pathway to be substantially less activated than in the absence of the drugs, *e.g.,* a change from strong activation without the drugs to weak, very weak, or less activation with the drugs, and causes most or all of the signal transduction molecule in second signaling pathway to be substantially more activated than in the absence of the drugs, *e.g.,* a change from weak activation without the drugs to strong or very strong activation with the drugs, then the drug treatment should include a combination drug therapy. For instance, the patient should receive a targeted drug therapy directed to the first signaling pathway as well as a targeted drug therapy directed to the second signaling pathway. In some instances, a single-agent drug can be used that targets multiple signaling pathways (e.g., multi-kinase or pan-kinase inhibitor). During the course of therapy, if the drug treatment is deemed unsuitable for treatment of the patient's cancer, different drugs are selected and used to generate a new test pathway profile, which is then compared to the reference activation profile.

In some aspects, the methods are useful for detecting an increased level of activated PI3K and AKT in a sample from a subject, and predicting that the subject will likely respond to PI3K inhibitor alone or PI3K inhibitor combination therapy for ameliorating cancer.

In some aspects, the methods are useful for detecting an increased level of activated PI3K signaling, and an elevated level of HER3 signaling in a sample from a patient receiving single-agent MEK inhibitor, and recommending that the patient receive a combination of MEK and PI3K inhibitor therapy. In some instances, MEK inhibitor relieves the ERK-mediated negative feedback mechanism on EGFR and HER2, in particular, the threonine phosphorylation of the juxtamembrane domains of EGFR (T669) and HER2 (T677), thereby activating the PI3K signaling pathway, increasing the formation of HER3 heterodimers (e.g., EGFR:HER3 and HER2:HER3) and driving phosphorylation of HER3.

In some aspects, the methods are used for detecting an increased level of activated MEK signaling and a decreased level of activated PI3K signaling in a sample from a patient receiving a PI3K inhibitor, and recommending that the patient receive a combination of PI3K and MEK inhibitor therapy.

In another aspect, the methods are used for detecting an increased level of activated HER2 signaling and a decreased level of activated PI3K signaling in a sample from a patient receiving a PI3K inhibitor, and recommending that the patient receive a combination of PI3K and HER2 inhibitor therapy.

In another aspect, the methods are used for detecting an increased level of activated HER3 signaling and a decreased level of activated PI3K signaling in a sample from a patient receiving a PI3K inhibitor, and recommending that the patient receive a combination of PI3K and HER3 inhibitor therapy.

In some aspects, the methods are used for detecting an increased level of activated PI3K signaling and a decreased level of activated MEK signaling in a sample from a patient receiving a MEK inhibitor, and recommending that the patient receive a combination of MEK and PI3K inhibitor therapy.

In some aspects, the methods are used for detecting an increased level of activated HER2 signaling and a decreased level of activated MEK signaling in a sample from a patient receiving a MEK inhibitor, and recommending that the patient receive a combination of MEK and HER2 inhibitor therapy.

In some aspects, the methods are used for detecting an increased level of activated HER3 signaling and a decreased level of activated MEK signaling in a sample from a patient receiving a MEK inhibitor, and recommending that the patient receive a combination of MEK and HER3 inhibitor therapy.

In other aspects, the methods are used for detecting an increased level of activated MEK signaling and a decreased level of activated HER2 signaling in a sample from a patient receiving a HER2 inhibitor, and recommending that the patient receive a combination of HER2 and MEK inhibitor therapy.

In yet another aspect, the methods are used for detecting an increased level of activated PI3K signaling and a decreased level of activated HER2 signaling in a sample from a patient receiving a HER2 inhibitor, and recommending that the patient receive a combination of HER2and PI3K inhibitor therapy.

In other aspects, the methods are used for detecting an increased level of activated MEK signaling and a decreased level of activated HER3 signaling in a sample from a patient receiving a HER3 inhibitor, and recommending that the patient receive a combination of HER3 and MEK inhibitor therapy.

In yet another aspect, the methods are used for detecting an increased level of activated PI3K signaling and a decreased level of activated HER3 signaling in a sample from a patient receiving a HER3 inhibitor, and recommending that the patient receive a combination of HER3 and PI3K inhibitor therapy.

### D. Methods of Administration

The anticancer drugs described herein may be administered to a subject by any convenient means known in the art. The methods of the disclosure can be used to determine, predict, identify, and/or monitor the response of a tumor (*e.g.,* a primary or metastatic breast tumor) to treatment with one or more anticancer drugs. The methods of the disclosure can also be used to select one or more suitable anticancer drugs for the treatment of a tumor (*e.g.,* a primary or metastatic breast tumor) in a subject. One of skill in the art will appreciate that the anticancer drug(s) can be administered alone or as part of a combined therapeutic approach with conventional chemotherapy, radiotherapy, hormonal therapy, immunotherapy, and/or surgery.

Optionally, the anticancer drug may comprise an anti-signaling agent (*i.e.,* a cytostatic drug) such as a monoclonal antibody or a tyrosine kinase inhibitor; an anti-proliferative agent; a chemotherapeutic agent (*i.e.,* a cytotoxic drug); a hormonal therapeutic agent; a radiotherapeutic agent; a vaccine; and/or any other compound with the ability to reduce or abrogate the uncontrolled growth of aberrant cells such as cancerous cells. The subject may be treated with one or more anti-signaling agents, anti-proliferative agents, and/or hormonal therapeutic agents in combination with at least one chemotherapeutic agent. Exemplary monoclonal antibodies, tyrosine kinase inhibitors, anti-proliferative agents, chemotherapeutic agents, hormonal therapeutic agents, radiotherapeutic agents, and vaccines are described herein.

In particular aspects, the anticancer drug comprises one or more compounds that modulate HER2 activity including monoclonal antibodies, tyrosine kinase inhibitors, and combinations thereof. Non-limiting examples of HER2-modulating compounds include monoclonal antibodies such as trastuzumab (Herceptin®), trastuzumab-DMl (trastuzumab emtansine), ertumaxomab and pertuzumab (2C4; Omnitarg™); small molecule tyrosine kinase inhibitors such as gefitinib (Iressa®), erlotinib (Tarceva®), pelitinib (Wyeth), CP-654577 (Pfizer), CP-724714 (Pfizer), canertinib /CI 1033/PD183805 (Parke-Davis/Pfizer), HKI-272 (Whyeth), lapatinib (GW-572016; Tykerb®), neratinib (Pfizer), PKI-166/CGP-75166 (Novartis), AEE788 (Novartis), BMS-599626 (Bristal Myers Squibb), HKI-727 (Wyeth), HKI-357 (Wyeth), BIBW 2992 (Boehringer Ingelheim), AG1478, Arry-380 (Array Biopharma), ARRY-334543 (Array Biopharma), Bay846, D69491 (Sugen), DXL-702(InNexus Biotechnology), JNJ-26483327;(Johnson and Johnson), and combinations thereof. In certain aspects, HER2-modulating compounds can be used in combination with one or more other anticancer drugs described herein or known to one of skill in the art.

In other aspects, the anticancer drug comprises one of more compounds that modulate activity of HER3 and other ErbB family members including monoclonal antibodies, tyrosine kinase inhibitors, and combinations thereof. Non-limiting examples of these compounds include monoclonal antibodies such as MM-121, AMG-888 (U3-1287), trastuzumab (Herceptin®), pertuzumab (2C4; Omnitarg™), cetuximab (Erbitux®), MEHD7945A/RG7597 (Roche), RG7116 (Roche); small molecule tyrosine kinase inhibitors such as gefitinib (Iressa®), erlotinib (Tarceva®), canertinib (CI 1033), lapatinib (GW-572016; Tykerb®), MP-470, AZD8931, PF00299804; and combinations thereof. In certain aspects, HER1-, HER2- and HER3-modulating compounds can be used in combination with one or more other anticancer drugs described herein or known to one of skill in the art.

The anticancer drug may comprise one or more compounds that modulate PI3K activity including monoclonal antibodies, inhibitors, and combinations thereof. Non-limiting examples of these compounds include inhibitors such as SF1126 (Semafore Pharmaceuticals), LY294002, XL147 (Exelixis/Sanofi), XL765 (Exelixis/Sanofi), NVP-BEZ235 (Novartis), NVP-BGT226 (Novartis), NVP-BKM120 (Novartis), NVP-BYL719 (Novartis), BAY80946 (Bayer), BAY841236 (Bayer), GDC-0941 (Genentech/ Piramed Pharma), GDC-0032/RG7604 (Genentech/Roche), GDC-0980 /RG7422 (Genentech/Roche), GDC-0941/RG7321 (Genentech/Roche), PX-866 (ProIX Pharmaceuticals), GSK1059615 (GlaxoSmithKline), GSK2126458 (GlaxoSmithKline) CAL-101 (Calistoga Pharmaceuticals), INK1117 (Intellikine), ZSTK474 (Zenyaku Kogyo Co.), PWT33597 (Pathway Therapeutics), AEZS-136 (Aeterna Zentaris), PKI-587 (Pfizer), PF-4691502 (Pfizer), PF-05212384 (Pfizer),), IPI-145 (Infinity Pharmaceuticals), and combinations thereof.

Examples of anti-signaling agents include, without limitation, monoclonal antibodies such as trastuzumab (Herceptin®), pertuzumab (2C4; Omnitarg™), alemtuzumab (Campath®), bevacizumab (Avastin®), cetuximab (Erbitux®), gemtuzumab (Mylotarg®), panitumumab (Vectibix™), rituximab (Rituxan®), and tositumomab (BEXXAR®); tyrosine kinase inhibitors such as gefitinib (Iressa®), sunitinib (Sutent®), erlotinib (Tarceva®), lapatinib (GW-572016; Tykerb®), canertinib (CI 1033), semaxinib (SU5416), vatalanib (PTK787/ZK222584), sorafenib (BAY 43-9006; Nexavar®), imatinib mesylate (Gleevec®), leflunomide (SU101), vandetanib (ZACTIMA™; ZD6474), pilitinib, CP-654577, CP-724714, HKI-272, PKI-166, AEE788, BMS-599626, HKI-357, BIBW 2992, ARRY-334543, JNJ-26483327, and JNJ-26483327; and combinations thereof.

Examples of MEK inhibitors suitable for use in the present invention include, without limitation, trametinib, BAY869766 (Bayer), MEK162 (Novartis), GDC-0973/RG7420 (Roche), GDC-0623/RG7421 (Roche), RG7167 (Roche), RG7304 (Roche), XL518 (Genentech), PD-325901 (Pfizer), and combinations thereof.

Exemplary anti-proliferative agents include mTOR inhibitors such as GDC-0980 (Genentech), OSI-027 (OSI Pharmaceuticals), AZD8055 (AstraZeneca), INK-128 (Intellikine), sirolimus (rapamycin), temsirolimus (CCI-779), and everolimus (RAD001); AKT inhibitors such as MK-2206 (Merck), GDC-0068/RG7440 (Genentech/Array BioPharma), GDC-0980 /RG7422 (Genentech/Roche), GSK2110183 (GlaxoSmithKline), SR13668, perifosine, 1L6-hydroxymethyl-chiro-inositol-2-(R)-2-O-methyl-3-O-octadecyl-*sn*-glycerocarbonate, 9-methoxy-2-methylellipticinium acetate, 1,3-dihydro-1-(1-((4-(6-phenyl-1H-imidazo[4,5-g]quinoxalin-7-yl)phenyl)methyl)-4-piperidinyl)-2H-benzimidazol-2-one, 10-(4'-(N-diethylamino)butyl)-2-chlorophenoxazine, 3-formylchromone thiosemicarbazone (Cu(II)Cl₂ complex), API-2, a 15-mer peptide derived from amino acids 10-24 of the proto-oncogene TCL1 (Hiromura et al., J. Biol. Chem., 279:53407-53418 (2004), KP372-1, and the compounds described in Kozikowski et al., J. Am. Chem. Soc., 125:1144-1145 (2003) and Kau et al., Cancer Cell, 4:463-476 (2003); and combinations thereof.

Non-limiting examples of chemotherapeutic agents include platinum-based drugs (*e.g.,* oxaliplatin, cisplatin, carboplatin, spiroplatin, iproplatin, satraplatin, *etc*.), alkylating agents (*e.g.,* cyclophosphamide, ifosfamide, chlorambucil, busulfan, melphalan, mechlorethamine, uramustine, thiotepa, nitrosoureas, *etc*.), antimetabolites (*e.g.,* 5-fluorouracil, azathioprine, 6-mercaptopurine, methotrexate, leucovorin, capecitabine, cytarabine, floxuridine, fludarabine, gemcitabine (Gemzar®), pemetrexed (ALIMTA®), raltitrexed, *etc*.), plant alkaloids (*e.g.,* vincristine, vinblastine, vinorelbine, vindesine, podophyllotoxin, paclitaxel (Taxol®), docetaxel (Taxotere®), *etc*.), topoisomerase inhibitors (*e.g.,* irinotecan, topotecan, amsacrine, etoposide (VP16), etoposide phosphate, teniposide, *etc*.), antitumor antibiotics (*e.g.,* doxorubicin, adriamycin, daunorubicin, epirubicin, actinomycin, bleomycin, mitomycin, mitoxantrone, plicamycin, *etc*.), pharmaceutically acceptable salts thereof, stereoisomers thereof, derivatives thereof, analogs thereof, and combinations thereof.

Examples of hormonal therapeutic agents include, without limitation, aromatase inhibitors (*e.g.,* aminoglutethimide, anastrozole (Arimidex®), letrozole (Femara®), vorozole, exemestane (Aromasin®), 4-androstene-3,6,17-trione (6-OXO), 1,4,6-androstatrien-3,17-dione (ATD), formestane (Lentaron®), *etc*.), selective estrogen receptor modulators (*e.g.,* bazedoxifene, clomifene, fulvestrant, lasofoxifene, raloxifene, tamoxifen, toremifene, *etc*.), steroids (*e.g.,* dexamethasone), finasteride, and gonadotropin-releasing hormone agonists (GnRH) such as goserelin, pharmaceutically acceptable salts thereof, stereoisomers thereof, derivatives thereof, analogs thereof, and combinations thereof.

Non-limiting examples of cancer vaccines include ANYARA from Active Biotech, DCVax-LB from Northwest Biotherapeutics, EP-2101 from IDM Pharma, GV1001 from Pharmexa, 10-2055 from Idera Pharmaceuticals, INGN 225 from Introgen Therapeutics and Stimuvax from Biomira/Merck.

Examples of radiotherapeutic agents include, but are not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ^{117m}Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

The anticancer drugs described herein may be co-administered with conventional immunotherapeutic agents including, but not limited to, immunostimulants (*e.g.,* Bacillus Calmette-Guérin (BCG), levamisole, interleukin-2, alpha-interferon, *etc*.), immunotoxins (*e.g.,* anti-CD33 monoclonal antibody-calicheamicin conjugate, anti-CD22 monoclonal antibody-pseudomonas exotoxin conjugate, *etc*.), and radioimmunotherapy (*e.g.,* anti-CD20 monoclonal antibody conjugated to ¹¹¹In, ⁹⁰Y, or ¹³¹I, *etc*.).

Anticancer drugs can be administered with a suitable pharmaceutical excipient as necessary and can be carried out via any of the accepted modes of administration. Thus, administration can be, for example, oral, buccal, sublingual, gingival, palatal, intravenous, topical, subcutaneous, transcutaneous, transdermal, intramuscular, intra-joint, parenteral, intra-arteriole, intradermal, intraventricular, intracranial, intraperitoneal, intravesical, intrathecal, intralesional, intranasal, rectal, vaginal, or by inhalation. By "co-administer" it is meant that an anticancer drug is administered at the same time, just prior to, or just after the administration of a second drug (*e.g.,* another anticancer drug, a drug useful for reducing the side-effects associated with anticancer drug therapy, a radiotherapeutic agent, a hormonal therapeutic agent, an immunotherapeutic agent, *etc*.).

A therapeutically effective amount of an anticancer drug may be administered repeatedly, e.g., at least 2, 3, 4, 5, 6, 7, 8, or more times, or the dose may be administered by continuous infusion. The dose may take the form of solid, semisolid, lyophilized powder, or liquid dosage forms, such as, for example, tablets, pills, pellets, capsules, powders, solutions, suspensions, emulsions, suppositories, retention enemas, creams, ointments, lotions, gels, aerosols, foams, or the like, preferably in unit dosage forms suitable for simple administration of precise dosages.

As used herein, the term "unit dosage form" refers to physically discrete units suitable as unitary dosages for human subjects and other mammals, each unit containing a predetermined quantity of an anticancer drug calculated to produce the desired onset, tolerability, and/or therapeutic effects, in association with a suitable pharmaceutical excipient (*e.g.,* an ampoule). In addition, more concentrated dosage forms may be prepared, from which the more dilute unit dosage forms may then be produced. The more concentrated dosage forms thus will contain substantially more than, *e.g.,* at least 1, 2, 3, 4, 5, 6, 7, 8, 9, 10, or more times the amount of the anticancer drug.

Methods for preparing such dosage forms are known to those skilled in the art (*see, e.g.,* REMINGTON'S PHARMACEUTICAL SCIENCES, 18TH ED., Mack Publishing Co., Easton, PA (1990)). The dosage forms typically include a conventional pharmaceutical carrier or excipient and may additionally include other medicinal agents, carriers, adjuvants, diluents, tissue permeation enhancers, solubilizers, and the like. Appropriate excipients can be tailored to the particular dosage form and route of administration by methods well known in the art (*see, e.g., REMINGTON'S PHARMACEUTICAL SCIENCES, supra*).

Examples of suitable excipients include, but are not limited to, lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, saline, syrup, methylcellulose, ethylcellulose, hydroxypropylmethylcellulose, and polyacrylic acids such as Carbopols, *e.g.,* Carbopol 941, Carbopol 980, Carbopol 981, *etc.* The dosage forms can additionally include lubricating agents such as talc, magnesium stearate, and mineral oil; wetting agents; emulsifying agents; suspending agents; preserving agents such as methyl-, ethyl-, and propyl-hydroxy-benzoates (*i.e.,* the parabens); pH adjusting agents such as inorganic and organic acids and bases; sweetening agents; and flavoring agents. The dosage forms may also comprise biodegradable polymer beads, dextran, and cyclodextrin inclusion complexes.

For oral administration, the therapeutically effective dose can be in the form of tablets, capsules, emulsions, suspensions, solutions, syrups, sprays, lozenges, powders, and sustained-release formulations. Suitable excipients for oral administration include pharmaceutical grades of mannitol, lactose, starch, magnesium stearate, sodium saccharine, talcum, cellulose, glucose, gelatin, sucrose, magnesium carbonate, and the like.

The therapeutically effective dose may take the form of a pill, tablet, or capsule, and thus, the dosage form can contain, along with an anticancer drug, any of the following: a diluent such as lactose, sucrose, dicalcium phosphate, and the like; a disintegrant such as starch or derivatives thereof; a lubricant such as magnesium stearate and the like; and a binder such a starch, gum acacia, polyvinylpyrrolidone, gelatin, cellulose and derivatives thereof. An anticancer drug can also be formulated into a suppository disposed, for example, in a polyethylene glycol (PEG) carrier.

Liquid dosage forms can be prepared by dissolving or dispersing an anticancer drug and optionally one or more pharmaceutically acceptable adjuvants in a carrier such as, for example, aqueous saline (*e.g.,* 0.9% w/v sodium chloride), aqueous dextrose, glycerol, ethanol, and the like, to form a solution or suspension, *e.g.,* for oral, topical, or intravenous administration. An anticancer drug can also be formulated into a retention enema.

For topical administration, the therapeutically effective dose can be in the form of emulsions, lotions, gels, foams, creams, jellies, solutions, suspensions, ointments, and transdermal patches. For administration by inhalation, an anticancer drug can be delivered as a dry powder or in liquid form via a nebulizer. For parenteral administration, the therapeutically effective dose can be in the form of sterile injectable solutions and sterile packaged powders. Preferably, injectable solutions are formulated at a pH of from about 4.5 to about 7.5.

The therapeutically effective dose can also be provided in a lyophilized form. Such dosage forms may include a buffer, *e.g.,* bicarbonate, for reconstitution prior to administration, or the buffer may be included in the lyophilized dosage form for reconstitution with, *e.g.,* water. The lyophilized dosage form may further comprise a suitable vasoconstrictor, *e.g.,* epinephrine. The lyophilized dosage form can be provided in a syringe, optionally packaged in combination with the buffer for reconstitution, such that the reconstituted dosage form can be immediately administered to a subject.

A subject can also be monitored at periodic time intervals to assess the efficacy of a certain therapeutic regimen. For example, the activation states of certain signal transduction molecules may change based on the therapeutic effect of treatment with one or more of the anticancer drugs described herein. The subject can be monitored to assess response and understand the effects of certain drugs or treatments in an individualized approach. Additionally, subjects who initially respond to a specific anticancer drug or combination of anticancer drugs may become refractory to the drug or drug combination, indicating that these subjects have developed acquired drug resistance. These subjects can be discontinued on their current therapy and an alternative treatment prescribed in accordance with the methods described herein.

In certain aspects, the methods described herein can be used in conjunction with panels of gene expression markers that predict the likelihood of breast cancer prognosis and/or recurrence in various populations of women with for example, node-negative disease. These gene panels can be useful for identifying women who are unlikely to experience recurrence and, thus, unlikely to benefit from adjuvant chemotherapy. The expression panels can be used to identify women who can safely avoid adjuvant chemotherapy, without negatively affecting disease-free and overall survival outcomes. Suitable systems include, but are not limited to, Oncotype DX™, which is a 21-gene panel from Genomic Health, Inc.; MammaPrint,® which is a 70-gene panel from Agendia; and a 76-gene panel from Veridex.

In addition, in certain other aspects, the methods described herein can be used in conjunction with panels of gene expression markers that identify the original tumors for cancers of unknown primary (CUP). These gene panels can be useful in identifying women with metastatic cancer who would benefit from therapy consistent with that given to women diagnosed initially with breast cancer. Suitable systems include, but are not limited to, the Aviara CancerTYPE ID assay, an RT-PCR-based expression assay that measures 92 genes to identify the primary site of origin for 39 tumor types; and the Pathwork® Tissue of Origin Test, which measures the expression of more than 1600 genes on a microarray and compares a tumor's gene expression "signature" against those of 15 known tissue types."

### EXAMPLES

The following examples are offered to illustrate, but not to limit the teachings described herein.

### Example 1: Using CEER to Profile Signal Transduction Analytes in Growth Factor Stimulated Cancer Cell Lines.

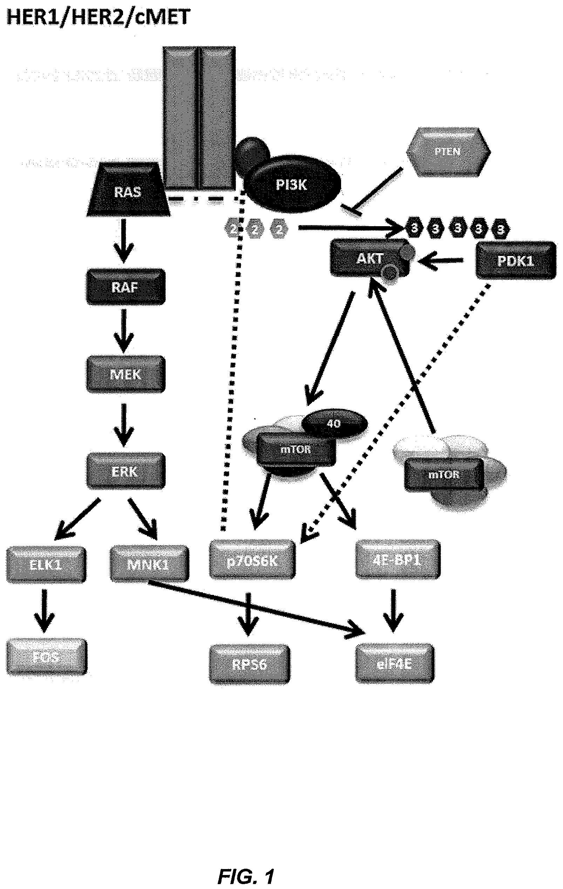

This example illustrates methods of using CEER to profile cancer pathway biomarkers including HER1, HER2, cMET, PI3K, AKT, ERK, PTEN, MEK, p70S6K, PRAS40, RPS6 and PDK1 (FIG. 1) in various cell lines. Detailed description of CEER is found in U.S. Patent No. 8,163,499, issued April 24, 2012.

With CEER, the levels of expression and/or activation of multiple signal transduction analytes can be determined from a sample of limited quantity or availability. For example, activated protein levels can be detected from a single hair follicle. The analytical sensitivity of the assay is less than 10 cells (*e.g.,* ≤10 pg) for some of signal transduction analytes (Table 1).

**Table 1. Analytical Sensitivity of Various Signal Transduction Analytes**

| **Marker** | **Analytical Sensitivity** | **Marker** | **Analytical Sensitivity** |
|---|---|---|---|
| HER1 | <1 cell | p70S6K | 3-10 cells (10 pg) |
| HER2 | <1 cell | RPS6 | 1 cell (10 pg) |
| cMET | <1 cell | MEK | 3-10 cells (10 pg) |
| PI3K | 3-10 cells | ERK | 3-10 cells (3 pg) |
| AKT | 1-3 cells (1 pg) | PDK1 | 1-3 cells (3 pg) |
| PRAS40 | 1-3 cells | PTEN | 125 cells (10 pg) |

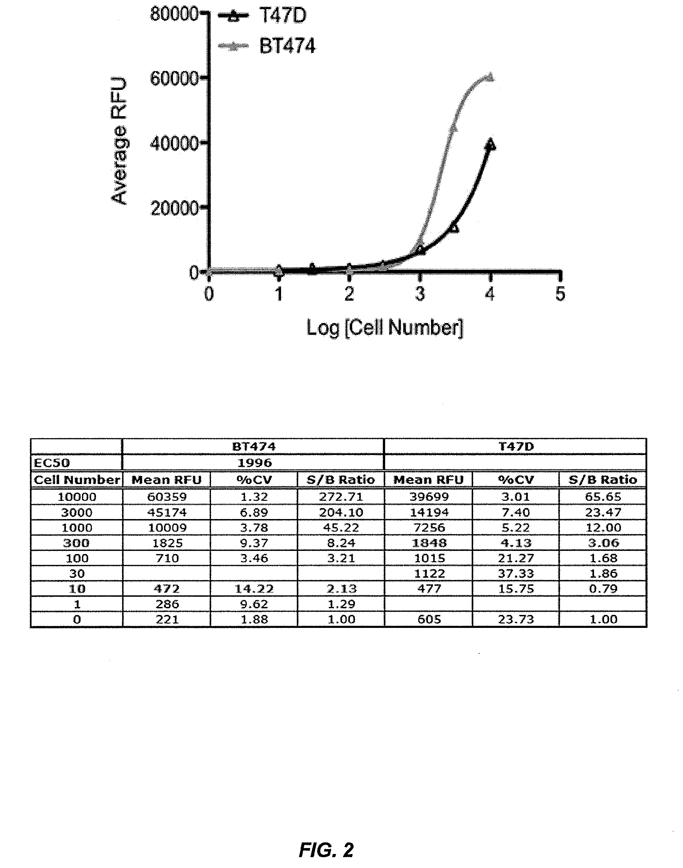

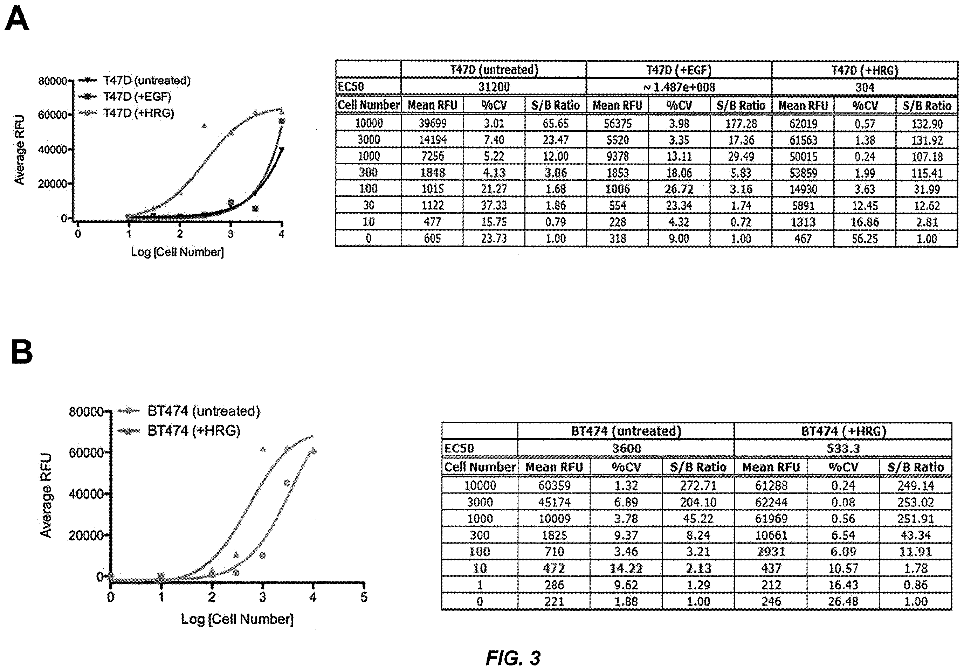

CEER has ultra-high sensitivity and specificity. As shown, it can simultaneously detect the activation state of multiple signal transduction proteins at the single cell level. The assay can be used to compare the activation status of an analyte of interest in multiple cell lines. For instance, activated PI3K complex was detected in two breast cancer cell lines T47D and BT474. The levels at baseline prior to growth factor stimulation are shown in FIG. 2. The levels after stimulation are displayed in FIG. 3. Both cell lines responded to heregulin (HRG) by activating PI3K (FIG. 3).

The expression and/or activation of signal transduction analytes in various cancer cell lines including those derived from lung, breast, skin, pancreas, stomach, bladder cancer, and myeloma, were analyzed. Table 2 shows some of the cell lines used in the experiment. The PI3K status, PTEN status, the presence of phospho-AKT, and the presence of somatic mutation (*e.g.,* EGFR or BRAF/KRAS mutation) varied among the cell lines.

**Table 2. Mutation/Detection Status of Markers in Various Cell Lines**

| **Cell Line** | **Type** | **PI3K Status** | **PTEN Status** | **pAKT** | **Other** |
|---|---|---|---|---|---|
| NCI-H1650 | Lung | Wild-type | Null | + | EGFR |
| ZR75 | Breast | Wild-type | L108R | | |
| A431 | Skin | Wild-type | Wild-type | | |
| MDA-MD-231 | Breast | Wild-type | Wild-type | | BRAF/KRAS |
| BT474 | Breast | K111N | Wild-type | ++ | |
| MDA-MD-468 | Breast | Wild-type | Null | +++ | |
| MCF7 | Breast | E545K | Wild-type | +++ | |
| HCC827 | Lung | Wild-type | Wild-type | + | EGFR |
| NCI-H1975 | Lung | Wild-type | Wild-type | | EGFR |
| BxPc3 | Pancreas | Wild-type | Wild-type | | |
| KPL4 | Breast | Mutant | Wild-type | ++ | |
| Kato III | Stomach | Wild-type | Wild-type | | |
| RT112 | Bladder | Wild-type | Wild-type | | |
| T47D | Breast | H1047 | Wild-type | ++ | |
| KMS11 | Myeloma | | Wild-type | | FGFR |

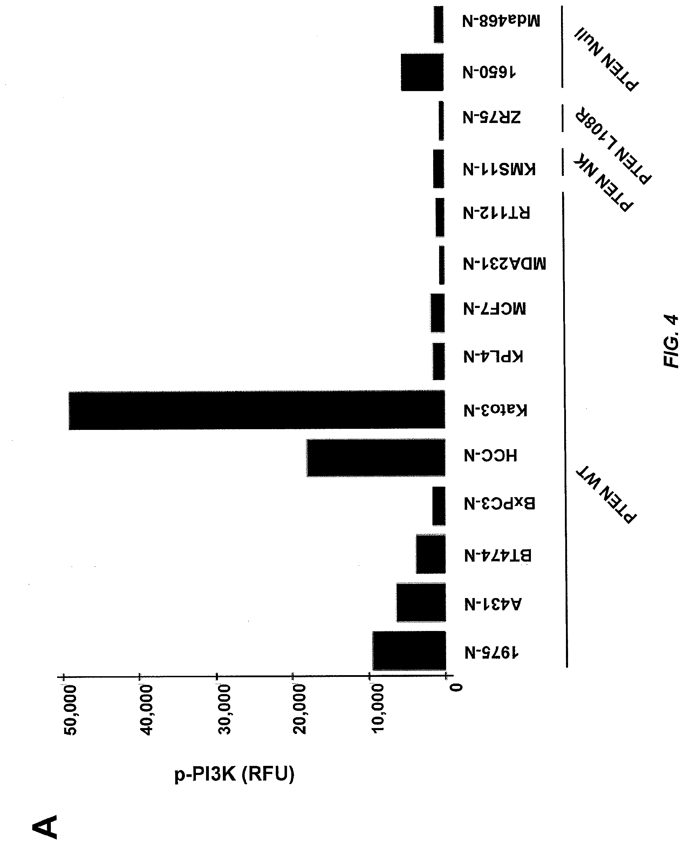

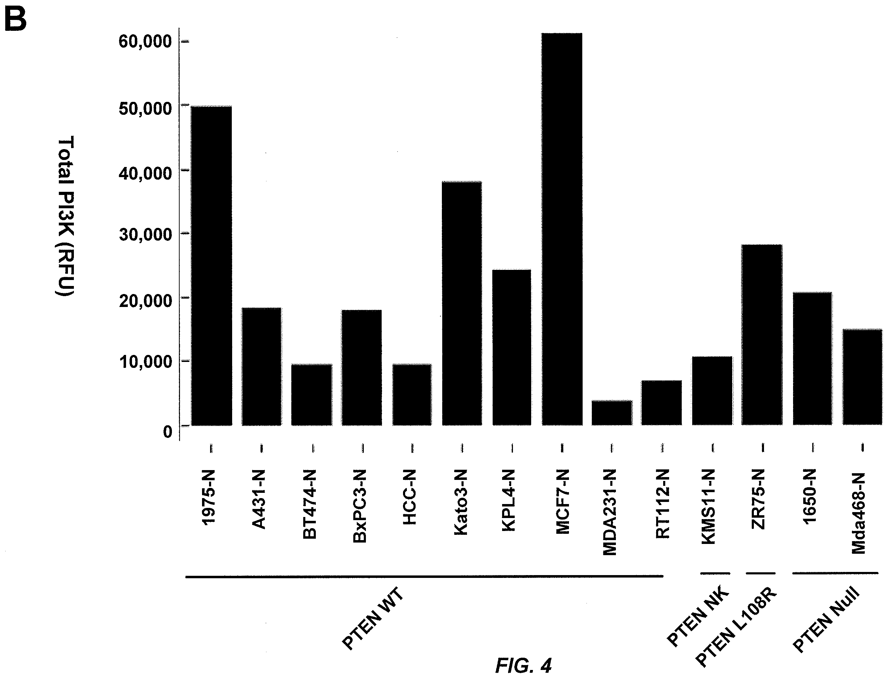

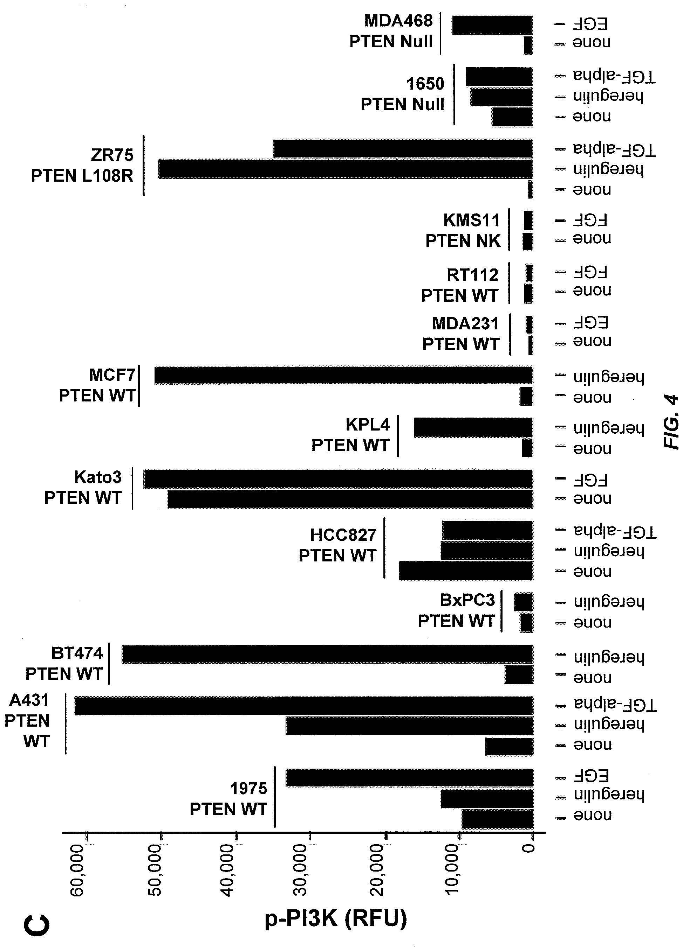

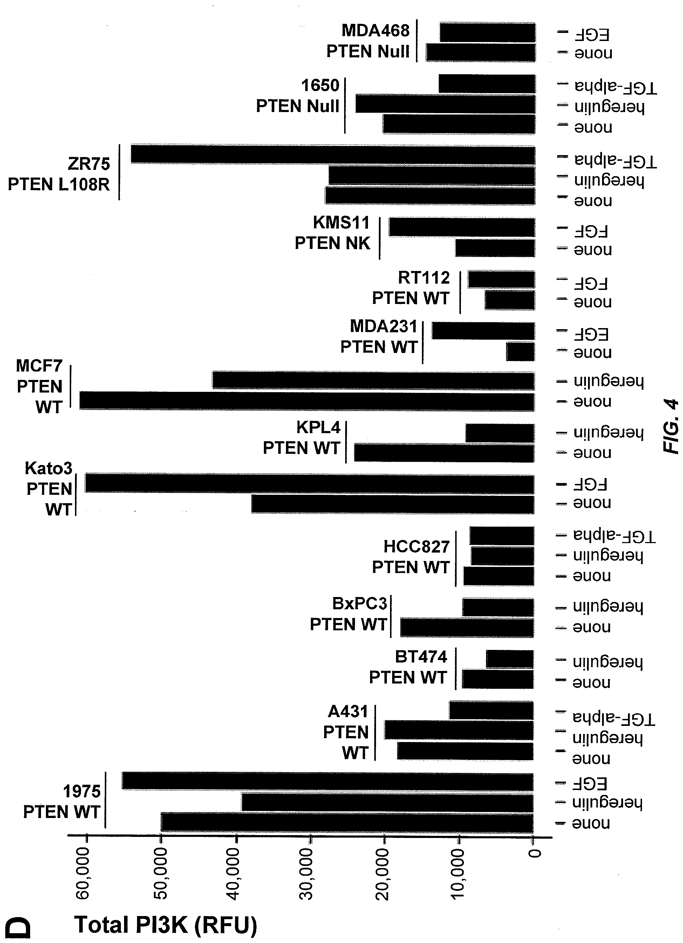

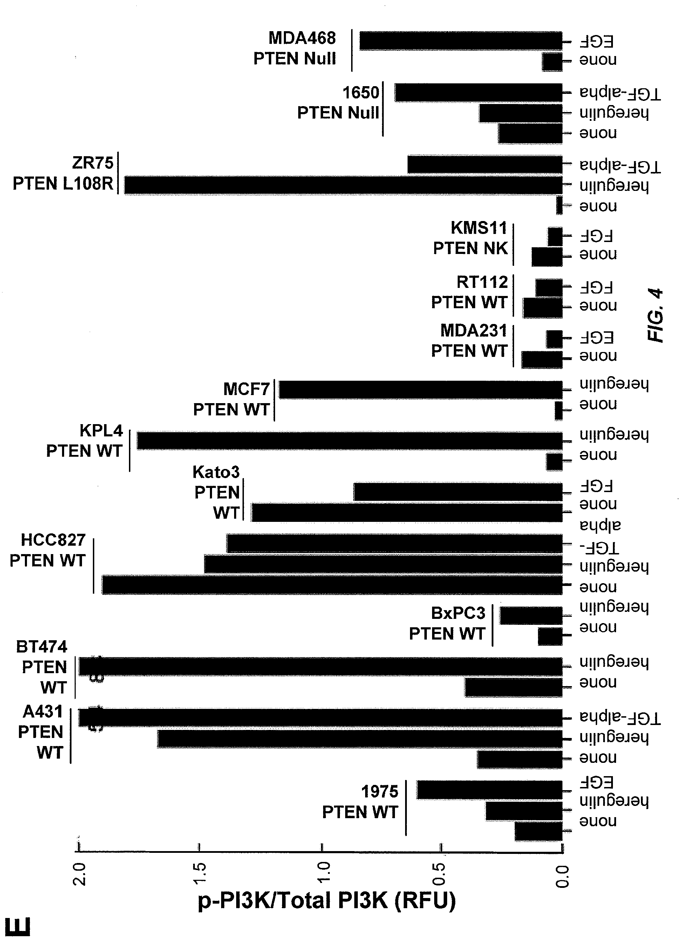

The levels of phospho-PI3K (FIG. 4A) and total PI3K (FIG. 4B), as detected by CEER, were different for each cell line. The effects of growth factor stimulation of the cells on phospho-PI3K (FIG. 4C), total PI3K protein (FIG. 4D), and the ratio of phospho-PI3K to total PI3K (FIG. 4E) were compared. HCC827 had a high p-PI3K/total PI3K ratio and displayed activated AKT, yet carries no PI3K mutations. The data also showed that PTEN status in certain cells did not correlate with PI3K activation.

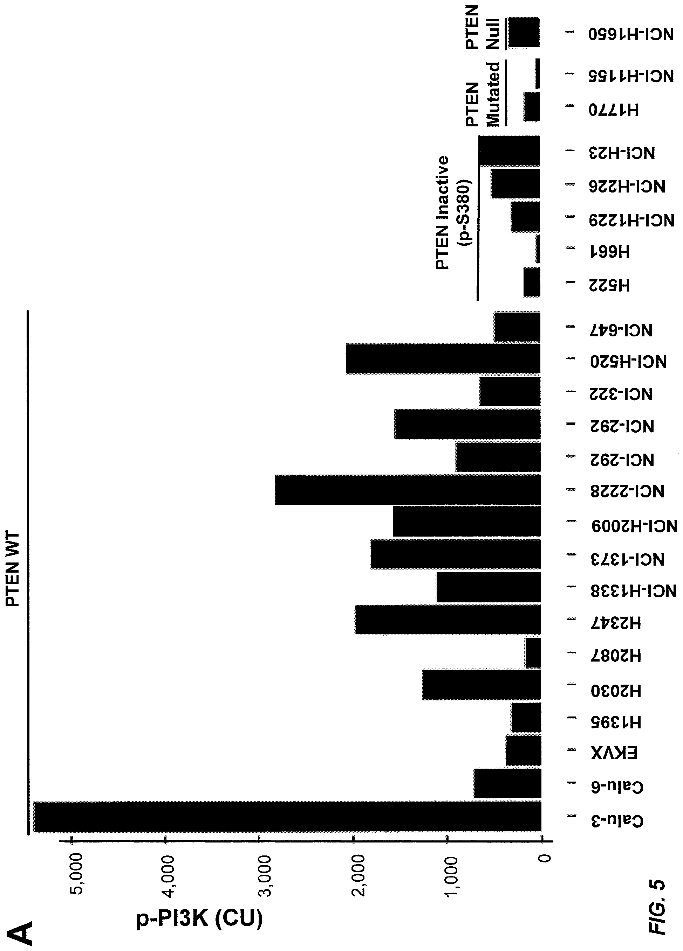

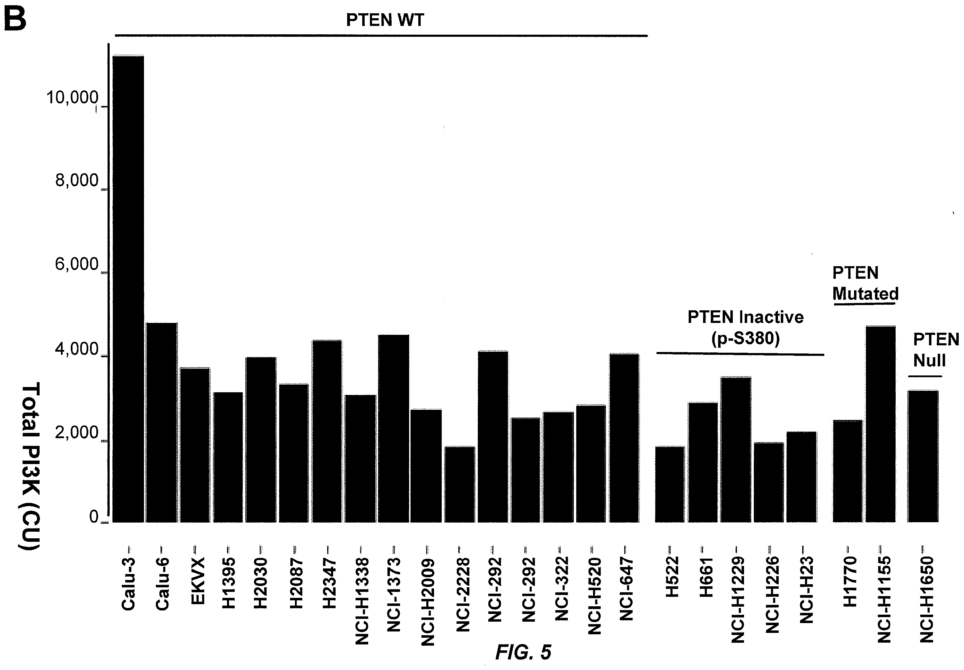

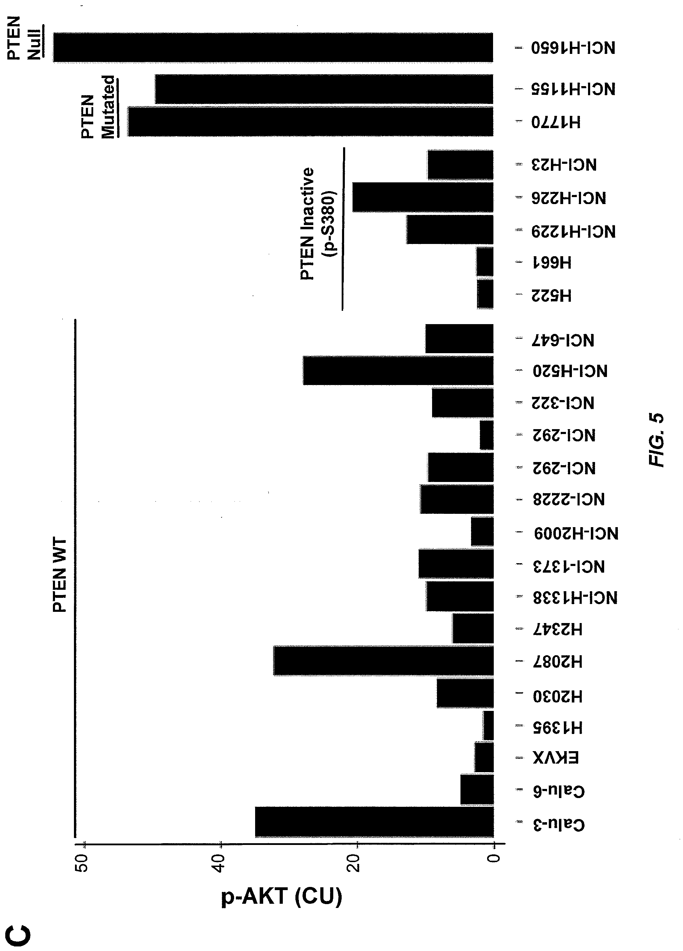

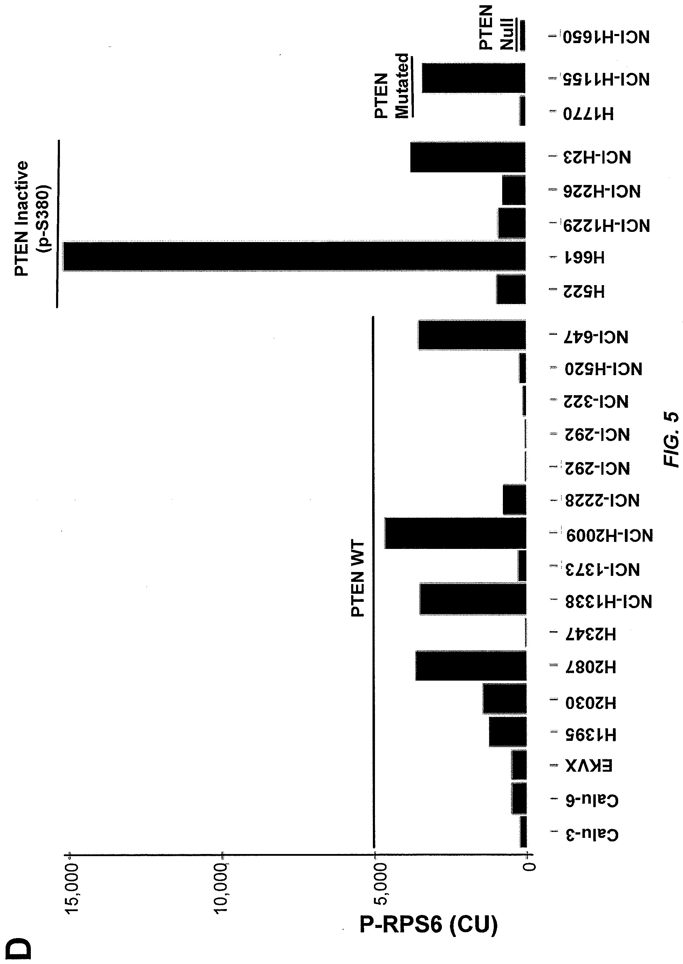

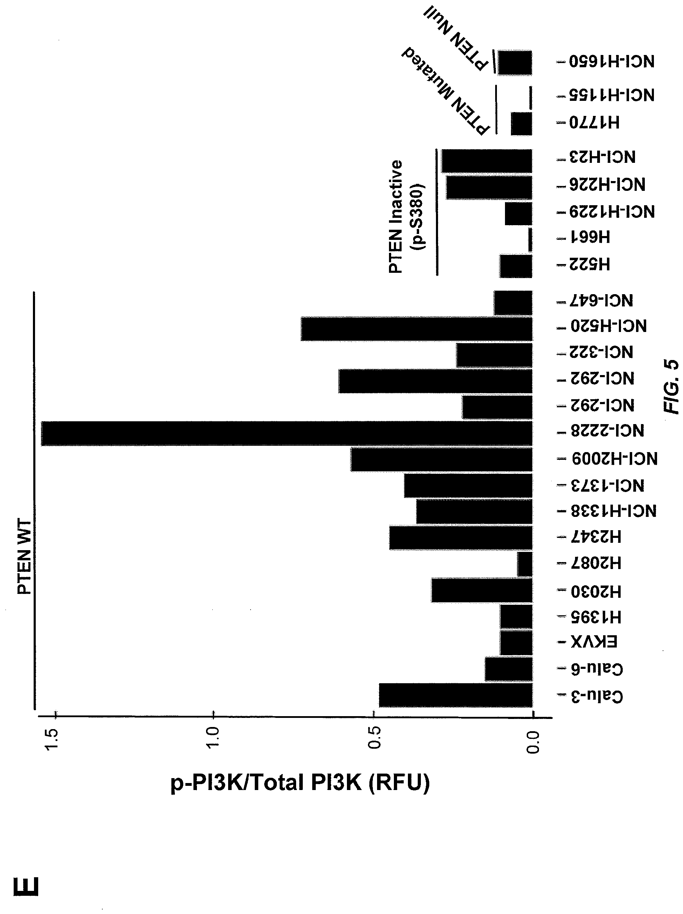

The analysis of cancer cell lines using CEER was expanded to include Calu-3, Calu-6, EKVX, H522, H661, H1395, H1651, H1770, H2030, H2087, H2347, NCI-H1338, NCI-H1373, NCI-H2009, NCI-H2228, NCI-H292, NCI-H520, NCI-H647, NCI-H1299, NCI-H226, NCI-H23, NCI-H1155, and NCI-H1650. The levels of phospho-PI3K (FIG. 5A), total PI3K (FIG. 5B), phospho-AKT (FIG. 5C), and phospho-RPS6 (FIG. 5D) were compared. The experiment showed that NCI-H2009 had the highest phospho-PI3K/total PI3K ratio compared to the other cell lines (FIG. 5E). The cell line Calu-3, which has a PI3KCA amplification mutation, had a lower phospho-PI3K/total PI3K ratio compared to other cell lines without a PI3K mutation, *e.g.,* NCI-H2009. This showed that PI3K mutation analysis cannot be used as the sole predictor of high levels of phospho-PI3K. The data illustrates that CEER is useful in determining the presence of activated signal transduction analytes.

### Example 2: Using CEER to Profile Signal Transduction Analytes in Inhibitor Treated Cancer Cell Lines.

This example illustrates that the methods described herein can be used to determine whether a specific cell is likely to respond to single inhibitor therapy, or alternatively combination inhibitor therapy. Using CEER technology, the PI3K pathway and other downstream signal transduction proteins were profiled in various cancer cell lines.

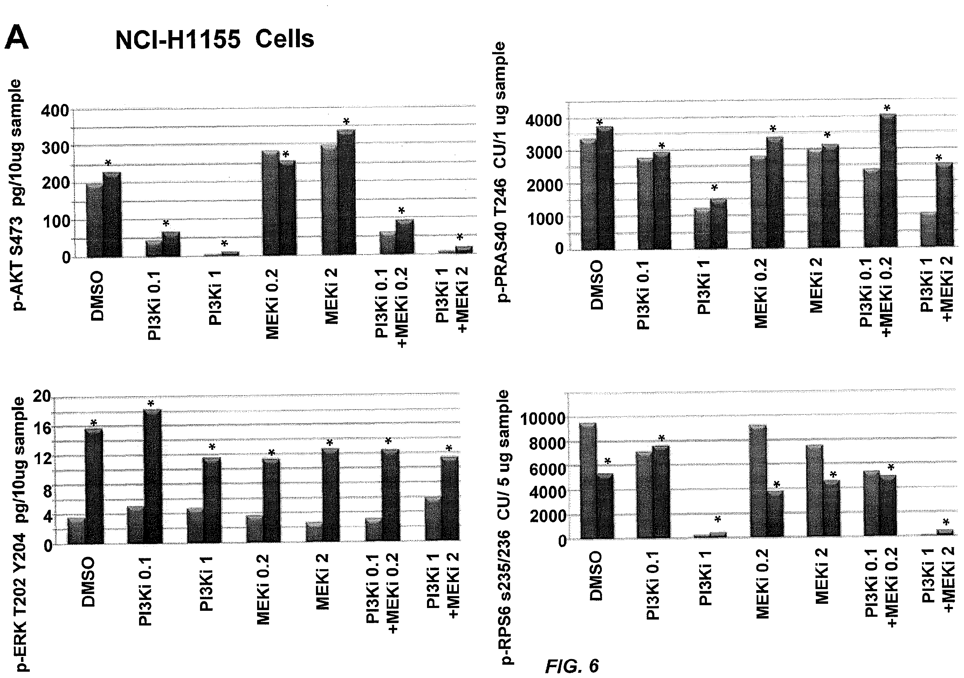

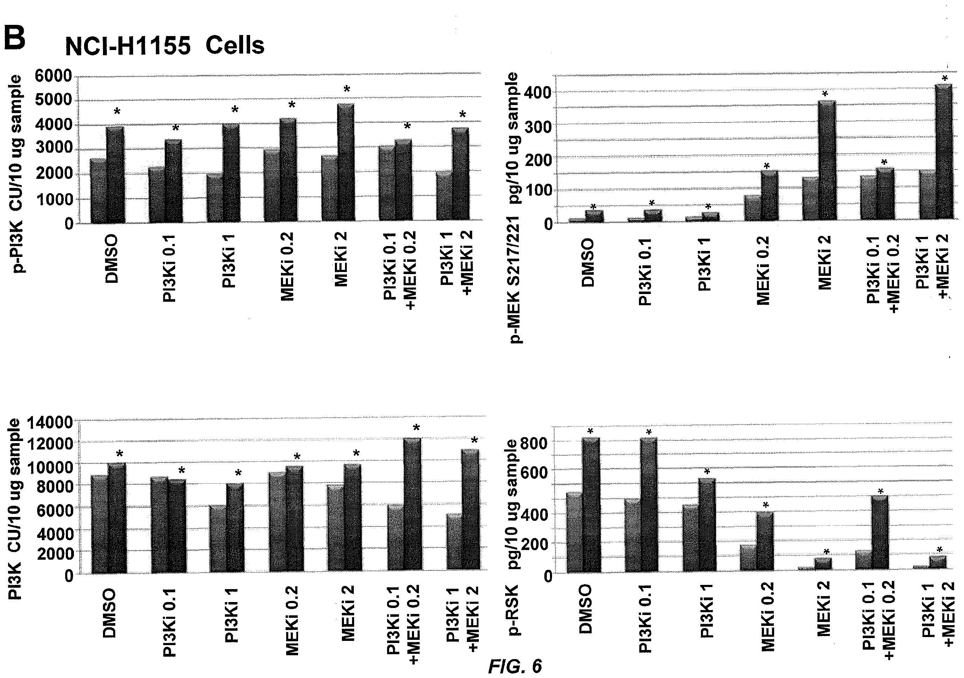

Lung cancer cell lines were treated with different inhibitor therapies (*e.g.,* different inhibitor amounts and treatment times with PI3K inhibitor, MEK inhibitor, and combination thereof) and showed that the level of PI3K activation was dependent on the cell line and the specific inhibitor therapy. The levels of other downstream signaling components of the pathway were measured, including HER1, HER2, cMET, PI3K, AKT, ERK, PTEN, MEK, p70S6K, PRAS40, RPS6 and PDK. Cell lines derived from non-small cell lung cancer such as NCI-H460, NCI-H647, NCI-H1155, NCI-1299, NCI-1975, NCI-H2228, and NCI-H661 were also used. The different therapies tested included a PI3K inhibitor alone at low or high dose, a MEK inhibitor alone at low or high dose, and a PI3K inhibitor plus a MEK inhibitor together at low dose or high dose. The cells were assayed at either 4 hours after treatment or 24 hours (see, * in FIGS. 6-10) after treatment.

FIG. 6A, B show that the levels of phospho-MEK (*e.g.,* phosphorylated S217, S221), phospho-PI3K, phospho-RSK, total PI3K, phospho-AKT *(*e.g., phosphorylated S473), phospho-PRAS40 (*e.g.,* phosphorylated T246), phospho-ERK (*e.g.,* phosphorylated T202, Y204), and phospho-RPS6 (*e.g.,* phosphorylated S235, S236) in the cell line H1155 vary after treatment of PI3K inhibitor alone, MEK inhibitor alone, and PI3K inhibitor in combination with MEK inhibitor. FIG.6 also shows that the activation status of these biomarkers changed at 4 hours and at 24 hours after treatment.

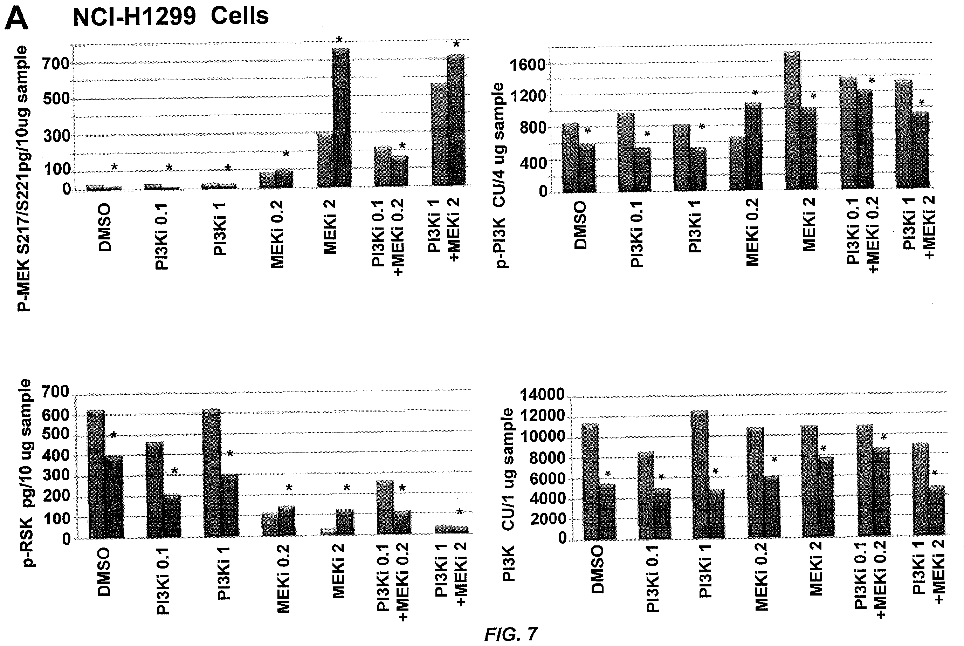

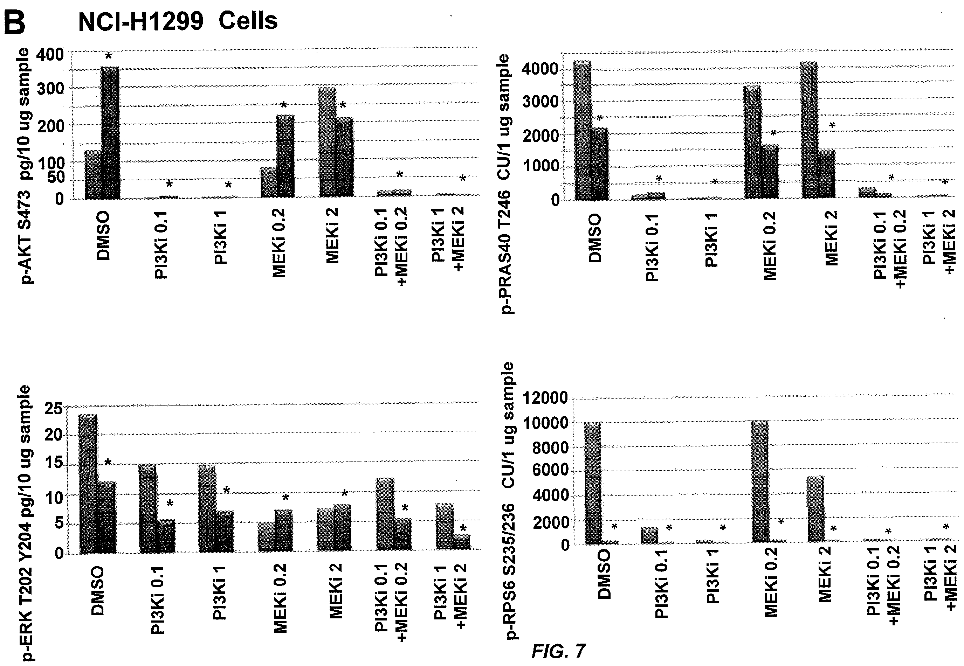

FIG. 7A, B show that the levels of phospho-MEK, phospho-PI3K, phospho-RSK, total PI3K, phospho-AKT, phospho-PRAS40, phospho-ERK, and phospho-RPS6 in the cell line H1299 are dependent on the treatment received as well as the time point tested.

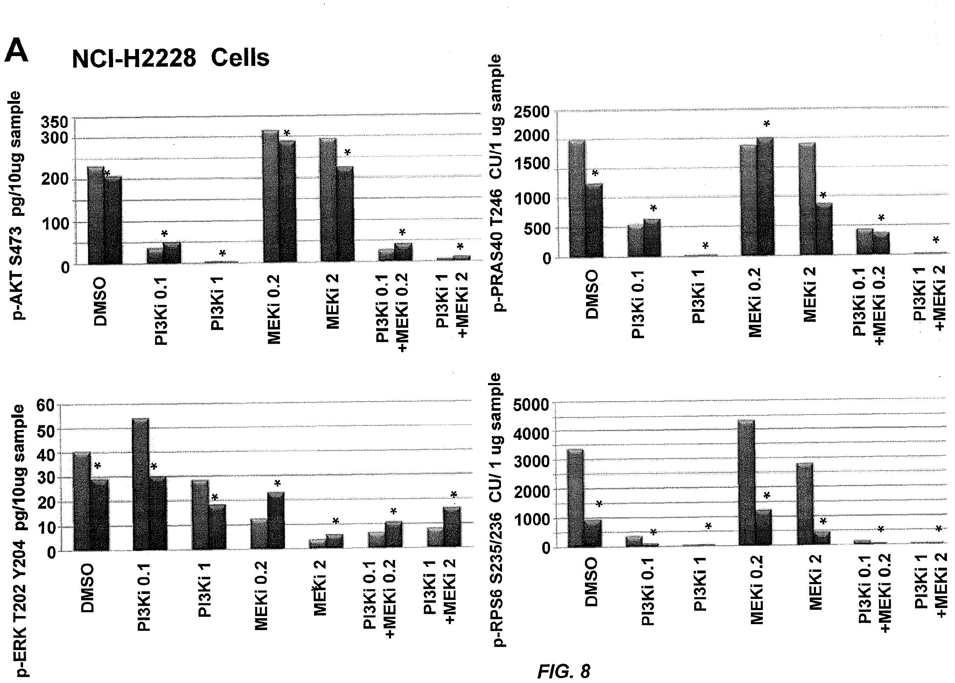

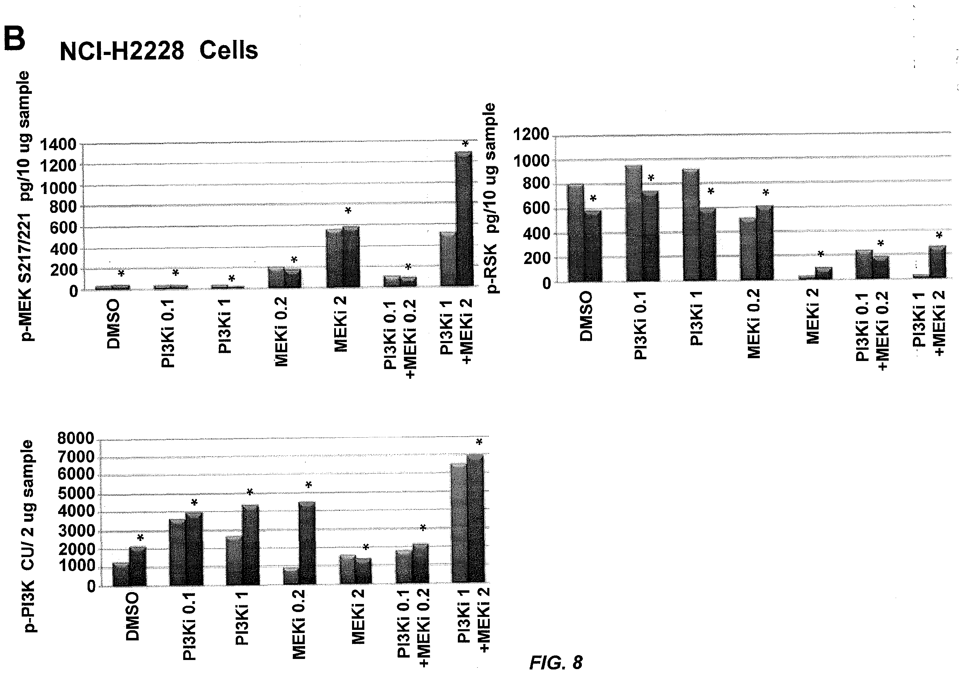

FIG. 8A, B show the levels of phospho-MEK, phospho-PI3K, phospho-RSK, phospho-AKT, phospho-PRAS40, phospho-ERK, and phospho-RPS6 in the cell line H2228 after the different treatments. The lower level of phospho-PI3K indicated that the H2228 cells were more responsive to low dose combination treatment than high dose combination treatment. The cells also responded to high dose MEK inhibitor alone and had lower levels of phospho-PI3K, phospho-RSK, and phospho-ERK.

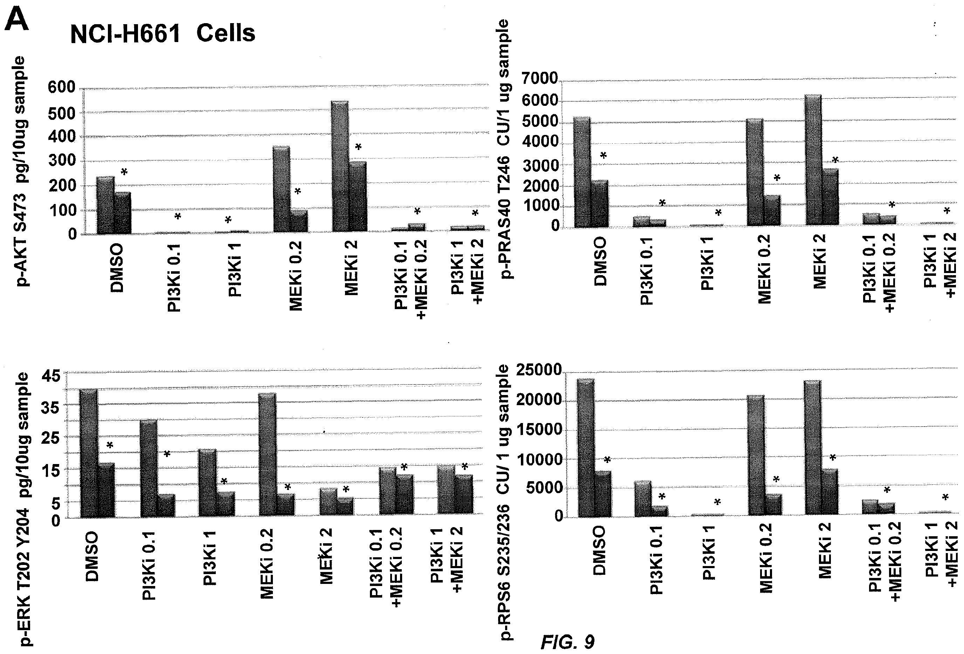

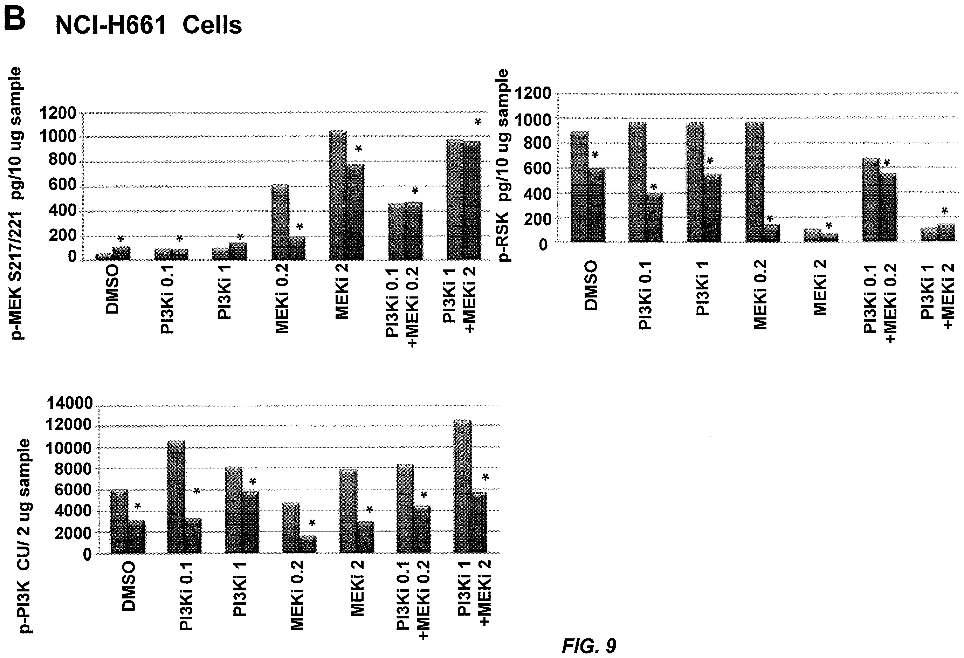

FIG. 9A, B show the levels of phospho-MEK, phospho-PI3K, phospho-RSK, phospho-AKT, phospho-PRAS40, phospho-ERK, and phospho-RPS6 in the cell line H661 after the different treatments.

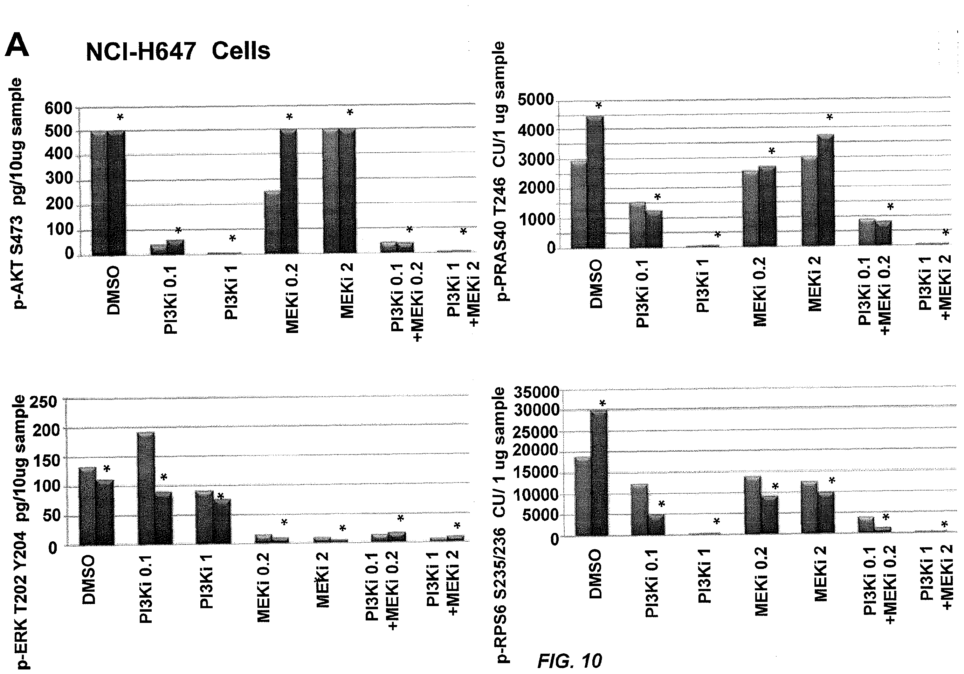

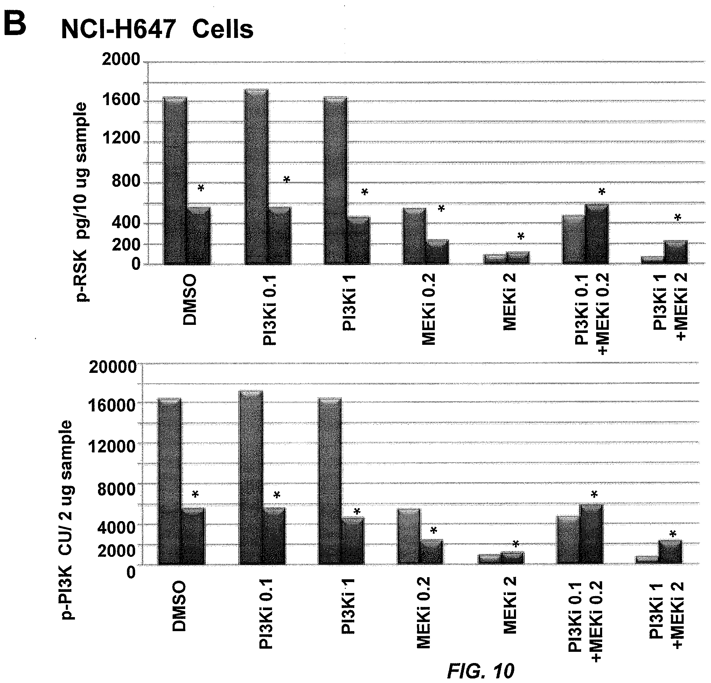

FIG. 10A, B show the levels of phospho-PI3K, phospho-RSK, phospho-AKT, phospho-PRAS40, phospho-ERK, and phospho-RPS6 in the cell line H647 after the different treatments.

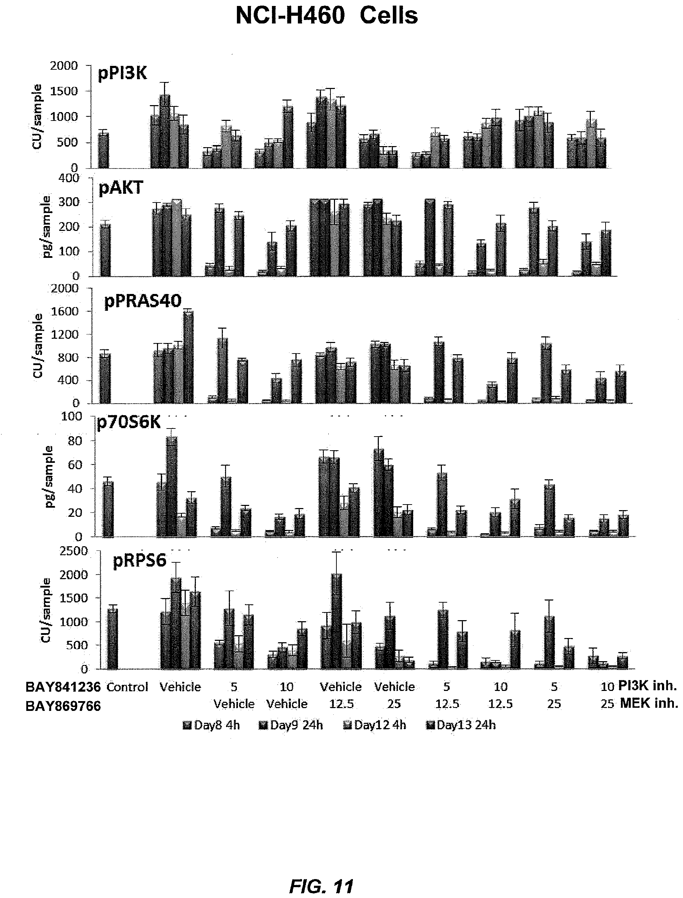

FIG. 11 shows the response of cell line H460 to different treatment regimens including a PI3K inhibitor (*e.g.,* BAY841236), a MEK inhibitor (*e.g.,* BAY869766), and combinations thereof. CEER analysis revealed that activated proteins of the HER1/HER2/cMET pathway (p-PI3K, p-AKT, p-PRAS40, p-70S6K, and p-RPS6) were downregulated in response to a PI3K inhibitor alone or in combination with a MEK inhibitor. The pathway profile was determined at several time points during the course of treatment including on day 8, day 9, day 12 and day 13 and either at 4 hours or 24 hours of drug exposure.

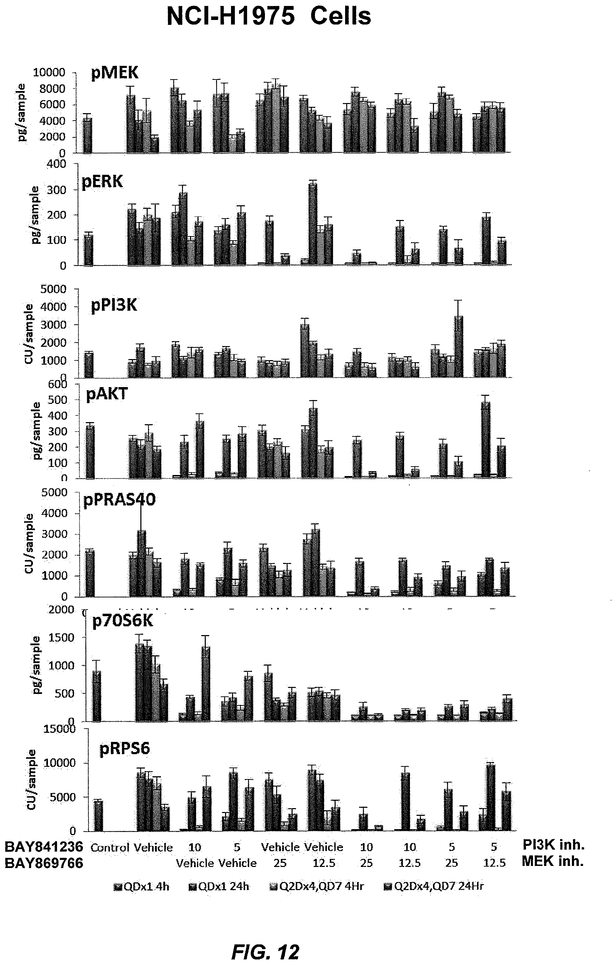

FIG. 12 shows that levels of activated PI3K are relatively similar in H1975 cells treated with either PI3K inhibitor (*e.g.,* BAY841236), MEK inhibitor (*e.g.,* BAY869766), or combinations thereof. CEER analysis showed that the levels of other activated proteins (p-MEK, p-ERK, p-AKT, p-PRAS40, p-70S6K, and p-RPS6) were greatly lower in response to treatment.

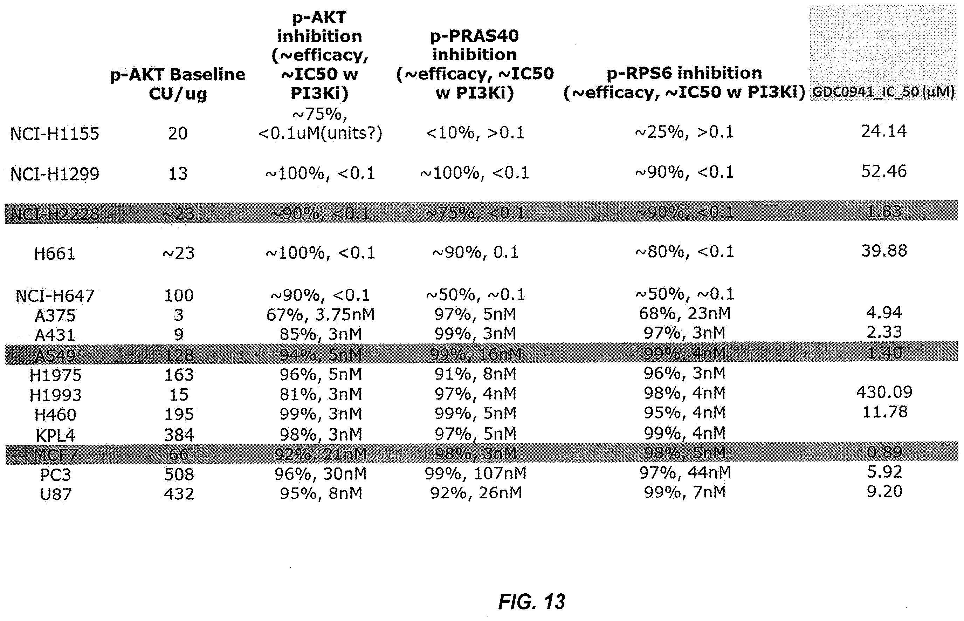

FIG. 13 illustrates that the CEER assay can be used to calculate the IC50 of an anticancer drug relative to the level of activated PI3K and other downstream components of the HER1/HER2/cMET pathway. FIG. 13 also shows that in some cell lines PI3K inhibition can be correlated to the inhibition of downstream proteins such as AKT, PRAS40, and RPS6. In some cancer cells inhibition of PI3K activation corresponds to inhibition of activation of downstream proteins. In addition the extent of the inhibition is specific to the cell line and the inhibitor treatment.

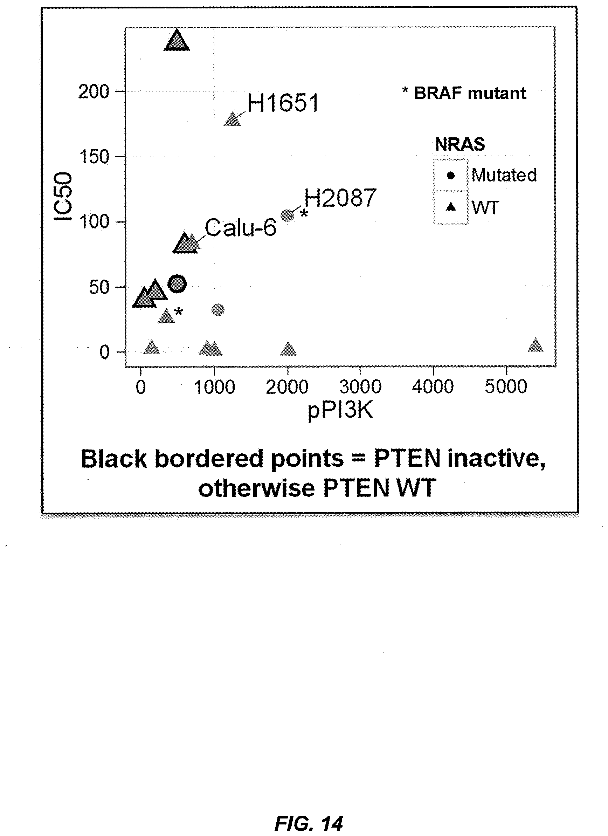

The relationship between the IC50 of the PI3K inhibitor drug (GDC0941) and activated PI3K levels was analyzed in several cell lines. It was determined that cell lines (*e.g.,* H1651 or H2087) with PI3K activation have low IC50. H1651 cells carry no known genetic mutations but have high EGFR expression. H2087 has NRAS and BRAF mutations. Cell lines (*e.g.,* Calu-6) with two more alterations such as multiple mutations (*e.g.,* MAPK mutations) and PI3K activation had high IC50 values. Thus, phospho-PI3K can be an indicator or predictor of sensitivity to PI3K inhibitors. FIG. 14 also illustrates that cell lines with inactive PTEN (*e.g.,* point mutation or null mutation) had higher IC50 values than cell lines with PTEN wild-type status.

This example illustrates that signal transduction analytes in a specific cancer cell have a specific expression and activation. Moreover, the expression and the activation status of the analytes change when the cell is exposed to an anticancer drug or combination thereof. Thus, signal transduction analytes can be monitored to determine the efficacy of anticancer drugs on tumor cells. Methods of pathway profiling can also be used to identify alternative targets for the development of new anticancer drugs.

### Example 3: Using PI3K CEER to Predict a Patient's Response to Inhibitor Therapy

This example illustrates that PI3K activation as detected by CEER can be used to determine a patient's likely response to PI3K inhibitor therapy. The example also illustrates that the methods herein are useful for longitudinal drug monitoring during the course of treatment.

In a retrospective study, a series of samples taken from patients with cancer were evaluated to determine if pathway profiling using the CEER assay is predictive of responsiveness to PI3K treatment. Samples analyzed included those from patients on a therapy time course, e.g., prior to receiving therapy, after initiating therapy, and during the course of therapy. The results clearly showed that measuring signal transduction analytes and determining a patient's pathway profiling are effective for determining whether the patient is likely to benefit from a specific drug therapy. Furthermore, the methods of selecting drug therapy for a patient with cancer and methods for longitudinal drug monitoring and treatment were demonstrated.

The example demonstrates that CEER for cancer can be used to profile cancer pathways in patient's biological samples (*e.g.,* FNA), thus enable the clinician to monitor disease progress during the course of therapy. The method also allows the clinical to determine the therapeutic efficacy of a specific treatment, and can adjust or modify the treatment specific to the evolving disease state.

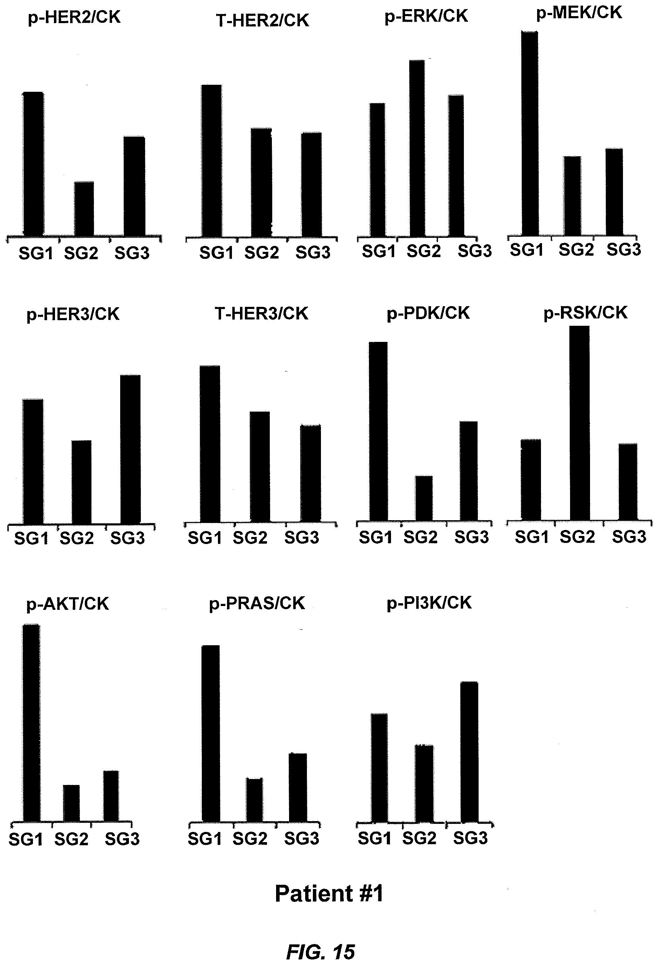

Samples from Patient #1 were taken prior to receiving therapy during the screening phase, and at Day 8 and Day 28 of PI3K inhibitor treatment phase. The sample from the screening phase was used to establish a baseline pathway profile for the patient. In the assay, the levels of activated proteins and total proteins in the HER1/HER2/cMET pathway were measured and normalized to the levels of cytokeratin (CK). It was determined that Patient #1 exhibited activated PI3K prior to treatment at SG1 (FIG. 15). Yet, on Day 8 of inhibitor treatment (SG1), Patient #1 had a lower level of activated PI3K. Downstream analytes, such as p-PDK, p-AKT, pPRAS, and p-MEK were also downregulated at Day 8. The analysis indicated that Patient #1 responded to the treatment, which was further confirmed by PET. Longitudinal drug monitoring using PI3K CEER showed that Patient #1 relapsed on treatment by Day 28 (SG2), as indicated by activation of the PI3K pathway via phospho-HER3 (FIG. 15). The results showed that other components of the signaling pathway (FIG. 1) can compensate for any blockage created by inhibitor therapy.

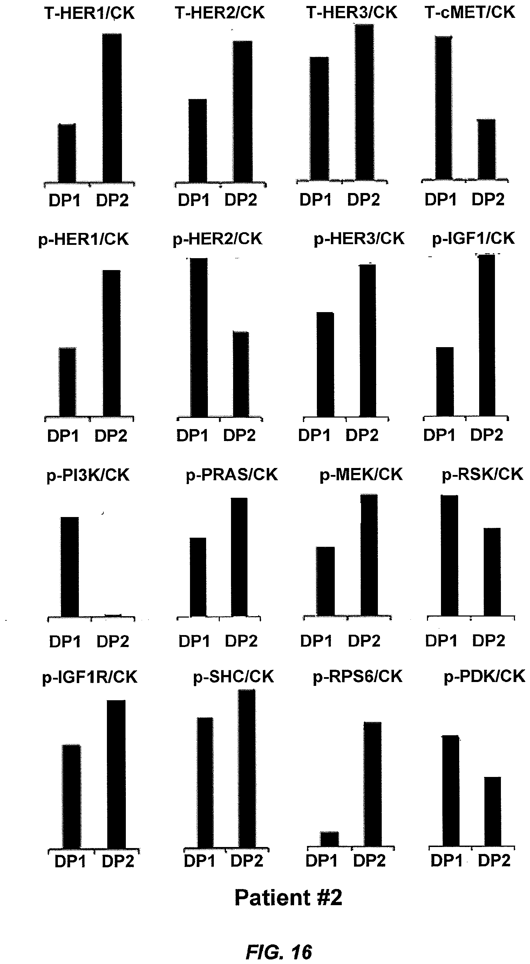

Patient #2 received PI3K inhibitor therapy and relapsed on treatment. Analysis using PI3K CEER showed that components of the HER1/HER2/cMET pathway were upregulated in cancers treated with PI3K inhibitor. A sample (DPI) taken after initiating therapy showed that Patient #2 was responding to PI3K therapy (FIG. 16). A second sample (DP2) taken after relapse showed that components of the HER1/HER2/cMET pathway were increased. In particular, activated and total protein was higher in the DP2 sample compared to the DP1 sample for most of the markers screened. PI3K inhibitor was effective at decreasing p-PI3K (FIG. 16), but other activated signal transduction molecules increased, thus compensating for PI3K inhibition. Patient #2's pathway profile shows that combination therapy is recommended following tumor adaptation.

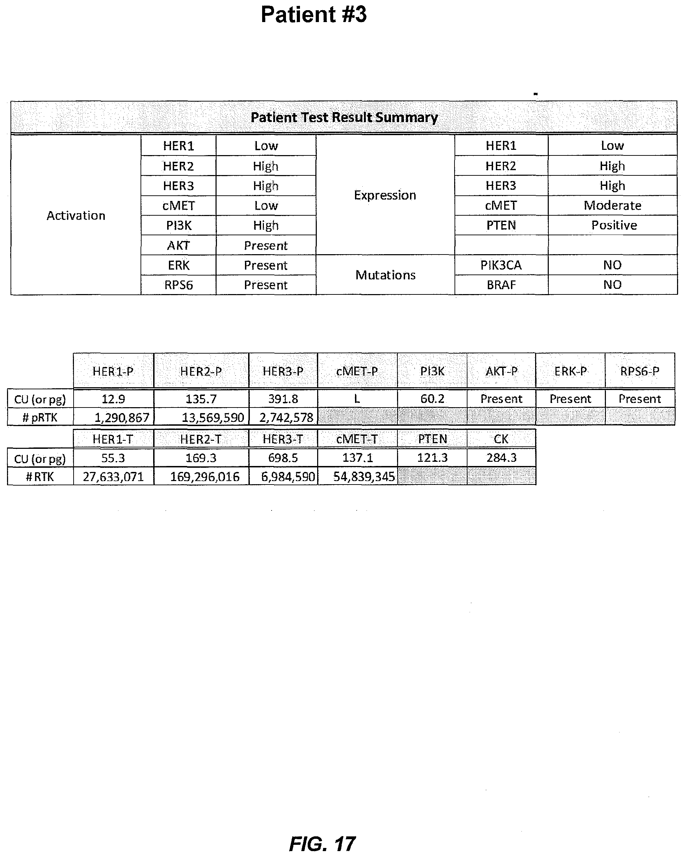

Patient #3 showed high PI3K activity in a sample taken prior to therapy. Notably, activated PI3K was not caused by a PI3KCA mutation. Following treatment with the PI3K inhibitor drug BAY806946, another sample was taken and analyzed. This FNA sample showed that PI3K driven cell proliferation was successfully inhibited with PI3K inhibitor treatment. This finding was confirmed by CT scan. FIG. 17 shows the CEER profile of Patient #3.

This example demonstrates that measuring signal transduction analytes in a patient sample is predictive of the patient's response to anticancer drug *(e.g.,* PI3K inhibitor). It also shows that monitoring of the analytes during the course of therapy can show that the patient is no longer responding to drug and/or has relapsed. The method described herein can be used to monitor tumor adaptation to therapy.

### Example 4: Using PI3K CEER to Assess PI3K Activation in Samples from Patients with Cancer

This example illustrates that pathway profiling of tumor samples from a patient provides useful information regarding cancer progression, treatment response, and disease status.

Patient #4 represents a 35 year old patient diagnosed with Stage III triple negative breast cancer (*e.g.,* ER-, PR-, HER2-). The patient underwent radical mastectomy. Routine follow-up examination revealed multiple metastatic lesions in the patient's liver and lung. Patient #4 received a combination therapy of HER2 inhibitor (Herceptin) and TKI (Lapatinib). Patient #4 responded to treatment and was in good condition for over 7 months.

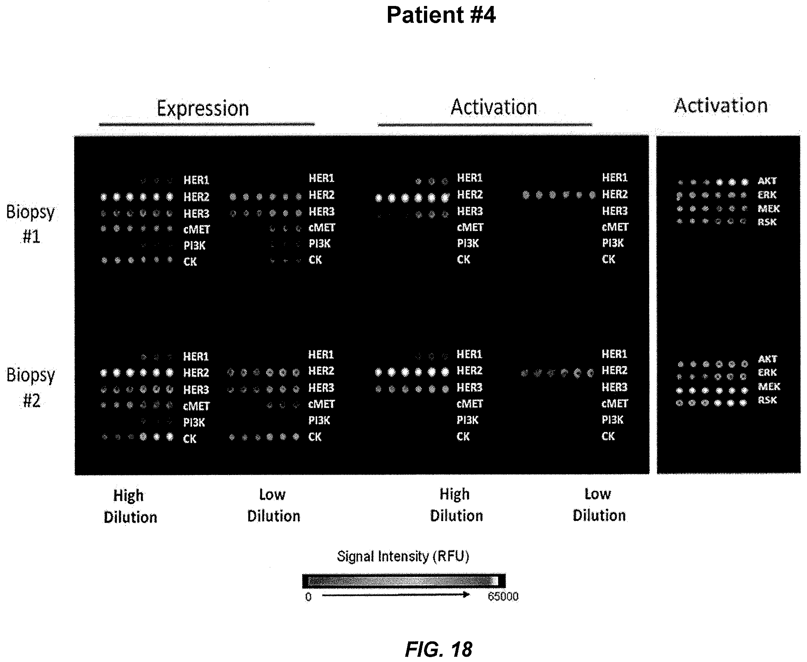

To determine if CEER analysis is predictive of treatment response, two biopsy samples (biopsy #1 and biopsy #2) from Patient #4 taken prior to receiving therapy (FIG. 18) were analyzed. In both biopsies, there was evidence of HER2 overexpression and activation, along with co-expression of HER3 and cMET. Low levels of HER2 and HER3 phosphorylation were observed. Downstream activation of both AKT and MAPK pathways was detected. The PI3KCA mutation H1047R was present in the biopsies. Pathway profiling of the samples predicted that Patient #4 would benefit from HER2 inhibitor therapy.

Patient #5 represents a 75 year old patient diagnosed with Stage III triple negative breast cancer (*e.g.,* ER-, PR-, HER2-). The patient underwent radical mastectomy. Follow-up evaluation revealed the presence of multiple metastatic lesions including tumors in the brain and bone. Patient #5 received a combination therapy of Herceptin with lapatinib or pertuzumab and showed response to therapy for over 6 months.

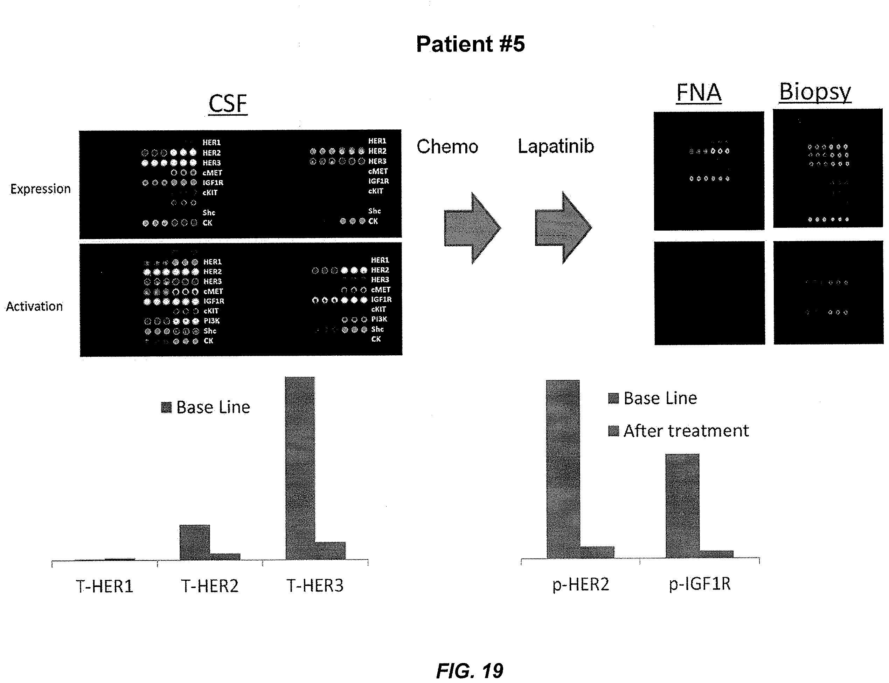

The pathway profile of several samples taken from Patient #5 prior to the start of treatment (*e.g.,* baseline) and after treatment (FIG. 19) were analyzed. The samples were cerebrospinal fluid (CSF), fine needle aspirate (FNA) and a biopsy. CEER analysis showed that PI3K activation as well as HER2 and IGF1R activation decreased after treatment, predicting that Patient #5 would respond to treatment of lapatinib.

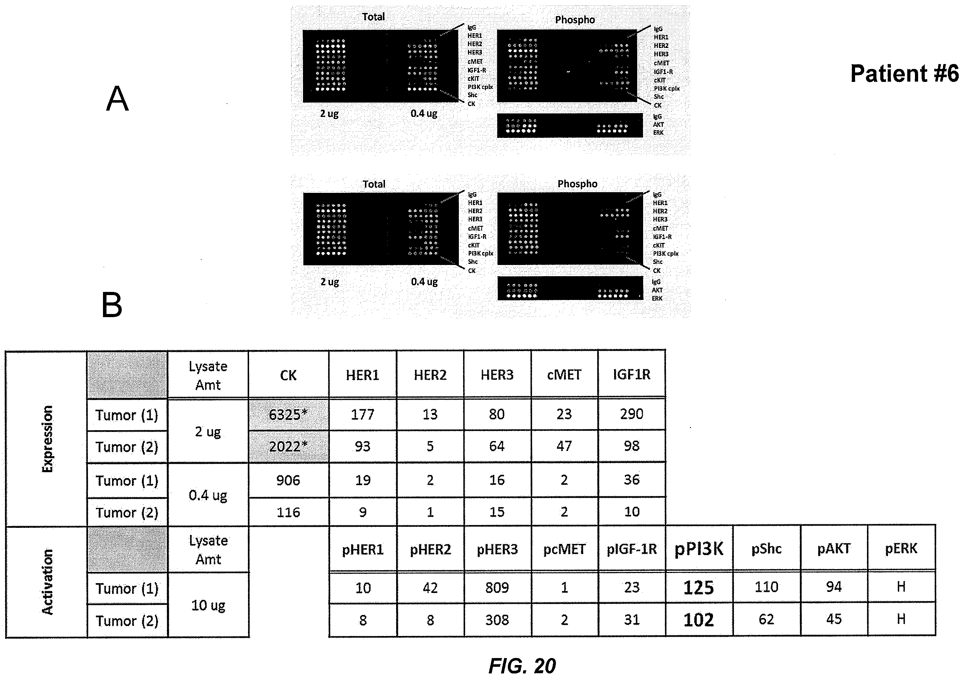

Patient #6 represents a patient with prostate cancer. FNA samples from two separate tumor sites were profiled by CEER analysis. The analysis showed that the HER1, HER2, HER3, cMET, IGF-1R and PI3K signaling pathways are active in the patient's samples. Hyperactivation of HER1, HER2, HER3, and PI3K was detected. Activation of p-AKT and p-ERK correlated to tumor proliferation. The pathway profile (FIG. 20A, B) further indicated that the cancer progression was of an aggressive nature, which is in agreement with Patient #6's Gleason score of 9. The patient's pathway profile and Gleason score both indicate that Patient #6 has a high grade cancer with a likelihood of disease progression (e.g., metastasis).

PI3K CEER was used to identify possible druggable targets for the treatment of triple negative breast cancer. In particular, Patient #7 (a triple negative BCA patient) had low levels of activated HER2 and high levels p-AKT and p-ERK, along with activated JAK1, JAK2, JAK3, CRKL, STAT1, STAT3, and FAK. It was also determined that the tumor sample carried the PI3KCA mutant allele H1047R.

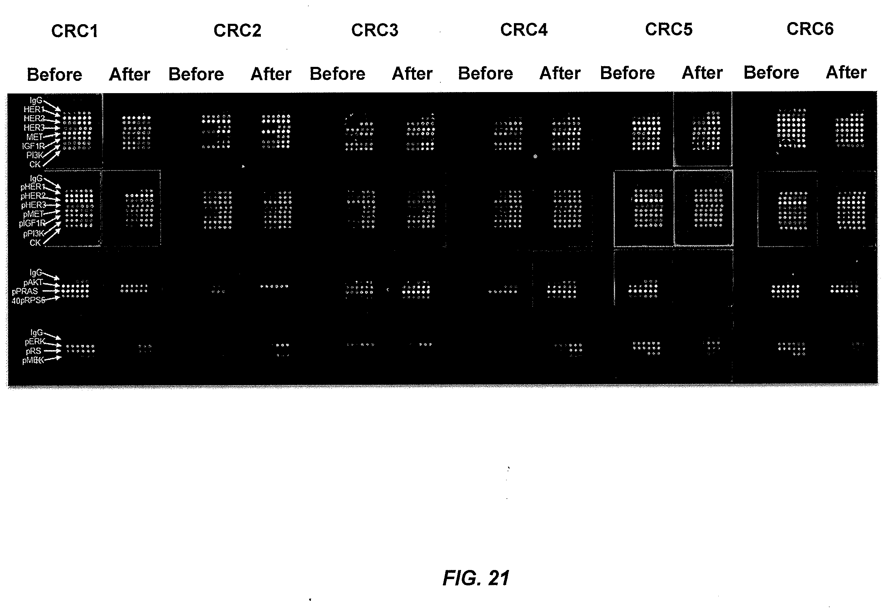

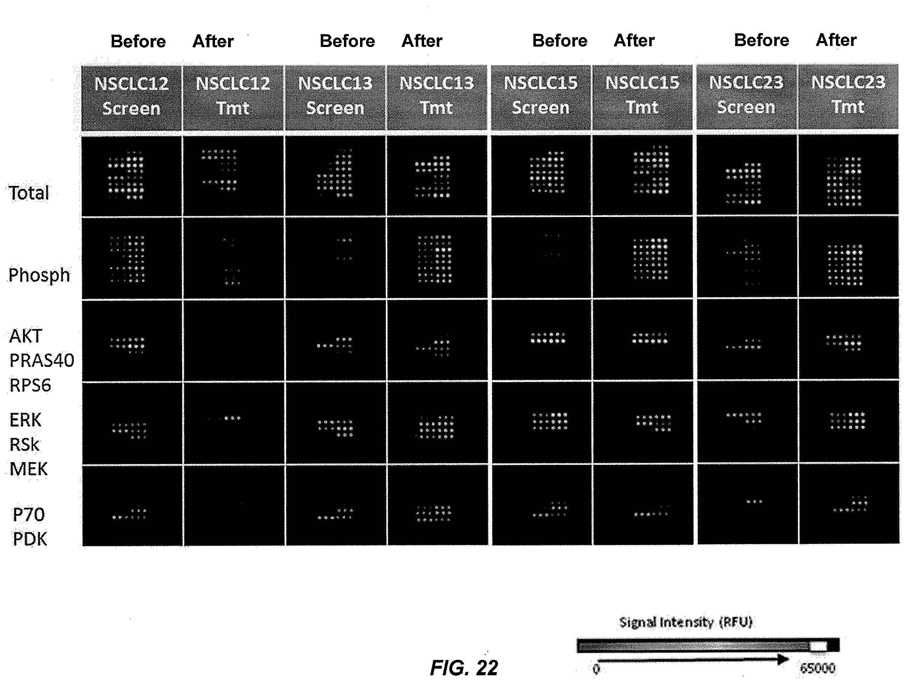

The pathway profiles of patients with colorectal cancer or non-small cell lung cancer before and after treatment were compared. FIGS. 21 and 22 showed that the tumor cells had a different pathway profile before and after treatment, indicating that a cancer patient has a specific, differential pathway or signature. The pathway profiles or signatures varied among patients, yet robust pathway activation patterns were observed in patients with cancer. The results show functional pathway profiling is critical for predicting potential therapy response and in selecting therapy for treating cancer patients. The methods described herein can be used to select patients before initiating therapy as well as monitoring the patients on therapy to measure changes in the pathway profile of cancer cells.

### Example 5: Quantitative Analysis of PI3K Activation for Selecting Patients for PI3K-Modulating Compounds.

This example demonstrates the presence of activated (phosphorylated) PI3K complex correlates with the presence of total and activated PI3K complex pathway components, including, but not limited to, HER1, HER2, cMET, PI3K, AKT, mTOR, p70S6K, RPS6, MEK, ERK, PDK1, and PTEN. This example illustrates that in some instances, breast cancer patients with elevated levels of PI3K activation also express activated (phosphorylated) AKT.

Pathway profiling analysis was performed on 60 FNA samples collected from breast cancer patients. A CEER assay was utilized to determine the levels of pathway protein expression and phosphorylation. Signal transduction analytes that were detected included HER1, HER2, HER3, cMET, PI3K, AKT, ERK, MEK, p70S6K, PDK1, PRAS40, PTEN, RPS6, SHC and associated downstream proteins.

CEER analysis includes normalizing the measured expression/activation levels of signaling transduction analytes against standard controls using statistical algorithms. In particular, Relative Fluorescence Unit (RFU) values are converted to Computed Units (CU), a standard functional unit of measure based on cell line controls with known levels of analyte expression. For each slide of the CEER assay, a standard curve of serial diluted cell lysate is prepared for a particular analyte-positive cell line (*e.g.,* HER1-positive cell line is MDA-MB-468; HER2-positive cell line is SKBr3). Thus, normalized levels of each particular analyte and the degree of phosphorylation in each sample can be obtained against standard cell lines.

For instance, a sample with 1 CU of HER1 expression has a RFU value equivalent to RFU value of 1 standard reference MDA-MB-468 cell. As reference cells have about 1-2 x10⁶ HER1 or HER2 receptors per cell with about 10% phosphorylated receptors, 1 CU represent 1-2 x10⁶ RTKs or 1-2 x10⁵ phosphorylated RTKs. The limit of detection (LOD) value by CU is determined empirically for each analyte in a CEER assay.

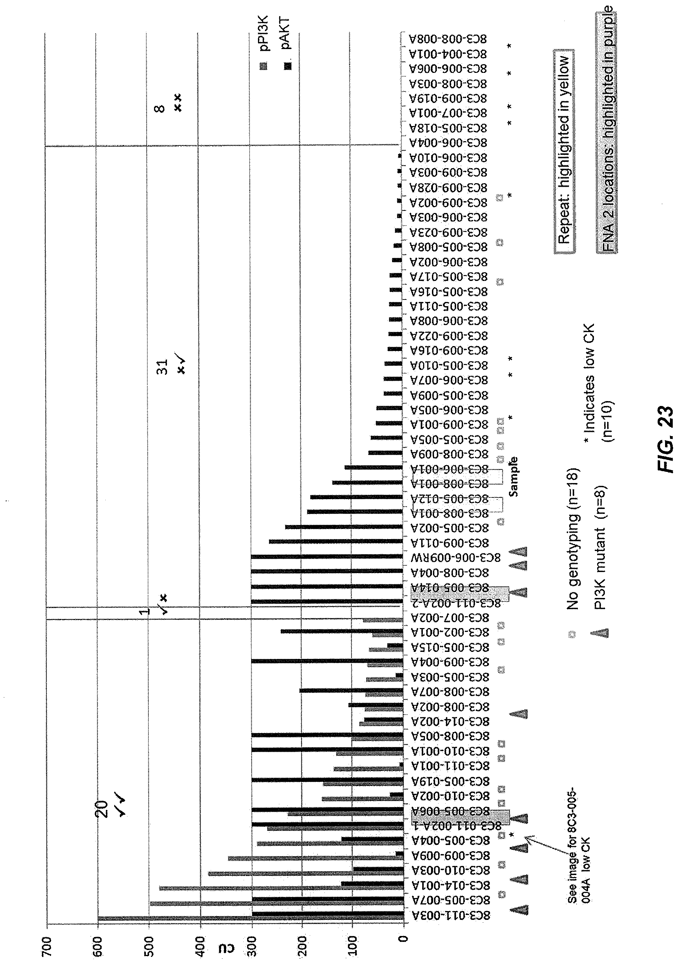

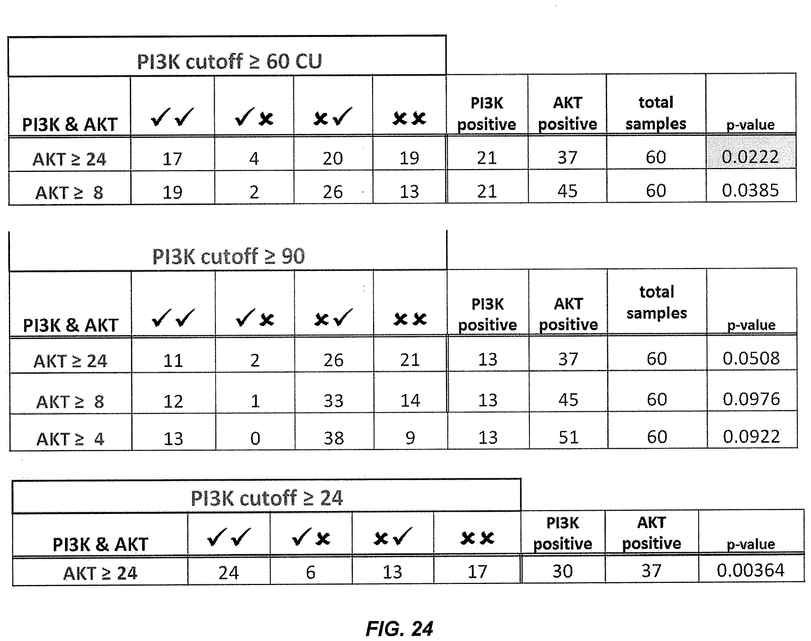

Of the 21 patients with activated PI3K, most (n=20) had elevated levels of phospho-AKT. This supports the discovery that phospho-AKT acts downstream of PI3K and that activation of PI3K can result in activation of AKT. The high degree of correlation (p-value=0.0222; FIGS. 23 and 24) between PI3K activation and phosphorylated AKT in breast cancer patient samples confirms that PI3K pathway activation is biologically relevant to tumorigenesis. The p-value was calculated for the rejection of null hypothesis and the alternative of positive correlation between PI3K and AKT.

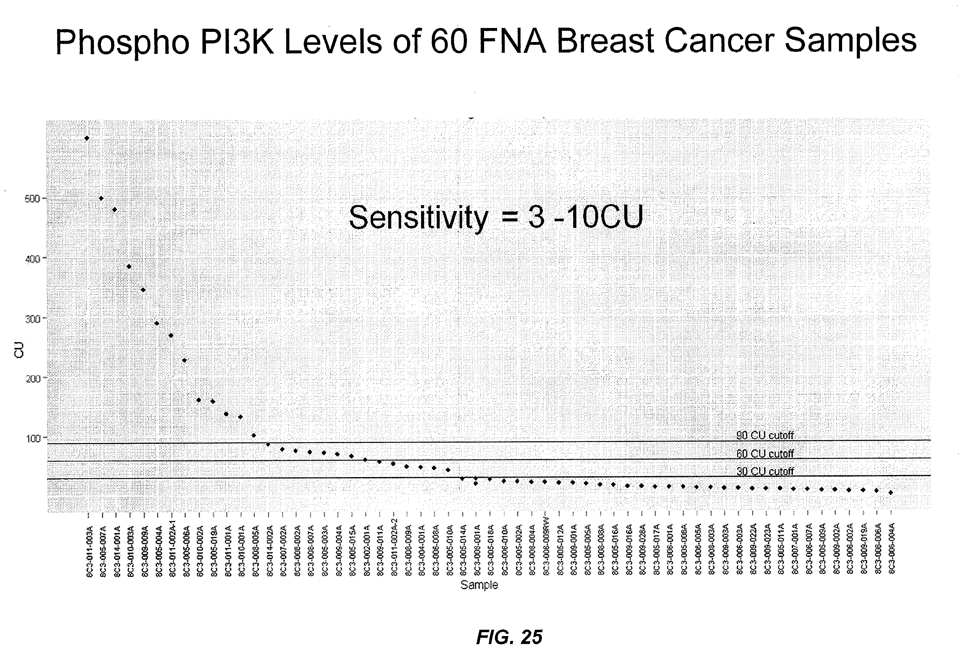

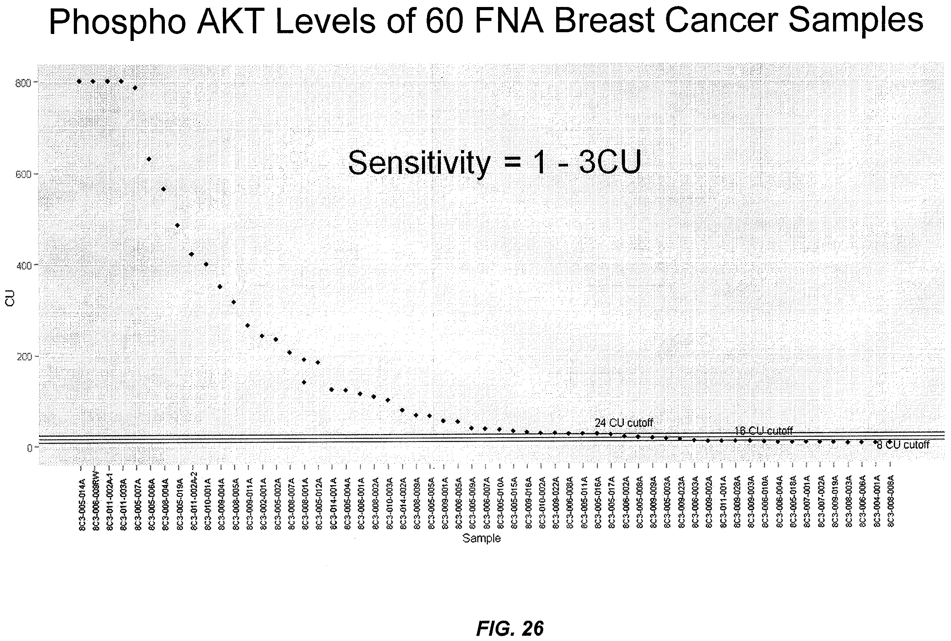

The distributions of p-PI3K levels and p-AKT levels in the breast cancer samples tested are shown in FIGS. 25 and 26, respectively. In the figures, the levels were sorted from high to low CUs and the CU cutoffs are displayed. The sensitivity of CEER was 3-10 CU PI3K and 1-3 CU for p-AKT.

The results also show that AKT induced cell proliferation is due, in part, to activated PI3K. FIG. 23 also shows that only 5 out of 21 patients with PI3K activation carried PI3KCA mutations. Thus, mutational analysis of PI3KCA should not be the only measure of PI3K activation and protein-based analysis, such as CEER are needed to identify patients who are likely to respond to PI3K inhibitor therapy (*e.g.,* patients with activated PI3K). The data indicates that cancer patients without PI3KCA mutations can benefit from PI3K inhibitor therapy.

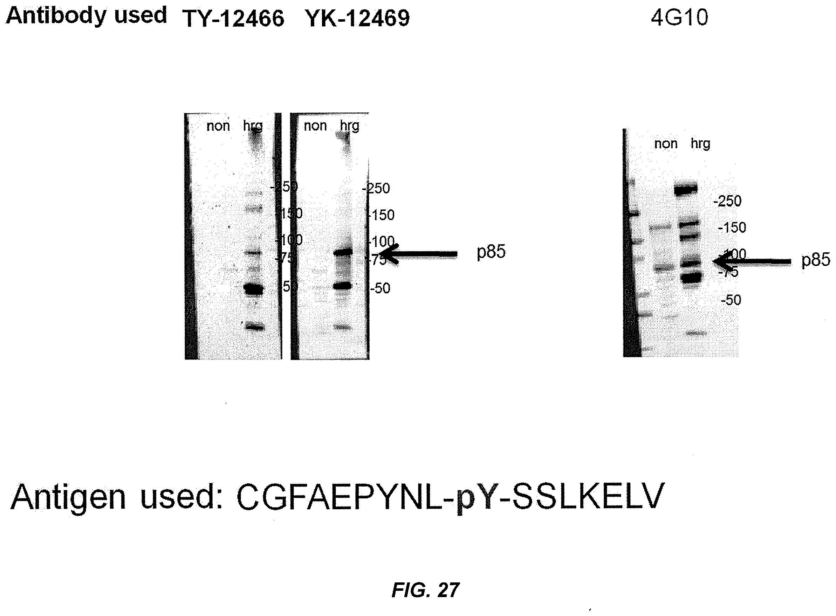

An antibody that can be used in CEER for measuring p-PI3K includes a monoclonal or polyclonal antibody raised to the antigen depicted in FIG. 27. The amino acid sequence (SEQ ID NO: 1) of the antigen is CGFAEPYNL-pY-SSLKELV, wherein pY represents a phosphorylated tyrosine residue.

### Example 6: Analysis of Breast Cancer (BCA) Clinical Samples and Identification of Changes in Signaling Pathway Profiles upon Treatment with PI3K Inhibitors.

This example illustrates a CEER-based analysis of BCA clinical samples from patients treated with a PI3K inhibitor, BYL719 (Novartis). In particular, signaling pathway profiles from individual patients are shown to shift during the course of BYL719 treatment. Therefore, CEER-based analysis of PI3K-related pathways allows for responsive, individualized management of BYL719 treatment to improve clinical outcomes. Oncogenic receptor tyrosine kinases (HER1, HER2, HER3, cMET, IGF-1R, and cKIT) were quantified, as well as PI3K and other tyrosine kinase signaling cascade components including SHC, AKT, ERK, MEK, RSK, pRAS 40, RPS6, P70S6K, and PTEN. Levels of total protein and activated protein were measured.

In general, pathway profile shifts were observed to vary from patient to patient. For example, prior to treatment, Patient 1 exhibited HER2 overexpression, HER3 and PI3K activation, and upregulation of p-AKT, p-PRAS, p-P70S6, p-RPS6, p-MEK, p-ERK, and p-RSK. Also at this time point, HER2 was highly activated and Patient 1 was PTEN positive. Treatment of Patient 1 with BYL719 resulted in increased activation of HER3, ERK, MEK, and RSK and decreased activation of AKT. On Day 8 of treatment, total HER3 expression increased, coinciding with an increased level of activated HER3 and a downregulation of p-AKT, p-pPRAS, p-P70S6, and p-RPS6. An initial decrease in activation of PRAS40 was observed, followed by an increase in activation as treatment progressed. On Day 28 of treatment, p-AKT, p-PRAS, p-P70S6 and p-RPS6 levels were higher than at Day 8, as well as HER3 expression and activation.

Prior to treatment, Patient 2, was PTEN positive and exhibited activated p-AKT, p-PRAS, p-MEK, p-ERK, and p-RSK. Treatment of Patient 2 with BYL719 resulted in a decrease in activation of AKT and PRAS40. An initial increase, followed by a decrease as treatment progressed, in the activation of ERK, MEK, and RSK was observed. On Day 8 of treatment, p-AKT and p-PRAS were downregulated, and p-MEK, p-ERK, and p-RSK were upregulated. On Day 28, p-AKT, p-PRAS, p-MEK, p-ERK, and p-RSK were downregulated.

The pathway profile of Patient 3 showed that at pretreatment, HER1, HER2, HER3 and cMET were expressed, and HER2 and cMET were highly activated. HER1, HER3 and PI3K were also seen to be activated. The analysis revealed that Patient 3 was PTEN positive and exhibited high levels of activated p-AKT and p-RAS. Activated p-MEK, p-ERK and p-RSK were also detected in Patient 3 at pretreatment. On Day 8, activated HER2, HER3 and cMET were all downregulated, while activated HER1 was upregulated. Also at this time point, Patient 3 exhibited downregulated levels of p-AKT, p-PRAS, p-MEK, p-ERK and p-RSK. A general decrease in RTK activation was observed upon treatment of Patient 3 with BYL719.

During pretreatment, Patient 4 was PTEN positive and expressed activated p-AKT, p-PRAS, p-MEK, p-ERK and p-RSK. On Day 8, the patient exhibited downregulation of p-AKT, p-PRAS, p-MEK, p-ERK and p-RSK. BYL719 therapy resulted in a decrease in PRAS40 activation in Patient 4.

The pathway profile of Patient 5 revealed that HER1, HER2, HER3 and cMET were expressed during pretreatment, along with activated HER1, HER2, PI3K, p-AKT, p-PRAS, RPS6, p-MEK, p-ERK, and p-RSK. Patient 5 was also PTEN positive during pretreatment. BYL719 therapy resulted in a decrease in PRAS40 activation in Patient 5. P-AKT and p-RAS were slightly upregulated and p-RPS6 was highly upregulated. Little change was detected for p-MEK, p-ERK and p-RSK levels on Day 8 of therapy.

## Claims

1. A method for selecting treatment for a subject diagnosed with lung cancer, the method comprising:
(a) measuring the levels of phospho-PI3K, phospho-AKT, phospho-ERK, phospho-MEK, phospho-RSK, phospho-PRAS40, and phospho-RPS6 in a sample taken from the subject using a proximity assay to determine a pathway profile, wherein the proximity assay is a Collaborative Enzyme Enhanced Reactive immunoassay (CEER);
(b) comparing the subject's pathway profile to a reference pathway profile, wherein the reference pathway profile is determined using a sample from a cancer cell line or from a subject having a specific type of cancer; and
(c) recommending a targeted PI3K inhibitor, a MEK inhibitor or a combination of a PI3K inhibitor and a MEK inhibitor therapy by determining whether the subject is likely to respond to the targeted inhibitor therapy based on the subject's pathway profile.

2. A method for optimizing therapy or monitoring therapeutic efficacy of a targeted inhibitor for a subject with lung cancer, the method comprising:
(a) measuring the levels of phospho-PI3K, phospho-AKT, phospho-ERK, phospho-MEK, phospho-RSK, phospho-PRAS40, and phospho-RPS6 in a sample taken from the subject using a proximity assay at a plurality of time points over the course of therapy with a targeted inhibitor selected from the group consisting of a PI3K inhibitor, a MEK inhibitor and a combination thereof to determine a pathway profile, wherein the proximity assay is a CEER;
(b) determining whether the subject is responsive to the targeted inhibitor based on the subject's pathway profile;
(c) comparing the subject's pathway profile to a reference pathway profile, wherein the reference pathway profile is determined using a sample from a cancer cell line or from a subject having a specific type of cancer; and
(d) recommending the administration of the targeted inhibitor be continued if the subject is responsive.

3. The method of claim 2, further comprising recommending the administration of the targeted inhibitor be modified or of at least two targeted inhibitors if the subject is non-responsive.

4. The method of claim 2, wherein the first time point in the plurality of time points is prior to the course of therapy with the targeted inhibitor.

5. The method of claim 2, wherein the subsequent time point or points in the plurality of time points is during the course of therapy with the targeted inhibitor.

6. The method of claims 1 or 2, wherein the sample is a fine needle aspirate, a tumor tissue biopsy, a tumor cell, or a circulating tumor cell.

7. The method of any one of claims 1-6, wherein the PI3K inhibitor is selected from the group consisting of BYL719, BAY841236, BAY806946, SF1 126, XL147, XL765, NVP-BEZ235, NVP-BGT226, NVP-BKM120, GDC-0941, PX-866, GSK1059615, CAL-101, and combinations thereof.

8. The method of any one of claims 1-7, wherein the MEK inhibitor is selected from the group consisting of BAY869766, MEK 162, GDC-0973/RG7420, GDC-0623/RG7421, RG7167, RG7304, XL518, PD-325901, and combinations thereof.

## Patentansprüche

1. Verfahren zum Auswählen einer Behandlung für ein Subjekt, bei dem Lungenkrebs diagnostiziert wurde, wobei das Verfahren Folgendes umfasst:
(a) Messen des Gehalts von Phospho-PI3K, Phospho-AKT, Phospho-ERK, Phospho-MEK, Phospho-RSK, Phospho-PRAS40 und Phospho-RPS6 in einer Probe, die von dem Subjekt unter Verwendung eines Proximitätsassays genommen wird, um ein Verlaufsprofil zu bestimmen, wobei das Proximitätsassay ein Collaborative Enzyme Enhanced Reactive-Immunassay (CEER) ist;
(b) Vergleichen des Verlaufsprofils des Subjekts mit einem Bezugs-Verlaufsprofil, wobei das Bezugs-Verlaufsprofil unter Verwendung einer Probe aus einer Krebszelllinie oder aus einem Subjekt bestimmt ist, das eine bestimmte Krebsart hat; und
(c) Empfehlen eines zielgerichteten PI3K-Inhibitors, eines MEK-Inhibitors oder einer Kombination aus einer PI3K-Inhibitor- und einer MEK-Inhibitortherapie durch Bestimmen, ob das Subjekt wahrscheinlich auf die zielgerichtete Inhibitortherapie basierend auf dem Verlaufsprofil des Subjekts anspricht.

2. Verfahren zum Optimieren einer Therapie oder zum Überwachen von therapeutischer Wirksamkeit eines zielgerichteten Inhibitors für ein Subjekt mit Lungenkrebs, wobei das Verfahren Folgendes umfasst:
(a) Messen des Gehalts von Phospho-PI3K, Phospho-AKT, Phospho-ERK, Phospho-MEK, Phospho-RSK, Phospho-PRAS40 und Phospho-RPS6 in einer Probe, die von dem Subjekt unter Verwendung eines Proximitätsassays zu einer Vielzahl von Zeitpunkten über den Verlauf der Therapie mit einem zielgerichteten Inhibitor genommen wird, der ausgewählt ist aus der Gruppe bestehend aus einem PI3K-Inhibitor, einem MEK-Inhibitor und einer Kombination davon, um ein Verlaufsprofil zu bestimmen, wobei das Proximitätsassay ein CEER ist;
(b) Bestimmen, ob das Subjekt auf den zielgerichteten Inhibitor basierend auf dem Verlaufsprofil des Subjekts anspricht;
(c) Vergleichen des Verlaufsprofils des Subjekts mit einem Bezugs-Verlaufsprofil, wobei das Bezugs-Verlaufsprofil unter Verwendung einer Probe aus einer Krebszelllinie oder aus einem Subjekt bestimmt ist, das eine bestimmte Krebsart hat; und
(d) Empfehlen, dass die Verabreichung des zielgerichteten Inhibitors fortgeführt wird, wenn das Subjekt darauf anspricht.

3. Verfahren nach Anspruch 2, ferner umfassend das Empfehlen, dass die Verabreichung des zielgerichteten Inhibitors modifiziert wird, oder von mindestens zwei zielgerichteten Inhibitoren, wenn das Subjekt darauf nicht anspricht.

4. Verfahren nach Anspruch 2, wobei der erste Zeitpunkt in der Vielzahl von Zeitpunkten vor dem Verlauf der Therapie mit dem zielgerichteten Inhibitor ist.

5. Verfahren nach Anspruch 2, wobei der nachfolgende Zeitpunkt oder die nachfolgenden Zeitpunkte in der Vielzahl von Zeitpunkten während dem Verlauf der Therapie mit dem zielgerichteten Inhibitor ist.

6. Verfahren nach Anspruch 1 oder 2, wobei die Probe ein Feinnadelaspirat, eine Tumorgewebebiopsie, eine Tumorzelle oder eine zirkulierende Tumorzelle ist.

7. Verfahren nach einem der Ansprüche 1 bis 6, wobei der PI3K-Inhibitor ausgewählt ist aus der Gruppe bestehend aus BYL719, BAY841236, BAY806946, SF1 126, XL147, XL765, NVP-BEZ235, NVP-BGT226, NVP-BKM120, GDC-0941, PX-866, GSK1059615, CAL-101 und Kombinationen davon.

8. Verfahren nach einem der Ansprüche 1 bis 7 wobei der MEK-Inhibitor ausgewählt ist aus der Gruppe bestehend aus BAY869766, MEK 162, GDC-0973/RG7420, GDC- 0623/RG7421, RG7167, RG7304, XL518, PD-325901 und Kombinationen davon.

## Revendications

1. Procédé de sélection d'un traitement destiné à un sujet atteint d'un cancer du poumon, le procédé comprenant :
(a) la mesure des taux de phospho-PI3K, phospho-AKT, phospho-ERK, phospho-MEK, phospho-RSK, phospho-PRAS40 et phospho-RPS6 dans un échantillon prélevé chez le sujet en utilisant un dosage de proximité pour déterminer un profil de parcours de soins, où le dosage de proximité est un immunodosage réactif amplifié par enzyme collaborative (CEER) ;
(b) la comparaison du profil de parcours de soins du sujet à un profil de parcours de soins de référence, où le profil de parcours de soins de référence est déterminé en utilisant un échantillon d'une lignée cellulaire du cancer ou d'un sujet ayant un type spécifique de cancer ; et
(c) la préconisation d'un traitement par un inhibiteur de PI3K, un inhibiteur de MEK ou une combinaison d'un inhibiteur de PI3K et d'un inhibiteur de MEK ciblés en déterminant si le sujet est susceptible de répondre à un traitement par inhibiteur ciblé sur la base du profil du parcours de soins du sujet.

2. Procédé destiné à optimiser un traitement ou à suivre l'efficacité thérapeutique d'un inhibiteur ciblé chez un sujet ayant un cancer du poumon, le procédé comprenant :
(a) la mesure des taux de phospho-PI3K, phospho-AKT, phospho-ERK, phospho-MEK, phospho-RSK, phospho-PRAS40 et phospho-RPS6 dans un échantillon prélevé chez le sujet en utilisant un dosage de proximité à une pluralité d'instants pendant le processus thérapeutique avec un inhibiteur ciblé sélectionné dans le groupe constitué par un inhibiteur de PI3K, un inhibiteur de MEK et une combinaison de ceux-ci pour déterminer un profil de parcours de soins, où le dosage de proximité est un CEER ;
(b) la détermination de la réaction du sujet à l'inhibiteur ciblé sur la base du profil du parcours de soins du sujet ;
(c) la comparaison du profil du parcours de soins du sujet avec un profil de parcours de soins de référence, où le profil de parcours de soins de référence est déterminé en utilisant un échantillon d'une lignée cellulaire du cancer ou d'un sujet ayant un type spécifique de cancer ; et
(d) la préconisation de la poursuite de l'administration de l'inhibiteur ciblé si le sujet réagit.

3. Procédé selon la revendication 2, comprenant en outre la préconisation de l'administration de l'inhibiteur ciblé modifié ou d'au moins deux inhibiteurs ciblés si le sujet ne réagit pas.

4. Procédé selon la revendication 2, dans lequel le premier instant dans la pluralité des instants est antérieur au processus thérapeutique avec l'inhibiteur ciblé.

5. Processus selon la revendication 2, dans lequel l'instant ou les instants ultérieurs dans la pluralité des instants se situent au cours du processus thérapeutique avec l'inhibiteur ciblé.

6. Procédé selon les revendications 1 ou 2, dans lequel l'échantillon est prélevé par aspiration à l'aide d'une aiguille fine, est une biopsie de tissu tumoral, une cellule tumorale, ou une cellule tumorale circulante.

7. Procédé selon l'une quelconque des revendications 1 à 6, dans lequel l'inhibiteur de PI3K est sélectionné dans le groupe constitué par BYL719, BAY841236, BAY806946, SF1 126, XL147, XL765, NVP-BEZ235, NVP-BGT226, NVP-BKM120, GDC-0941, PX-866, GSK1059615, CAL-101, et les combinaisons de ceux-ci.

8. Procédé selon l'une quelconque des revendications 1 à 7, dans lequel l'inhibiteur de MEK est sélectionné dans le groupe constitué par BAY869766, MEK 162, GDC-0973/RG7420, GDC-0623/RG7421, RG7167, RG7304, XL518, PD-325901, et les combinaisons de ceux-ci.
